# EUROPEAN PATENT SPECIFICATION

(11) **EP 2 225 376 B1**
(45) Date of publication and mention of the grant of the patent: **08.01.2014**
(21) Application number: 08857617.8
(22) Date of filing: 26.11.2008
(51) Int. Cl.: C12N 15/11

(54) **RNA ANTAGONIST COMPOUNDS FOR THE MODULATION OF PIK3CA EXPRESSION**
RNA-ANTAGONISTVERBINDUNGEN ZUR MODULATION DER PIK3CA-EXPRESSION
COMPOSÉS D'ANTAGONISTES D'ARN UTILISÉS POUR MODULER L'EXPRESSION DE PIK3CA

(30) Priority: 03.12.2007 US 992050 P
(43) Date of publication of application: 08.09.2010
(73) Proprietor: Santaris Pharma A/S, 2970 Hørsholm (DK)
(72) Inventor: HEDT JÅRN, Maj, 2450 København (DK)
(74) Representative: Wroe, Stephanie
(86) International application number: PCT/DK2008/000418
(87) International publication number: WO 2009/071082

(56) References cited:
- WO-A-2005/091849
- WO-A-2007/107162
- WO-A-2007/136758
- BRODERICK DANIEL K ET AL: "Mutations of PIK3CA in anaplastic oligodendrogliomas, high-grade astrocytomas, and medulloblastomas" CANCER RESEARCH, vol. 64, no. 15, 1 August 2004 (2004-08-01), pages 5048-5050, XP002517093 ISSN: 0008-5472
- KURRECK J ET AL: "Design of antisense oligonucleotides stabilized by locked nucleic acids" NUCLEIC ACIDS RESEARCH, OXFORD UNIVERSITY PRESS, SURREY, GB, vol. 30, no. 9, 1 May 2002 (2002-05-01), pages 1911-1918, XP002281375 ISSN: 0305-1048

## Description

### FIELD OF INVENTION

The present invention relates to oligomeric compounds (oligomers) that target PIK3CA mRNA in a cell, leading to reduced expression of PIK3CA. In particular, this invention relates to oligomeric compounds (oligomers), which target the PIK3CA kinase mRNA in a cell, leading to reduced expression of the PIK3CA kinase. Reduction of PIK3CA expression is beneficial for a range of medical disorders, such as hyperproliferative diseases such as cancer.

### RELATED CASES

This application claims the benefit under 35 U.S.C. § 119(e) of U.S. Provisional Application Serial No. 60/992,050, filed December 3, 2007.

### BACKGROUND

Phospatidylinositol 3-kinase (PI3K) is a ubiquitous lipid kinase involved in receptor signal transduction by tyrosine kinase receptors. PI3K comprises a large and complex family that includes 3 classes with multiple subunits and isoforms. The class I P13Ks are composed of a Src homology-2 domain-containing an 85 kDa regulatory subunit (p85) and a 100-kDa catalytic subunit (p110), which catalyses the phosphorylation of phosphoinositol 4-phosphate and phosphoinisitol 4,5-phosphate at their D3 positions. The PI3K regulatory subunits include p85alpha and its truncated splice variants p50alpha and p55alpha, as well as p85beta and p55gamma; the catalytic subunits include p110alpha, p110beta, and p110delta. The human catalytic subunit p110alpha is encoded by the PIK3CA gene, located on the human chromosome 3 [Chr 3: 180.35 - 180.44 M bp] specifically [chr3:180,349,005-180,435,191 bp](NCBI reference sequence annotation) (3q26.3), which is frequently mutated in a variety of human cancers; PIK3CA has been shown to be mutated in 32% of colorectal cancer, 27% of glioblastomas, 25% of gastric cancers, 36% of hepatocellular carcinomas, 18-40% of breast cancers, 4-12% of ovarian cancers and 4% of lung cancers (Samuels et al., 2006). Most of the mutations found map to three mutational hotspots within the PIK3CA coding sequence, known as E542K, E545K and H1047R (Kang et al., 2005). A comprehensive map of detected PIK3CA mutations and their prevalence in different human tissues can be found on
http://www.sanger.ac.uk/perl/genetics/CGP/cosmic?action=gene&In=PIK3CA.

PI3K has been indicated in a wide range of cancers, such as colorectal carcinoma, where is has been shown that the activation of PI3K/Akt is associated with colorectal carcinoma and can convert differentiated human gastric or colonic carcinoma cells to a less differentiated and more malignant phenotype (Rychahou et al 2006).

The effects of P13K on tumor growth and progression are thought to be mediated by Akt, a downstream effector of P13K. In humans there are three memebers of the Akt gene family, Akt 1, Akt 2 and Akt3. Akt is overexpressed in a number of cancers, including colon, pancreatic, ovarian and some steroid hormone-insensitive breast cancers.

Inhibitors of proteins that are involved in the P13K/Akt signalling which have been suggested as potential therapeutic agents include both siRNAs and antisense oligonucleotides (US2006030536A), however to date most research in this area appears to have focused on the use of siRNAs:
WO2005/091849 refers to antisense down-regulation of PI3K, however no specific antisense oligonucleotides are disclosed.

Zhang et al., 2004 (Cancer Biology and Therapy 3:12 1283-1289) disclose siRNAs targeting p110alpha and suggest its use in gene therapy in ovarian cancer.

Rychahou et al 2006 (Annals of Surgery 243833 - 844) discloses siRNA complexes targeting p85alpha and p110alpha which were found to decrease *in vitro* colon cell cancer survival and increase apoptosis in human colon cancer cells, and decreased liver metastasis in in vivo experiments.

Meng et al., 2006 (Cellular Signalling 18 2262-2271) disclose siRNAs targeting p110alpha for inhibiting P13K activity in ovarian cancer cells. The authors determined that inhibition of AKT1 is sufficient to affect cell migration, invasion and proliferation.

Hsieh et al., 2004 (NAR 32 893-901) reports on the use of 148 siRNA duplexes targeting 30 genes within the PI3K pathway.

US 2005/0272682 discloses siRNA complexes targeting a phosphoinositide 3-kinase (PI3K) signal transduction pathway.

In an exemplary aspect, the present invention provides highly efficient antisense oligonucleotides which target the PI3K pathway, specifically the PIK3CA mRNA, and in particular a new class of PIK3CA antagonists which have been selected based on LNA chemistry, and/or by the selection of particularly effective target sites on the PIK3CA mRNA.

### SUMMARY OF INVENTION

The present invention provides an oligomer having a contiguous nucleotide sequence of a total of 10 - 30 nucleotides, wherein said contiguous nucleotide sequence is at least 80% homologous to the reverse complement of a corresponding region of a mammalian PIK3CA gene or mRNA, wherein said oligomer comprises at least one LNA unit; and wherein the contiguous nucleotide sequence comprises at least 10 contiguous nucleotides which are 100% identical to a corresponding region of SEQ ID NO 115 or SEQ ID NO 7.

In another aspect, the present invention provides a conjugate comprising the oligomer according to the present invention, and at least one non-nucleotide or non-polynucleotide moiety covalently attached to said oligomer.

In a further aspect, the present invention provides a pharmaceutical composition comprising the oligomer according to the present invention, or the conjugate according to the present invention, and a pharmaceutically acceptable diluent, carrier, salt or adjuvant.

The present invention provides, in another aspect, an oligomer according to the present invention, or the conjugate according to the present invention, for use as a medicament, such as for the treatment of such as hyperproliferative diseases such as cancer.

The present invention provides, in a further aspect, the use of an oligomer according to the present invention, or a conjugate as defined herein, for the manufacture of a medicament for the treatment of such as hyperproliferative diseases such as cancer.

In a further aspect, the present invention provides an *in vitro* method for the inhibition of PIK3CA in a cell which is expressing PIK3CA, said method comprising administering an oligomer according to the present invention, or a conjugate according to the present invention to said cell so as to inhibit PIK3CA in said cell.

The description provides an oligomer of between 10 - 50, such as 10 - 30 nucleotides in length which comprises a contiguous nucleotide sequence of a total of between 10 - 30 nucleotides, wherein said contiguous nucleotide sequence is at least 80% (e.g., 85%, 90%, 95%, 98%, or 99%) homologous to a region corresponding to the reverse complement of a mammalian PIK3CA gene or mRNA, such as SEQ ID NO: 1 or naturally occurring variants thereof.

The description provides an oligomer of 10 - 50 monomers, such as 10 - 30 monomers which comprises a first region of 10 - 30 monomers, wherein the sequence of the first region is at least 80% (e.g., 85%, 90%, 95%, 98%, or 99%) identical to the reverse complement of a target region of a nucleic acid which encodes a mammalian PIK3CA, such as a mammalian PIK3CA gene or mRNA, such as a nucleic acid have the sequence set forth in SEQ ID NO: 1 or naturally occurring variants thereof.

The invention provides for a conjugate comprising the oligomer according to the invention, and at least one non-nucleotide or non-polynucleotide moiety ("conjugated moiety") covalently attached to said oligomer.

The invention provides for a pharmaceutical composition comprising an oligomer or a conjugate according to the invention, and a pharmaceutically acceptable diluent, carrier, salt or adjuvant.

The description provides an oligomer of between 10-50 nucleobases in length which comprises a contiguous nucleobase sequence of a total of between 10-50 nucleobases, wherein said contiguous nucleobase sequence is at least 80% identical to the reverse complement of a target region of a nucleic acid which encodes mammalian PIK3CA.

The description provides for the oligomer or the conjugate according to invention, for use as a medicament, such as for the treatment of hyperproliferative diseases such as cancer.

The invention provides for the use of an oligomer or the conjugate according to the invention, for the manufacture of a medicament for the treatment of hyperproliferative diseases such as cancer.

The description provides for a method of treating hyperproliferative diseases such as cancer, said method comprising administering an oligomer, a conjugate or a pharmaceutical composition according to the invention, to a patient suffering from, or likely to suffer from said hyperproliferative disease such as cancer.

The description provides for a method for the inhibition of both PIK3CA and beta-catenin in a cell which is expressing both PIK3CA and beta-catenin, said method comprising administering an oligomer, or a conjugate according to the invention to said cell so as to effect the inhibition of PIK3CA and beta-catenin in said cell. Suitably the oligomer which is capable of inhibiting or down-regulating both PI3CA and beta-catenin in a cell has significantly homology to, or is capable of hybriodising to the reverse complement of both target nucleic acids, such as an oligomer with a sequence of nucleobases of SEQ ID 82.

The description further provides for an oligomer according to the invention, for use in medicine.

The invention further provides for an oligomer according to the invention, for use for the treatment of one or more of the diseases referred to herein, such as a disease selected from the group consisting of: hyperproliferative diseases such as cancer,

Further provided are methods of down-regulating the expression of PIK3CA in a cell, cells or tissues comprising contacting said cells or tissues, *in vitro* or *in vivo*, with one or more of the oligomers, conjugates or compositions of the invention.

Also disclosed are methods of treating an animal, such as a non-human animal, or a human, suspected of having or being prone (susceptible) to a hyperproliferative disease such as cancer and/or diseases or conditions, associated with expression, or over-expression, of PIK3CA, by administering to said animal or human a therapeutically or prophylactically effective amount of one or more of the oligomers, conjugates or pharmaceutical compositions of the invention. Further, methods of using oligomers for the inhibition of expression of PIK3CA, and for treatment of diseases associated with activity of PIK3CA are provided.

The description provides for a method for treating a disease selected from the group consisiting of hyperproliferative diseases such as cancer; the method comprising administering an (effective amount of) one or more oligomers of the invention, conjugates, or pharmaceutical compositions thereof, to a patient in need thereof.

The description provides for a method of inhibiting or reducing the expression of PIK3CA (and optionally beta-catenin) in a cell or a tissue, such as by down-regulation, the method comprising the step of contacting said cell or tissue with an (effective amount of) one or more oligomers of the invention, conjugates, or pharmaceutical compositions thereof so that expression of PIK3CA (and optionally beta-catenin) is inhibited or reduced.

In some aspects, the oligomer of the description is selected from the group consisting of
5' AₛGₛ^{Me}CₛcₛaₛtₛtₛcₛaₛtₛtₛcₛcₛAₛ^{Me}Cₛ^{Me}C-3' (SEQ ID NO: 67); and
5'- TₛTₛAₛtₛtₛgₛtₛgₛcₛaₛtₛcₛtₛ^{Me}CₛAₛG-3' (SEQ ID NO: 77),
wherein uppercase letters denote beta-D-oxy-LNA monomers and lowercase letters denote DNA monomers, the subscript "s" denotes a phosphorothioate linkage, and ^{Me}C denotes a beta-D-oxy-LNA monomer containing a 5-methylcytosine base.

### BRIEF DESCRIPTION OF FIGURES

**Figure 1****.** Oligonucleotides presented in Table 3 were evaluated for their potential to knockdown the PIK3CA mRNA at concentrations of 0.8, 4 and 20 nM in MCF7 cells 24 hours after transfection using Real-time PCR. All results were normalised to GAPDH and inhibition of PIK3CA mRNA is shown as percent of mock-transfected control. Results shown are average of three independent experiments.
**Figure 2****.** Oligonucleotides presented in Table 3 were evaluated for their potential to knockdown the PIK3CA mRNA at concentrations of 0.8, 4 and 20 nM in PC3 cells 24 hours after transfection using Real-time PCR. All results were normalised to GAPDH and inhibition of PIK3CA mRNA is shown as percent of mock-transfected control. Results shown are average of three independent experiments.

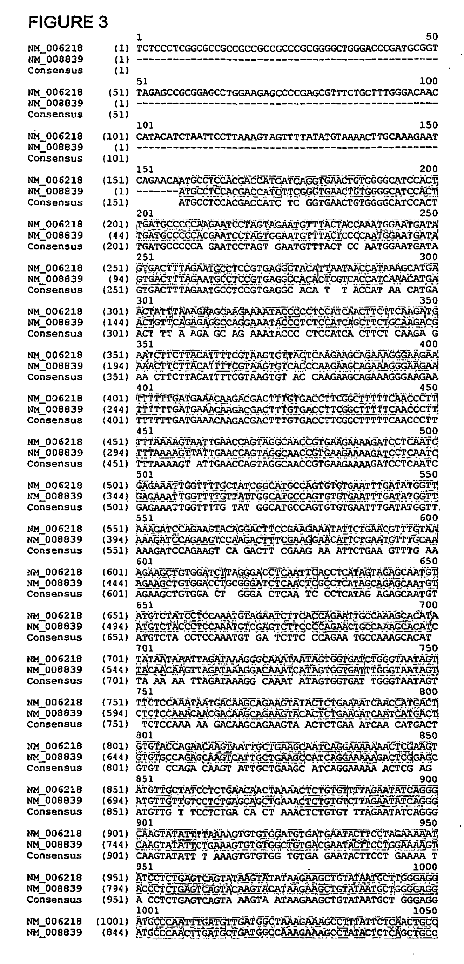

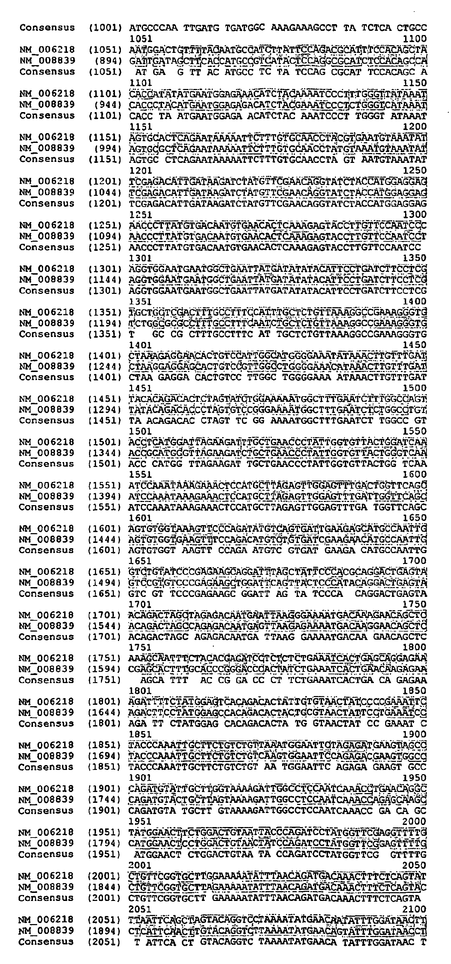

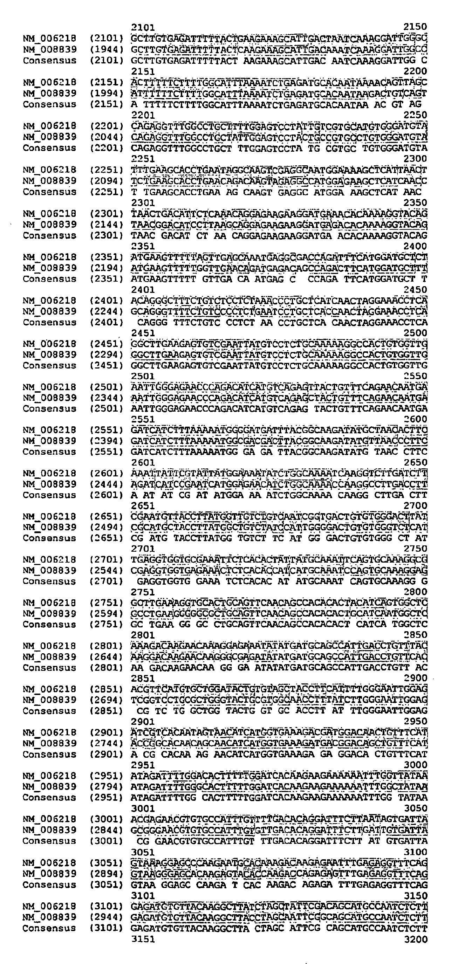

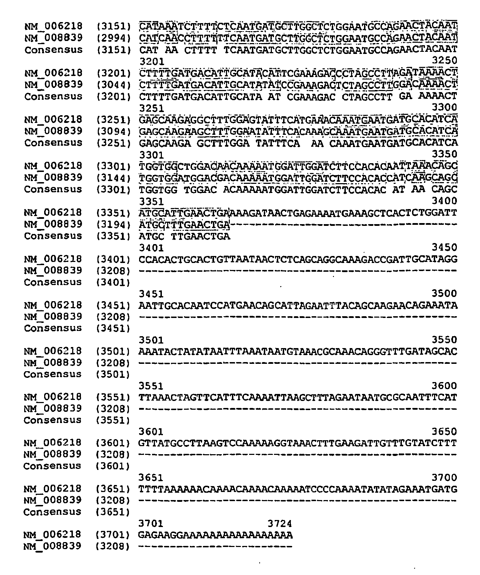

**Figure 3****.** Sequence alignment of the human PIK3CA mRNA sequence (Genbank Accession No: M_006218) and the mouse PIK3CA mRNA sequence (Genbank Accession No: NM_008839).

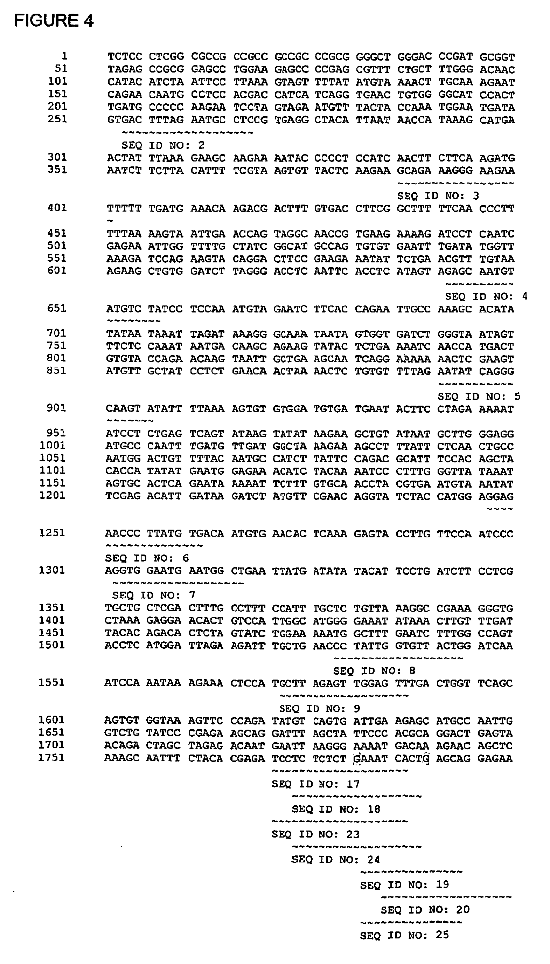

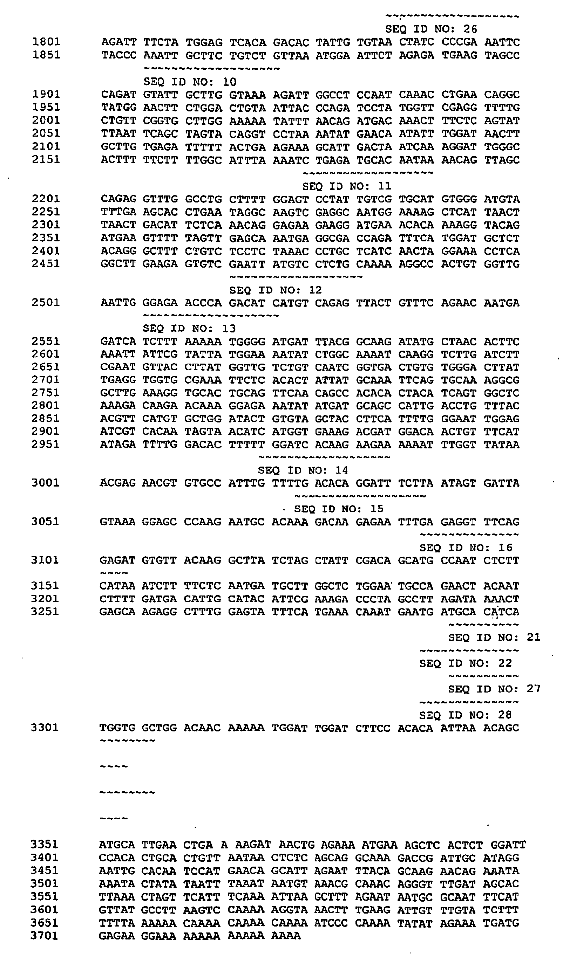

**Figure 4****.** Location of target sequences on the human PIK3CA mRNA sequence (Genbank Accession No: NM_006218). Positions marked in grey are mutation hotspots -1781, 1790 and 3297.

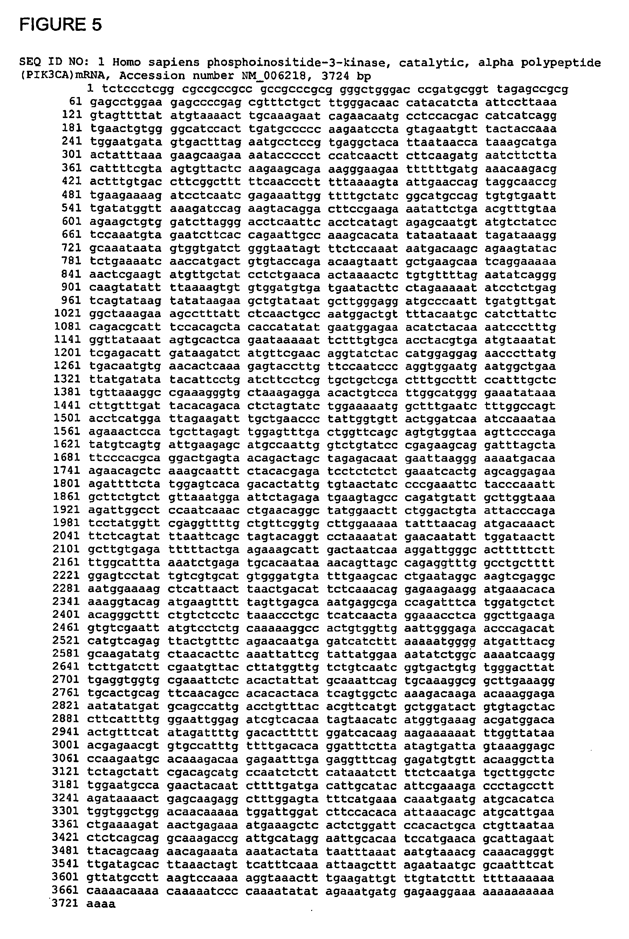

**Figure 5****.** SEQ ID NO: 1 *Homo sapiens* phosphoinositide-3-kinase, catalytic, alpha polypeptide (PIK3CA) mRNA, (Genbank Accession No: M_006218) 3724 bp.

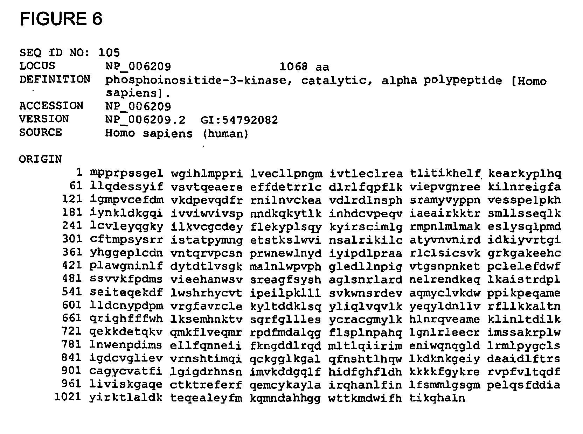

**Figure 6****.** SEQ ID NO 105. *Homo sapiens* phosphoinositide-3-kinase catalytic, alpha polypeptide protein sequence (PIK3CA) (Genbank Accession No: NP_006209).

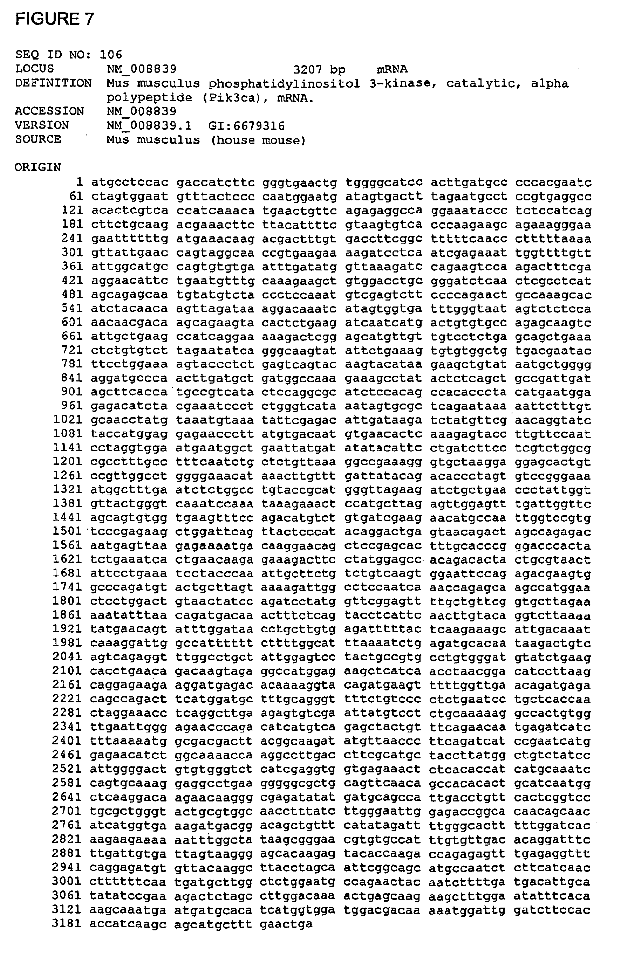

**Figure 7****.** SEQ ID NO 106. *Mus musculus* phosphatidylinositol 3-kinase, catalytic, alpha polypeptide (Pik3ca), mRNA. (Genbank Accession No: NM_008839).

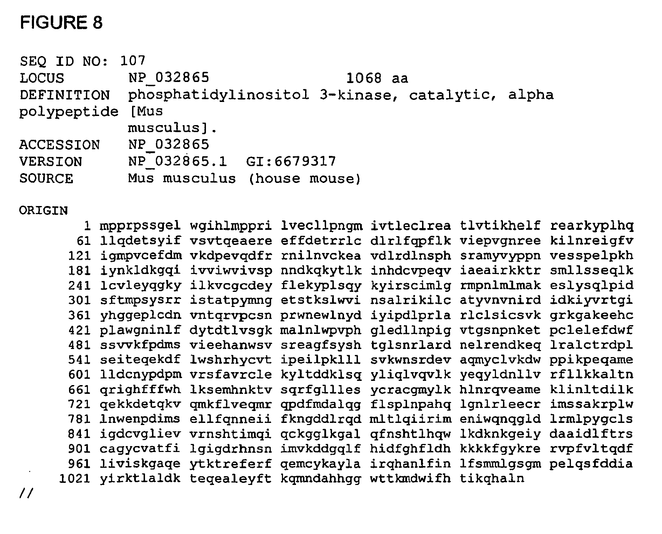

**Figure 8****.** SEQ ID NO 107. *Mus musculus* phosphoinositide-3-kinase catalytic, alpha polypeptide protein sequence (PIK3CA) Accession number NP_032865.

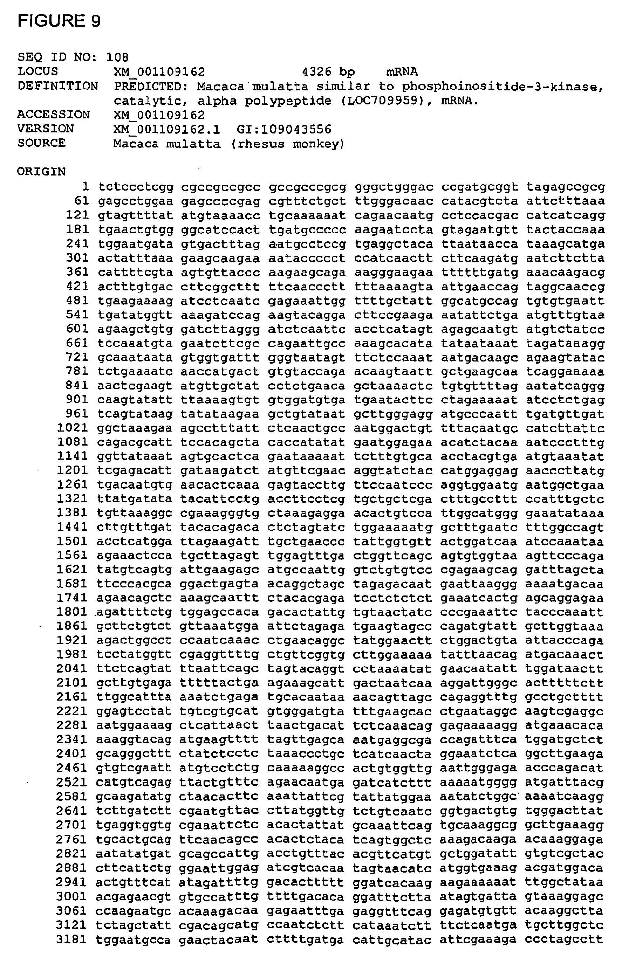

**Figure 9****.** SEQ ID NO 108. *Macaca mulatta* phosphatidylinositol 3-kinase, catalytic, alpha polypeptide (Pik3ca), mRNA. (Genbank Accession No: M_001109162).

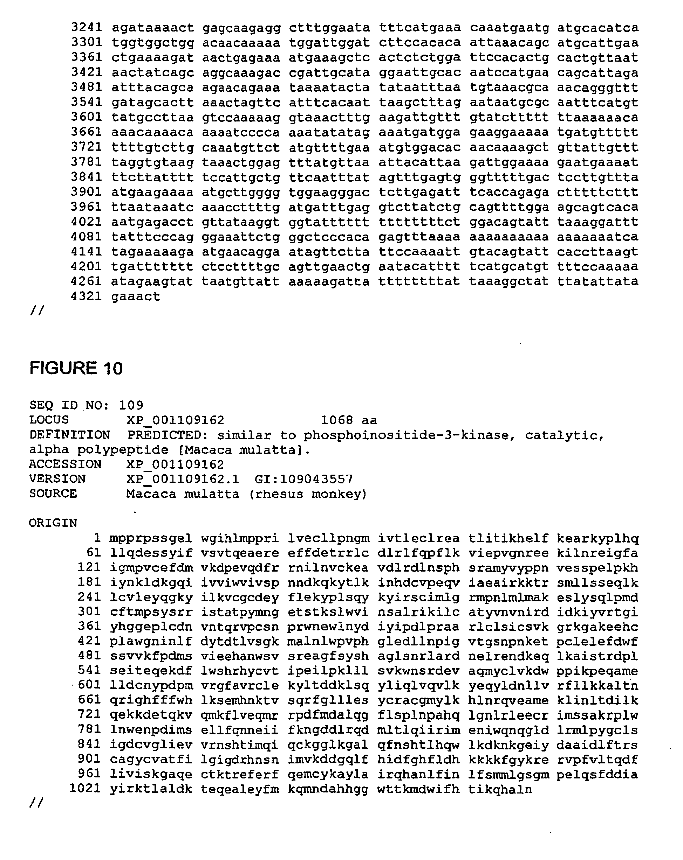

**Figure 10****.** SEQ ID NO 109. *Macaca mulatta* phosphoinositide-3-kinase catalytic, alpha polypeptide protein sequence (PIK3CA) (Genbank Accession No: XP_001109162).
**Figure 11****.** Cell proliferation assay (MTS assay) in MCF7 cells. Four independent experiments were performed. The results from the experiment best representing the average activity of each oligo is shown.
**Figure 12****.** Cell proliferation assay (MTS) in PC3 cells. Two independent experiments were performed. The results from the experiment best representing the average activity of each oligo is shown.
**Figure 13****.** Caspase 3/7 activity in PC3 cells after transfection with PIK3CA oligonucleotides.
Data are expressed as fold induction compared to mock.
**Figure 14****.** Cell proliferation assay (MTS) in HCT116 cells.
**Figure 15****.** Caspase 3/7 activity in HCT116 cells after transfection with PIK3CA oligos. Data are expressed as fold induction compared to mock.
**Figure 16****.** Plasma stability of PIK3CA oligonucleotides. The LNA oligonucleotides were incubated with mouse plasma at 37 °C and aliquots were taken at 0, 24, 48 and 120 h. The results are visualized by gel electrophoresis using an SDS-PAGE gel.
**Figure 17****.** Tₘ determination of PIK3CA oligonucleotides hybridised to a target region of a target nucleic acid. Bold, uppercase letters with a superscript "o" to the right represent β-D-oxy LNA monomers. ^{M}C represent LNA monomers with 5-methylcytosine bases. Subscript "s" represents a phosphorothioate linkage. Lowercase letters represent DNA monomers.
**Figure 18****.** Down-regulation of PIK3CA protein and pAkt in A549 cells
**Figure 19****.** Down-regulation of PIK3CA protein and pAkt in 15PC3 cells
**Figure 20****.** Analysis of knock down of PIK3CA mRNA in liver.
**Figure 21***. In vivo* knock down of PIK3CA mRNA in mouse liver. Data are expressed as % down-regulation compared to saline (100%) ± stdev. There were 5 animals in each group.
**Figure 22****.** The most potent oligonucleotides were evaluated for their potential to knock down the PIK3CA mRNA at concentrations of 0.04 nM, 0.2 nM, 0.8 nM, 4 nM, 10 nM and 20 nM in PC3 cells 24 hours after transfection using Real-time PCR. All results were normalised to GAPDH and inhibition of PIK3CA mRNA is shown as percent of mock-transfected control. Results shown are the average of two independent experiments.
**Figure 23****.** The most potent oligonucleotides were evaluated for their potential to knock down the PIK3CA mRNA at concentrations of 0.04 nM, 0.2 nM, 0.8 nM, 4 nM, 10 nM and 20 nM in MCF7 cells 24 hours after transfection using Real-time PCR. All results were normalised to GAPDH and inhibition of PIK3CA mRNA is shown as percent of mock-transfected control. Results shown are the average of two independent experiments.

### DETAILED DESCRIPTION OF INVENTION

### The Oligomer

The present description employs oligomeric compounds (referred herein as oligomers), for use in modulating the function of nucleic acid molecules encoding mammalian PIK3CA, such as the PIK3CA nucleic acid shown in SEQ ID 1, and naturally occurring variants of such nucleic acid molecules encoding mammalian PIK3CA. The term "oligomer" in the context of the present invention, refers to a molecule formed by covalent linkage of two or more nucleotides (*i.e*. an oligonucleotide). Herein, each single nucleotide, such as the nucleotides present in the oligomer of the invention, may also be referred to as a "monomer" or "unit". In some embodiments, the oligomer may consist or comprises of a contiguous nucleotide sequence of between 10 - 30 nucleotides in length (*i.e*. comprises or consists of from 10 - 30 covalently linked monomers).

In various embodiments, the compounds according to the description are linear molecules or are synthesised as a linear molecule. The oligomer, in such embodiments, is a single stranded molecule, and typically does not comprise short regions of, for example, at least 3, 4 or 5 contiguous nucleotides, which are complementary to another region within the same oligomer (*i.e*. duplexes) - in this regards, In some embodiments, the oligomer is not (essentially) double stranded. In some embodiments, the oligomer is essentially not double stranded, such as is not a siRNA. In various embodiments, the oligomer of the description may consist entirely of the contiguous nucleotide region. Thus, the oligomer is not substantially self-complementary. siRNAs comprise of 2 complementary short RNA (or equivalent nucleobase units) sequences, such as between 21 and 23nts long, with, typically a 2nt 3' overhang on either end. In order to enhance *in vivo* uptake, the siRNAs may be conjugated, such as conjugated to a sterol, such as a cholesterol group (typically at the 3' or 5' termini of one or both of the strands).

### The Target

Suitably the oligomer of the invention is capable of down-regulating expression of the PIK3CA gene. In this regards, the oligomer of the invention can effect the inhibition of PIK3CA, typically in a mammalian such as a human cell. In some embodiments, the oligomers of the invention bind to the target nucleic acid and effect inhibition of expression of at least 10% or 20% compared to the normal expression level, more preferably at least a 30%, 40%, 50%, 60%, 70%, 80%, 90% or 95% inhibition as compared to the normal expression level (such as immediatley prior to dosing of the oligomer). In some embodiments, such modulation is seen when using between 0.04 and 25nM, such as between 0.8 and 20nM concentration of the compound of the invention. In the same or a different embodiment, the inhibition of expression is less than 100%, such as less than 98% inhibition, less than 95% inhibition, less than 90% inhibition, less than 80% inhibition, such as less than 70% inhibition. Modulation of expression level may be determined by measuring protein levels, e.g. by the methods such as SDS-PAGE followed by western blotting using suitable antibodies raised against the target protein. Alternatively, modulation of expression levels can be determined by measuring levels of mRNA, e.g. by northern blotting or quantitative RT-PCR. When measuring via mRNA levels, the level of down-regulation when using an appropriate dosage, such as between 0.04 and 25nM, such as between 0.8 and 20nM concentration, is, in some embodiments, typically to a level of between 10-20% the normal levels in the absence of the compound of the invention.

The PIK3CA gene is likely to be a naturally occurring gene, and therefore in relation to the human gene it will have the sequence of SEQ ID NO 1 or a natural variant thereof, whether a "silent" (e.g. not associated with a disease or other alteration in PIK3CA kinase properties) or "functional" (e.g. disease-associated) variant. The target sequence from the PIK3CA gene will be DNA or RNA, preferably mRNA. The oligomer hybridizes, such as specifically hybridises or hybridises under stringent conditions, to a single stranded nucleic acid molecule having the sequence of a portion of SEQ ID NO: 1.

It will be noted that, in some embodiment, some oligomers are capable of targeting both PIK3CA and beta-catenin. Therefore in some embodiments, the target may be PIK3CA and beta-catenin.

The description therefore, in some embodiments, provides a method of down-regulating or inhibiting the expression of PIK3CA protein and/or mRNA in a cell which is expressing PIK3CA protein and/or mRNA, said method comprising administering or contacting the oligomer or conjugate according to the invention (suitably in an effective amount) to said cell to down-regulating or inhibiting the expression of PIK3CA protein and/or mRNA in said cell. Suitably the cell is a mammalian cell such as a human cell. The administration may occur, in some embodiments, *in vitro.* The administration may occur in some embodiments, *in vivo.*

The term "target nucleic acid", as used herein refers to the DNA or RNA encoding mammalian PIK3CA polypeptide, such as human PIK3CA, such as SEQ ID NO: 1. PIK3CA encoding nucleic acids or naturally occurring variants thereof, and RNA nucleic acids derived therefrom, preferably mRNA, such as pre-mRNA, although preferably mature mRNA. In some embodiments, for example when used in research or diagnostics the "target nucleic acid" may be a cDNA or a synthetic oligonucleotide derived from the above DNA or RNA nucleic acid targets. The oligomer according to the invention is preferably capable of hybridising to the target nucleic acid. It will be recognised that SEQ ID NO: 1 is a cDNA sequences, and as such, corresponds to the mature mRNA target sequence, although uracil is replaced with thymidine in the cDNA sequences.

The mammalian PIK3CA kinase is preferably selected from the group consisting of human or mouse PIK3CA kinase. Preferably the mammalian PIK3CA kinase is human PIK3CA kinase, such as the PIK3CA Kinase encoded by the nucleic acid as shown in SEQ ID NO 1. The nucleic acid which encodes the mammalian PIK3CA kinase is, in a preferable embodiment, the human PIK3CA kinase cDNA sequence is shown as SEQ ID NO 1 and/or the mouse PIK3CA kinase cDNA sequence is shown as SEQ ID NO 106, or allelic variants thereof, such as the PIK3CA mutations listed on
http://www.sanger.ac.uk/perl/genetics/CGP/cosmic?action=gene&ln=PIK3CA.

The term "naturally occurring variant thereof" refers to variants of the PIK3CA polypeptide of nucleic acid sequence which exist naturally within the defined taxonomic group, such as mammalian, such as mouse, monkey, and preferably human. Typically, when referring to "naturally occurring variants" of a polynucleotide the term also may encompass any allelic variant of the PIK3CA encoding genomic DNA which are found at the Chromosome 3 [Chr 3: 180.35 - 180.44 M bp] specifically [chr3:180,349,005-180,435,191 bp](NCBI reference sequence annotation) (3q26.3) by chromosomal translocation or duplication, and the RNA, such as mRNA derived therefrom. "Naturally occurring variants" may also include variants derived from alternative splicing of the PIK3CA mRNA. When referenced to a specific polypeptide sequence, e.g., the term also includes naturally occurring forms of the protein which may therefore be processed, e.g. by co- or post-translational modifications, such as signal peptide cleavage, proteolytic cleavage, glycosylation, etc. Variant sequences, may have at least 60%, more preferably at least 70%, more preferably at least 80%, more preferably at least 85%, more preferably at least 90%, more preferably at least 95% sequence homology to a target sequence in PIK3CA. Typically, an oligomer of the invention corresponding to said variant sequences is still capable of down-regulating PIK3CA.

Particularly relevant variants of PIK3CA which may be targeted by the oligomers of the invention are spontaneous point mutations which are related to hyperproliferative diseases such as cancer - in this regards, point mutations which cause amino acid substitutions at positions E542, E545 or H1047, such as point mutations which cause the substitutions E542K, E545K and H1047R are particularly relevant. By designing oligomers which target PIK3CA mRNA which comprises such a spontaneous mutation, oligomers can be created which have a preferential activity (down-regulation) on the mRNA of variant (mutated) form of PIK3CA, which, as in the case of point mutations which cause amino acid substitutions at positions E542, E545 or H1047 are associated with a cancer phenotype.

In some embodiments, the PIK3CA gene or mRNA target comprises a single base substitution at a position which corresponds to position 1781, 1790 or 3297 of SEQ ID NO 1. In such embodiments, the nucleotide present at position 1781 may be other than G, such as A, C or T/U, the nucleotide present at position 1790 may be other than G, such as A, C or T/U, or the nucleotide present at position 3297 may be other than A, such as G, C or T/U. (T refers to DNA target, U to mRNA target).

In some embodiments, the contiguous nucleotide sequence of the oligomer according to the description may comprise a nucleobase which corresponds to position 1781 of SEQ ID NO 1, wherein the nucleobases which corresponds to position 1781 is not a C, such is selected from A, G or T. Suitably, in some embodiments, the remaining nucleobases of such a contiguous nucleotide sequence may be fully complementary to the corresponding region of SEQ ID NO 1.

In some embodiments, the contiguous nucleotide sequence of the oligomer according to the description may comprise a nucleobase which corresponds to position 1790 of SEQ ID NO 1, wherein the nucleobases which corresponds to position 1790 is not a C, such is selected from A, G or T. Suitably, in some embodiments, the remaining nucleobases of such a contiguous nucleotide sequence may be fully complementary to the corresponding region of SEQ ID NO 1.

In some embodiments, the contiguous nucleotide sequence of the oligomer according to the description may comprise a nucleobase which corresponds to position 3297 of SEQ ID NO 1, wherein the nucleobases which corresponds to position 3297 is not a T, such is selected from A, G or C. Suitably, some embodiments, the remaining nucleobases of such a contiguous nucleotide sequence may be fully complementary to the corresponding region of SEQ ID NO 1.

### Oligomer Sequences

The oligomers comprise or consist of a contiguous nucleotide sequence which corresponds to the reverse complement of a nucleotide sequence present in SEQ ID NO: 1. Thus, the oligomer can comprise or consist of, or a sequence selected from the group consisting of SEQ ID NOS: 2 - 16 or 17 - 28 or 110 -124 or 125 -136 or 149 -160, wherein said oligomer (or contiguous nucleotide portion thereof) may optionally have one, two, or three mismatches against said selected sequence.

The oligomer may comprise or consist of a contiguous nucleotide sequence which is fully complementary (perfectly complementary) to the equivalent region of a nucleic acid which encodes a mammalian PIK3CA (e.g., SEQ ID NO: 1). Thus, the oligomer can comprise or consist of an antisense nucleotide sequence.

However, in some embodiments, the oligomer may tolerate 1, 2, 3, or 4 (or more) mismatches, when hybridising to the target sequence and still sufficiently bind to the target to show the desired effect, i.e. down-regulation of the target. Mismatches may, for example, be compensated by increased length of the oligomer nucleotide sequence and/or an increased number of nucleotide analogues, such as LNA, present within the nucleotide sequence.

In some embodiments, the contiguous nucleotide sequence comprises no more than 3, such as no more than 2 mismatches when hybridizing to the target sequence, such as to the corresponding region of a nucleic acid which encodes a mammalian PIK3CA.

In some embodiments, the contiguous nucleotide sequence comprises no more than a single mismatch when hybridizing to the target sequence, such as the corresponding region of a nucleic acid which encodes a mammalian PIK3CA.

The nucleotide sequence of the oligomers of the description or the contiguous nucleotide sequence is preferably at least 80% homologous to a corresponding sequence selected from the group consisting of SEQ ID NOS: 2 - 16 or 17 - 28 or 110 - 124 or 125 - 136 or 149 -160 , such as at least 85%, at least 90%, at least 91%, at least 92%, at least 93%, at least 94%, at least 95%, at least 96% homologous, at least 97%, at least 98%, at least 99%, such as 100% homologous (identical).

The nucleotide sequence of the oligomers of the description or the contiguous nucleotide sequence is preferably at least 80% homologous to the reverse complement of a corresponding sequence present in SEQ ID NO: 1, such as at least 85%, at least 90%, at least 91%, at least 92%at least 93%, at least 94%, at least 95%, at least 96% homologous, at least 97%, at least 98%, at least 99%, such as 100% homologous (identical).

The nucleotide sequence of the oligomers of the invention or the contiguous nucleotide sequence is preferably at least 80% complementary to a sub-sequence present in SEQ ID NO: 1, such as at least 85%, at least 90%, at least 91 %, at least 92%, at least 93%, at least 94%, at least 95%, at least 96% complementary, at least 97%, at least 98%, at least 99%, such as 100% complementary (perfectly complementary).

In some embodiments the oligomer (or contiguous nucleotide portion thereof) is selected from, or comprises, one of the sequences selected from the group consisting of SEQ ID NOS: 2, 3, 4, 5, 6, 7, 8, 9, 10, 11, 12, 13, 14, 15, 16, 17, 18, 19, 20, 21, 22, 23, 24, 25, 26 and 28 or a sub-sequence of at least 10 contiguous nucleotides thereof, wherein said oligomer (or contiguous nucleotide portion thereof) may optionally comprise one, two, or three mismatches when compared to the sequence.

In some embodiments the oligomer (or contiguous nucleotide portion thereof) is selected from, or comprises, one of the sequences selected from the group consisting of SEQ ID NOS: 110, 111, 112, 113, 114, 115, 116, 117, 118, 119, 120, 121, 122, 123, 124, 125, 126, 127, 28, 129, 130, 131, 132, 133, 134, 135 and 136 or a sub-sequence of at least 10 contiguous nucleotides thereof, wherein said oligomer (or contiguous nucleotide portion thereof) may optionally comprise one, two, or three mismatches when compared to the sequence.

In some embodiments the oligomer (or contiguous nucleotide portion thereof) is selected from, or comprises, one of the sequences selected from the group consisting of SEQ ID NOS: 149, 150, 151, 152, 153, 154, 155, 156, 157, 158, 159 and 160 or a sub-sequence of at least 10 contiguous nucleotides thereof, wherein said oligomer (or contiguous nucleotide portion thereof) may optionally comprise one, two, or three mismatches when compared to the sequence.

In some embodiments the sub-sequence may consist of 11, 12, 13, 14, 15, 16, 17, 18, 19, 20, 21. 22, 23, 24, 25, 26, 27, 28, or 29 contiguous nucleotides, such as between 12 -22, such as between 12-18 nucleotides. Suitably, in some embodiments, the sub-sequence is of the same length as the contiguous nucleotide sequence of the oligomer of the invention.

However, it is recognised that, in some embodiments the nucleotide sequence of the oligomer may comprise additional 5' or 3' nucleotides, such as, independently, 1, 2, 3, 4 or 5 additional nucleotides 5' and/or 3', which are non-complementary to the target sequence. In this respect the oligomer of the invention, may, in some embodiments, comprise a contiguous nucleotide sequence which is flanked 5' and or 3' by additional nucleotides. In some embodiments the additional 5' or 3' nucleotides are naturally occurring nucleotides, such as DNA or RNA. In some embodiments, the additional 5' or 3' nucleotides may represent region D as referred to in the context of gapmer oligomers herein.

In some embodiments the oligomer (or contiguous nucleotide portion thereof) is selected from, or comprises, one of the sequences selected from the group consisting of SEQ ID NO 111, 112, 114, 115, 116, 118, 119, 122, 131, 132 and 136, or a sub-sequence of at least 10 contiguous nucleotides thereof, such as 11, 12, 13, 14, 15 or 16 contiguous nucleotides thereof, wherein said oligomer (or contiguous nucleotide portion thereof) may optionally comprise one, two, or three mismatches against said selected sequence.

In some embodiments the oligomer (or contiguous nucleotide portion thereof) is selected from, or comprises, one of the sequences selected from the group consisting of SEQ ID NO 149, 150, 153, 154, 157, and 158; or a sub-sequence of at least 10 contiguous nucleotides thereof, such as 11, 12, 13, 14, 15 or 16 contiguous nucleotides thereof, wherein said oligomer (or contiguous nucleotide portion thereof) may optionally comprise one, two, or three mismatches against said selected sequence.

In some embodiments the oligomer (or contiguous nucleotide portion thereof) is selected from, or comprises, one of the sequences selected from the group consisting of SEQ ID NO 3, 4, 6, 7, 8, 10, 11, 14, 23, 24 and 28, or a sub-sequence of at least 10 contiguous nucleotides thereof, such as 11, 12, 13, 14, 15 or 16 contiguous nucleotides thereof, wherein said oligomer (or contiguous nucleotide portion thereof) may optionally comprise one, two, or three mismatches against said selected sequence.

In some embodiments the oligomer (or contiguous nucleotide portion thereof) is selected from, or comprises, one of the sequences selected from the group consisting of SEQ ID NO 151, 152, 155, 156, 159 and 160; or a sub-sequence of at least 10 contiguous nucleotides thereof, such as 11, 12, 13, 14, 15 or 16 contiguous nucleotides thereof, wherein said oligomer (or contiguous nucleotide portion thereof) may optionally comprise one, two, or three mismatches against said selected sequence.

In some embodiments the oligomer (or contiguous nucleotide portion thereof) is selected from, or comprises, one of the sequences selected from the group consisting of SEQ ID NO: 57, 60, 64, 67, 70, 74, 77, 82, 87, 90 and 96, or a sub-sequence of at least 10 contiguous nucleotides thereof, such as 11, 12, 13, 14, 15 or 16 contiguous nucleotides thereof, wherein said oligomer (or contiguous nucleotide portion thereof) may optionally comprise one, two, or three mismatches against said selected sequence.

In some embodiments the oligomer (or contiguous nucleotide portion thereof) is selected from, or comprises, one of the sequences selected from the group consisting of SEQ ID NO: 87, 90 and 96, or a sub-sequence of at least 10 contiguous nucleotides thereof, such as 11, 12, 13, 14, 15 or 16 contiguous nucleotides thereof, wherein said oligomer (or contiguous nucleotide portion thereof) may optionally comprise one, two, or three mismatches against said selected sequence.

In some embodiments the oligomer (or contiguous nucleotide portion thereof) is selected from, or comprises, one of the sequences selected from the group consisting of SEQ ID NO: 99, 100, 101, 102, 103, or 104, or a sub-sequence of at least 10 contiguous nucleotides thereof, such as 11, 12, 13, 14, 15 or 16 contiguous nucleotides thereof, wherein said oligomer (or contiguous nucleotide portion thereof) may optionally comprise one, two, or three mismatches against said selected sequence.

In some embodiments the oligomer (or contiguous nucleotide portion thereof) is selected from, or comprises, one of the sequences selected from the group consisting of SEQ ID NO: 99, 100 or 104, or a sub-sequence of at least 10 contiguous nucleotides thereof, such as 11, 12, 13, 14, 15 or 16 contiguous nucleotides thereof, wherein said oligomer (or contiguous nucleotide portion thereof) may optionally comprise one, two, or three mismatches against said selected sequence.

In some embodiments the oligomer according to the description consists or comprises of a nucleotide sequence according to SEQ ID NO 2, such as SEQ ID NO 56, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 3, such as SEQ ID 57, 58 or 59, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 4, such as SEQ ID NO 60, 61 and 62, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 5, such as SEQ ID NO 63, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 6, such as SEQ ID NO 64, 65 and 66, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 7, such as SEQ ID NO 67, 68 or 69, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 8, such as SEQ ID 70, 71 or 72, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 9, such as SEQ ID 73, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 10, such as SEQ ID 74, 75 or 76, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 11, such as SEQ ID 77, 78 or 79, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 12, such as SEQ ID 80, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 13, such as SEQ ID 81 or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 14, such as SEQ ID 82, 83, or 84, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 15, such as SEQ ID 85, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 16, such as SEQ ID 86, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 17, such as SEQ ID 87, 88 or 89, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 18, such as SEQ ID 90, 91 or 92, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 19, such as SEQ ID 93, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 20, such as SEQ ID 94, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 21, such as SEQ ID 95, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 22, such as SEQ ID 96, 97 or 98, or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 23, such as SEQ ID 99, 137 or 138 or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 24, such as SEQ ID 100, 139 or 140 or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 25, such as SEQ ID 101, 141 or 142 or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 26, such as SEQ ID 102, 143 or 144 or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 27, such as SEQ ID 103, 145 or 146 or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence according to SEQ ID 28, such as SEQ ID 104, 147 or 148 or a sub-sequence of at least 10 contiguous nucleotides thereof, such as 11, 12, 13, 14, 15 or 16 contiguous nucleotides thereof.

In some embodiments the oligomer according to the description consists or comprises of a nucleotide sequence selected from the group consisting of SEQ ID 149, 150, 151, 152, 153, 154, 155, 156, 157, 158, 159 or 160 or a sub-sequence of at least 10 contiguous nucleotides thereof, such as 11, 12, 13, 14, 15 or 16 contiguous nucleotides thereof.

| | |
|---|---|
| SEQ ID NO: 149* | GCTCAGTGATTTXAGAGAGAGGAT |
| SEQ ID NO: 150* | TTTCTCCTGCTXAGTGATTTCAGA |
| SEQ ID NO: 151* | GATTTXAGAGAGAGGA |
| SEQ ID NO: 152* | AGTGATTTXAGAGAGA |
| SEQ ID NO: 153* | ATCTTTCTCCTGCTXAGTGATTTC |
| SEQ ID NO: 154* | CAGTGATTTXAGAGAGAGGATCTC |
| SEQ ID NO: 155* | TCCTGCTXAGTGATTT |
| SEQ ID NO: 156* | TTCTCCTGCTXAGTGA |
| SEQ ID NO: 157† | AGCCACCATGAXGTGCATCATTCA |
| SEQ ID NO: 158† | TCCAGCCACCATGAXGTGCATCAT |
| SEQ ID NO: 159† | GCCACCATGAXGTGCA |
| SEQ ID NO: 160† | ACCATGAXGTGCATCA |

| | |
|---|---|
| *Where X is either a nucleobases other than C, such as A, G or T, preferably T. † Where X is either a nucleobases other than T, such as A, G or C, preferably C. | |

In some embodiments, the oligomer according to the description consists or comprises a nucleobase sequence selected from the following (5' - 3'):
(G)(A)(T)TTXAG(A)(G)(A)(G), wherein the nucleobases in brackets are optional, and wherein X is a nucleobases other than C, such as A, G or T, preferably T. See SEQ ID NOs 149 - 152, for example.
(C)(T)(G)CTXAG(T)(G)(G), wherein the nucleobases in brackets are optional, and wherein X is a nucleobases other than C, such as A, G or T, preferably T. See SEQ ID NOs 153 -156, for example.
(C)(A)(T)GAXGT(G)(C)(A), wherein the nucleobases in brackets are optional, and wherein X is a nucleobases other than T, such as A, G or C, preferably C. See SEQ ID NOs 157 - 160, for example.

In some embodiments in the above embodiments X is a nucleobases in region B (gapmer or shortmer), such as a DNA nucleotide. Suitably X may be positioned as the middle nucleobases of region B, or has at least one, two, three or four other nucleobases of region B either 5' and/or at least one, two, three or four other nucleobases of region B 3' to X. Therefore, in some embodiments, X is not part of regions A, C or, where present D, or in some embodiments, is not a nucleobase of region B which is immediately adjacent to regions A or C.

However, it is considered that, in some embodiments, the oligomer or contiguous nucleobase sequence may comprise one or more mismatches when compared to the nucleic acid which encodes the PIK3CA kinase polypeptide.

The oligomer of the description, preferably, does not comprise more than four, such as not more than three, such as not more than two, such as not more than one mismatch, with the corresponding region of the sequence present in the nucleic acid which encodes the PIK3CA kinase polypeptide, such as SEQ ID NO 1 or naturally occurring variants thereof, such as a allelic variants and spontaneous variants, such as the nucleic acids which encode the E524K, E545K and H1047R mutants, such as the G1781A, G1790A or A3297G spontaneous mutants of SEQ ID NO 1 (the residue letter before the position refers to the WT version, the letter after refers to the preferred mutant residue).

In one aspect the description provides a method for the preparation of an oligomer for the down-regulation of a target mRNA associated with cancer cells, such as a PIK3CA mRNA target, said method comprising the steps of:
a) Identifying a single point mutation present in the target mRNA associated with cancer, wherein the single point mutation is present in said cancer cells but is absent in non-cancer cells.
b) Preparing an oligomers which is consists or comprises of a contiguous nucleobase sequence which is complmentary to said target mRNA associated with cancer, wherein said contiguous nucleobase sequence corresponds to a region of the target mRNA which comprises said single point mutation.

Suitably the single point mutation is associated to indicated with said cancer, such as the PIK3CA point mutations referred to herein.

Suitably the oligomer is a gapmer or shortmer oligonucleotide as described herein (although not limited necessarily to the oligomers targeting PIK3CA). As referred to herein, in some embodiments, the nucleobase residue present in such an oligomer which corresponds to the single point mutation is in region B.

In one aspect, it may be advantageous to specifically target regions of the mRNA target which comprise a point mutation associated to a cancer phenotype as it allows the design of oligomers which have a lower efficacy against the target in non-cancerous cells, as to those in cancerous cells, thereby allowing a differentiation between the effective treatment of cancerous cells and (reduced) negative effects on non-cancerous cells.

When determining "homology" between the oligomers of the invention (or contiguous nucleotide sequence) and the nucleic acid which encodes the mammalian PIK3CA or the reverse complement thereof, such as those disclosed herein, the determination of homology may be made by a simple alignment with the corresponding nucleotide sequence of the compound of the invention and the corresponding region of the nucleic acid which encodes the mammalian PIK3CA (or target nucleic acid), or the reverse complement thereof, and the homology is determined by counting the number of bases which align and dividing by the total number of contiguous nucleotides in the compound of the invention, and multiplying by 100. In such a comparison, if gaps exist, it is preferable that such gaps are merely mismatches rather than areas where the number of nucleotides within the gap differ between the nucleotide sequence of the invention and the target nucleic acid.

The terms "corresponding to" and "corresponds to" refer to the comparison between the nucleotide sequence of the oligomer or contiguous nucleotide sequence (a first sequence) and the equivalent contiguous nucleotide sequence of a further sequence selected from either i) a sub-sequence of the reverse complement of the nucleic acid target, such as the mRNA which encodes the PIK3CA protein, such as SEQ ID NO: 1, and/or ii) the sequence of nucleotides provided herein such as the group consisting of SEQ ID NOS: 2 - 16 or 17 - 28 or 110 - 124 or 125 -136 or 149 - 160, or sub-sequence thereof. Nucleotide analogues are compared directly to their equivalent or corresponding nucleotides. A first sequence which corresponds to a further sequence under i) or ii) typically is identicial to that sequence over the length of the first sequence (such as the contiguous nucleotide sequence) or, as described herein may, in some embodiments, is at least 80% homologous to a corresponding sequence, such as at least 85%, at least 90%, at least 91%, at least 92%at least 93%, at least 94%, at least 95%, at least 96% homologous, such as 100% homologous (identical).

The terms "corresponding nucleotide analogue" and "corresponding nucleotide" are intended to indicate that the nucleotide in the nucleotide analogue and the naturally occurring nucleotide are identical. For example, when the 2-deoxyribose unit of the nucleotide is linked to an adenine, the "corresponding nucleotide analogue" contains a pentose unit (different from 2-deoxyribose) linked to an adenine.

### Length

The oligomers comprise or consist of a contiguous nucleotide sequence of a total of between 10, 11, 12, 13, 14, 15, 16, 17, 18, 19, 20, 21, 22, 23, 24, 25, 26, 27, 28, 29 or 30 contiguous nucleotides in length.

In some embodiments, the oligomers comprise or consist of a contiguous nucleotide sequence of a total of between 10 - 22, such as 12 -18, such as 13 - 17 or 12 - 16, such as 13, 14, 15, 16 contiguous nucleotides in length.

In some embodiments, the oligomers comprise or consist of a contiguous nucleotide sequence of a total of 10, 11, 12, 13, or 14 contiguous nucleotides in length.

In some embodiments, the oligomer according to the invention consists of no more than 22 nucleotides, such as no more than 20 nucleotides, such as no more than 18 nucleotides, such as 15, 16 or 17 nucleotides. In some embodiments the oligomer of the invention comprises less than 20 nucleotides.

### Nucleotide analogues

The term "nucleotide" as used herein, refers to a glycoside comprising a sugar moiety, a base moiety and a covalently linked phosphate group and covers both naturally occurring nucleotides, such as DNA or RNA, preferably DNA, and non-naturally occurring nucleotides comprising modified sugar and/or base moieties, which are also referred to as "nucleotide analogues" herein.

Non-naturally occurring nucleotides include nucleotides which have modified sugar moieties, such as bicyclic nucleotides or 2' modified nucleotides, such as 2' substituted nucleotides.

"Nucleotide analogues" are variants of natural nucleotides, such as DNA or RNA nucleotides, by virtue of modifications in the sugar and/or base moieties. Analogues could in principle be merely "silent" or "equivalent" to the natural nucleotides in the context of the oligonucleotide, *i.e*. have no functional effect on the way the oligonucleotide works to inhibit target gene expression. Such "equivalent" analogues may nevertheless be useful if, for example, they are easier or cheaper to manufacture, or are more stable to storage or manufacturing conditions, or represent a tag or label. Preferably, however, the analogues will have a functional effect on the way in which the oligomer works to inhibit expression; for example by producing increased binding affinity to the target and/or increased resistance to intracellular nucleases and/or increased ease of transport into the cell. Specific examples of nucleoside analogues are described by *e.g*. Freier & Altmann; Nucl. Acid Res., 1997, 25, 4429-4443 and Uhlmann; Curr. Opinion in Drug Development, 2000, 3(2), 293-213, and in Scheme 1:

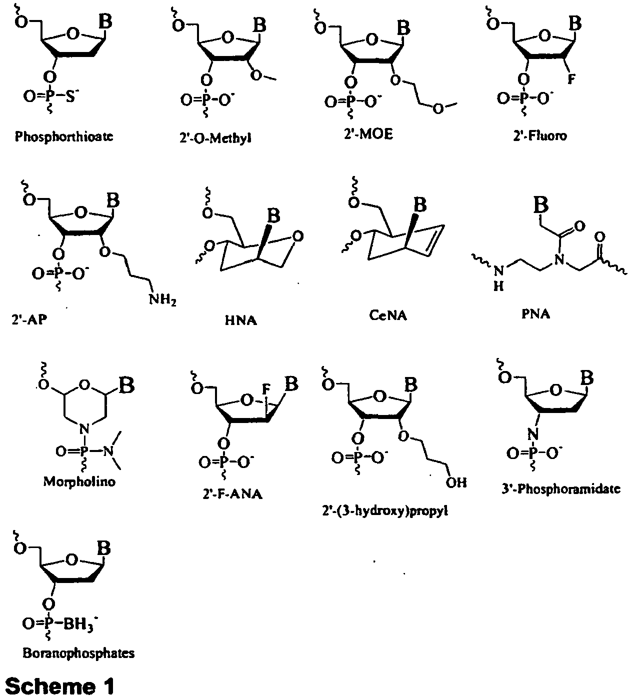

The oligomer may thus comprise or consist of a simple sequence of natural occurring nucleotides - preferably 2'-deoxynucleotides (referred to here generally as "DNA"), but also possibly ribonucleotides (referred to here generally as "RNA"), or a combination of such naturally occurring nucleotides and one or more non-naturally occurring nucleotides; *i.e*. nucleotide analogues. Such nucleotide analogues may suitably enhance the affinity of the oligomer for the target sequence.

Examples of suitable and preferred nucleotide analogues are provided by PCT/DK2006/000512 or are referenced therein.

Incorporation of affinity-enhancing nucleotide analogues in the oligomer, such as LNA or 2'-substituted sugars, can allow the size of the specifically binding oligomer to be reduced, and may also reduce the upper limit to the size of the oligomer before non-specific or aberrant binding takes place.

In some embodiments the oligomer comprises at least 2 nucleotide analogues. In some embodiments, the oligomer comprises from 3-8 nucleotide analogues, *e.g*. 6 or 7 nucleotide analogues. In the by far most preferred embodiments, at least one of said nucleotide analogues is a locked nucleic acid (LNA); for example at least 3 or at least 4, or at least 5, or at least 6, or at least 7, or 8, of the nucleotide analogues may be LNA. In some embodiments all the nucleotides analogues may be LNA.

It will be recognised that when referring to a preferred nucleotide sequence motif or nucleotide sequence, which consists of only nucleotides, the oligomers of the description which are defined by that sequence may comprise a corresponding nucleotide analogue in place of one or more of the nucleotides present in said sequence, such as LNA units or other nucleotide analogues, which raise the duplex stability/Tₘ of the oligomer/target duplex (*i.e*. affinity enhancing nucleotide analogues).

In some embodiments, any mismatches between the nucleotide sequence of the oligomer and the target sequence are preferably found in regions outside the affinity enhancing nucleotide analogues, such as region B as referred to herein, and/or region D as referred to herein, and/or at the site of non modified such as DNA nucleotides in the oligonucleotide, and/or in regions which are 5' or 3' to the contiguous nucleotide sequence.

Examples of such modification of the nucleotide include modifying the sugar moiety to provide a 2'-substituent group or to produce a bridged (locked nucleic acid) structure which enhances binding affinity and may also provide increased nuclease resistance.

A preferred nucleotide analogue is LNA, such as oxy-LNA (such as beta-D-oxy-LNA, and alpha-L-oxy-LNA), and/or amino-LNA (such as beta-D-amino-LNA and alpha-L-amino-LNA) and/or thio-LNA (such as beta-D-thio-LNA and alpha-L-thio-LNA) and/or ENA (such as beta-D-ENA and alpha-L-ENA). Most preferred is beta-D-oxy-LNA.

In some embodiments the nucleotide analogues present within the oligomer of the description (such as in regions A and C mentioned herein) are independently selected from, for example: 2'-O-alkyl-RNA units, 2'-amino-DNA units, 2'-fluoro-DNA units, LNA units, arabino nucleic acid (ANA) units, 2'-fluoro-ANA units, HNA units, INA (intercalating nucleic acid -Christensen, 2002. Nucl. Acids. Res. 2002 30: 4918-4925) units and 2'MOE units. In some embodiments there is only one of the above types of nucleotide analogues present in the oligomer of the description, or contiguous nucleotide sequence thereof.

In some embodiments the nucleotide analogues are 2'-O-methoxyethyl-RNA (2'MOE), 2'-fluoro-DNA monomers or LNA nucleotide analogues, and as such the oligonucleotide of the description may comprise nucleotide analogues which are independently selected from these three types of analogue, or may comprise only one type of analogue selected from the three types. In some embodiments at least one of said nucleotide analogues is 2'-MOE-RNA, such as 2, 3, 4, 5, 6, 7, 8, 9 or 10 2'-MOE-RNA nucleotide units. In some embodiments at least one of said nucleotide analogues is 2'-fluoro DNA, such as 2, 3, 4, 5, 6, 7, 8, 9 or 10 2'-fluoro-DNA nucleotide units.

In some embodiments, the oligomer according to the invention comprises at least one Locked Nucleic Acid (LNA) unit, such as 1, 2, 3, 4, 5, 6, 7, or 8 LNA units, such as between 3 - 7 or 4. to 8 LNA units, or 3, 4, 5, 6 or 7 LNA units. In some embodiments, all the nucleotide analogues are LNA. In some embodiments, the oligomer may comprise both beta-D-oxy-LNA, and one or more of the following LNA units: thio-LNA, amino-LNA, oxy-LNA, and/or ENA in either the beta-D or alpha-L configurations or combinations thereof. In some embodiments all LNA cytosine units are 5'methyl-Cytosine. In some embodiments of the invention, the oligomer may comprise both LNA and DNA units. Preferably the combined total of LNA and DNA units is 10-25, preferably 10-20, even more preferably 12-16. In some embodiments of the invention, the nucleotide sequence of the oligomer, such as the contiguous nucleotide sequence consists of at least one LNA and the remaining nucleotide units are DNA units. In some embodiments the oligomer comprises only LNA nucleotide analogues and naturally occurring nucleotides (such as RNA or DNA, most preferably DNA nucleotides), optionally with modified internucleotide linkages such as phosphorothioate.

The term "nucleobase" refers to the base moiety of a nucleotide and covers both naturally occuring a well as non-naturally occurring variants. Thus, "nucleobase" covers not only the known purine and pyrimidine heterocycles but also heterocyclic analogues and tautomeres thereof.

Examples of nucleobases include, but are not limited to adenine, guanine, cytosine, thymidine, uracil, xanthine, hypoxanthine, 5-methylcytosine, isocytosine, pseudoisocytosine, 5-bromouracil, 5-propynyluracil, 6-amifiopurine, 2-aminopurine, inosine, diaminopurine, and 2-chloro-6-aminopurine.

In some embodiments, at least one of the nucleobases present in the oligomer is a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

### LNA

The term "LNA" refers to a bicyclic nucleotide analogue, known as "Locked Nucleic Acid". It may refer to an LNA monomer, or, when used in the context of an "LNA oligonucleotide" refers to an oligonucleotide containing one or more such bicyclic nucleotide analogues.

The LNA used in the oligonucleotide compounds of the invention preferably has the structure of the general formula I 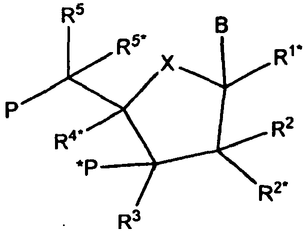
wherein X is selected from -O-, -S-, -N(R^{N*})-, -C(R⁶R^{6*})-;
B is selected from hydrogen, optionally substituted C₁₋₄-alkoxy, optionally substituted C₁₋₄-alkyl, optionally substituted C₁₋₄-acyloxy, nucleobases, DNA intercalators, photochemically active groups, thermochemically active groups, chelating groups, reporter groups, and ligands;
P designates the radical position for an internucleotide linkage to a succeeding monomer, or a 5'-terminal group, such internucleotide linkage or 5'-terminal group optionally including the substituent R⁵ or equally applicable the substituent R^{5*};
P* designates an internucleotide linkage to a preceding monomer, or a 3'-terminal group;
R^{4*} and R^{2*} together designate a biradical consisting of 1-4 groups/atoms selected from -C(R^{a}R^{b})-, -C(R^{a})=C(R^{b})-, -C(R^{a})=N-, -O-, -Si(R^{a})₂-, -S-, -SO₂-, -N(R^{a})-, and >C=Z,
wherein Z is selected from -O-, -S-, and -N(R^{a})-, and R^{a} and R^{b} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂), and each of the substituents R^{1*}, R², R³, R⁵, R^{5*}, R⁶ and R^{6*}, which are present is independently selected from hydrogen, optionally substituted C₁₋₁₂-alkyl, optionally substituted C₂₋₁₂-alkenyl, optionally substituted C₂-₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene, or together may form a spiro biradical consisting of a 1-5 carbon atom(s) alkylene chain which is optionally interrupted and/or terminated by one or more heteroatoms/groups selected from -O-, -S-, and -(NR^{N})- where R^{N} is selected from hydrogen and C₁₋₄-alkyl, and where two adjacent (non-geminal) substituents may designate an additional bond resulting in a double bond; and R^{N*}, when present and not involved in a biradical, is selected from hydrogen and C₁₋₄-alkyl; and basic salts and acid addition salts thereof;

In some embodiments R^{5*} is selected from H, -CH₃, -CH₂-CH₃,- CH₂-O-CH₃, and-CH=CH₂.

In some embodiments, R^{4*} and R^{2*} together designate a biradical selected from - C(R^{a}R^{b})-O-, -C(R^{a}R^{b})-C(R^{c}R^{d})-O-, -C(R^{a}R^{b})-C(R^{c}R^{d})-C(R^{e}R^{f})-O-, -C(R^{a}R^{b})-O-C(R^{c}R^{d})-, - C(R^{a}R^{b})-O-C(R^{c}R^{d})-O-, -C(R^{a}R^{b})-C(R^{c}R^{d})-, -C(R^{a}R^{b})-C(R^{c}R^{d})-C(R^{e}R^{f})-, - C(R^{a})=C(R^{d})-C(R^{c}R^{d})-, -C(R^{a}R^{b})-N(R^{c})-, -C(R^{a}R^{b})-C(R^{c}R^{d})- N(R^{e})-, -C(R^{a}R^{b})-N(R^{c})-O-, and - C(R^{a}R^{b})-S-, -C(R^{a}R^{b})-C(R^{c}R^{d})-S-, wherein R^{a}, R^{b}, R^{c}, R^{d}, R^{e}, and R^{f} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂),

In a further embodiment R^{4*} and R^{2*} together designate a biradical (bivalent group) selected from -CH₂-O-, -CH₂-S-, -CH₂-NH-, -CH₂-N(CH₃)-, -CH₂-CH₂-O-, -CH₂-CH(CH₃)-, - CH₂-CH₂-S-, -CH₂-CH₂-NH-, -CH₂-CH₂-CH₂-, -CH₂-CH₂-CH₂-O-, -CH₂-CH₂-CH(CH₃)-, - CH=CH-CH₂-, -CH₂-O-CH₂-O-, -CH₂-NH-O-, -CH₂-N(CH₃)-O-, -CH₂-O-CH₂-, -CH(CH₃)-O-, - CH(CH₂-O-CH₃)-O-.

For all chiral centers, asymmetric groups may be found in either *R* or *S* orientation.

Preferably, the LNA used in the oligomer of the invention comprises at least one LNA unit according to any of the formulas 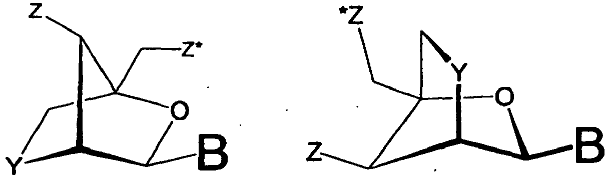 wherein Y is -O-, -O-CH₂- ,-S-, -NH-, or N(R^{H}); Z and Z* are independently selected among an internucleotide linkage, a terminal group or a protecting group; B constitutes a natural or non-natural nucleotide base moiety, and R^{H} is selected from hydrogen and C₁₋₄-alkyl.

Specifically preferred LNA units are shown in scheme 2:

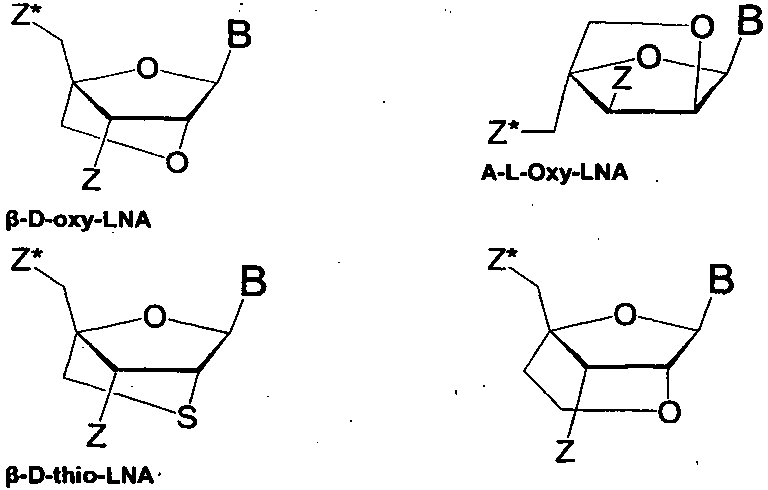

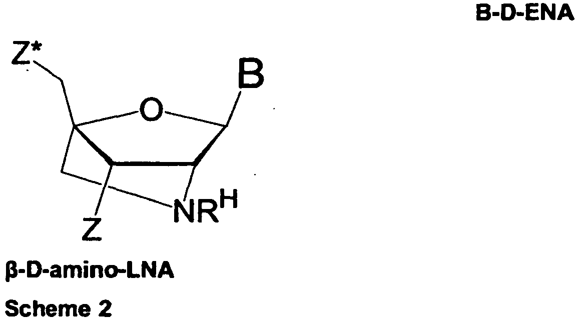

The term "thio-LNA" comprises a locked nucleotide in which Y in the general formula above is selected from S or -CH₂-S-. Thio-LNA can be in both beta-D and alpha-L-configuration.

The term "amino-LNA" comprises a locked nucleotide in which Y in the general formula above is selected from -N(H)-, N(R)-, CH₂-N(H)-, and -CH₂-N(R)- where R is selected from hydrogen and C₁₋₄-alkyl. Amino-LNA can be in both beta-D and alpha-L-configuration.

The term "oxy-LNA" comprises a locked nucleotide in which Y in the general formula above represents -0- or -CH₂-O-. Oxy-LNA can be in both beta-D and alpha-L-configuration.

The term "ENA" comprises a locked nucleotide in which Y in the general formula above is -CH₂-O- (where the oxygen atom of -CH₂-O- is attached to the 2'-position relative to the base B).

In a preferred embodiment LNA is selected from beta-D-oxy-LNA, alpha-L-oxy-LNA, beta-D-amino-LNA and beta-D-thio-LNA, in particular beta-D-oxy-LNA.

### RNAse recruitment

It is recognised that an oligomeric compound may function via non RNase mediated degradation of target mRNA, such as by steric hindrance of translation, or other methods, however, the preferred oligomers of the invention are capable of recruiting an endoribonuclease (RNase), such as RNase H.

It is preferable that the oligomer, or contiguous nucleotide sequence, comprises of a region of at least 6, such as at least 7 consecutive nucleotide units, such as at least 8 or at least 9 consecutive nucleotide units (residues), including 7, 8, 9, 10, 11, 12, 13, 14, 15 or 16 consecutive nucleotides, which, when formed in a duplex with the complementary target RNA is capable of recruiting RNase. The contiguous sequence which is capable of recruiting RNAse may be region B as referred to in the context of a gapmer as described herein. In some embodiments the size of the contiguous sequence which is capable of recruiting RNAse, such as region B, may be higher, such as 10, 11, 12, 13, 14, 15, 16, 17, 18, 19 or 20 nucleotide units.

EP 1 222 309 provides *in vitro* methods for determining RNaseH activity, which may be used to determine the ability to recruit RNaseH. A oligomer is deemed capable of recruiting RNase H if, when provided with the complementary RNA target, it has an initial rate, as measured in pmol/l/min, of at least 1 %, such as at least 5%, such as at least 10% or less than 20% of the equivalent DNA only oligonucleotide, with no 2' substitutions, with phosphorothioate linkage groups between all nucleotides in the oligonucleotide, using the methodology provided by Example 91 - 95 of EP 1 222 309.

In some embodiments, an oligomer is deemed essentially incapable of recruiting RNaseH if, when provided with the complementary RNA target, and RNaseH, the RNaseH initial rate, as measured in pmol/l/min, is less than 1%, such as less than 5%,such as less than 10% or less than 20% of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309.

In other embodiments, an oligomer is deemed capable of recruiting RNaseH if, when provided with the complementary RNA target, and RNaseH, the RNaseH initial rate, as measured in pmol/l/min, is at least 20%, such as at least 40 %, such as at least 60 %, such as at least 80 % of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309.

Typically the region of the oligomer which forms the consecutive nucleotide units which, when formed in a duplex with the complementary target RNA is capable of recruiting RNase consists of nucleotide units which form a DNA/RNA like duplex with the RNA target - and include both DNA units and LNA units which are in the alpha-L configuration, particularly preferred being alpha-L-oxy LNA.

The oligomer of the invention may comprise a nucleotide sequence which comprises both nucleotides and nucleotide analogues, and may be in the form of a gapmer, a headmer or a mixmer.

A headmer is defined by a contiguous stretch of non-RNase recruiting nucleotide analogues at the 5'-end followed by a contiguous stretch of DNA or modified nucleotide units recognizable and cleavable by the RNase towards the 3'-end (such as at least 7 such nucleotides), and a tailmer is defined by a contiguous stretch of DNA or modified nucleotides recognizable and cleavable by the RNase at the 5'-end (such as at least 7 such nucleotides), followed by a contiguous stretch of non-RNase recruiting nucleotide analogues towards the 3'-end. Other chimeras according to the description, called mixmers consisting of an alternate composition of DNA or modified nucleotides recognizable and cleavable by RNase and non-RNase recruiting nucleotide analogues. Some nucleotide analogues may also be able to mediate RNaseH binding and cleavage. Since α-L-LNA recruits RNaseH activity to a certain extent, smaller gaps of DNA or modified nucleotides recognizable and cleavable by the RNaseH for the gapmer construct might be required, and more flexibility in the mixmer construction might be introduced.

### Gapmer Design

Preferably, the oligomer of the invention is a gapmer. A gapmer oligomer is an oligomer which comprises a contiguous stretch of nucleotides which is capable of recruiting an RNAse, such as RNAseH, such as a region of at least 6 or 7 DNA nucleotides, referred to herein in as region B, wherein region B is flanked both 5' and 3' by regions of affinity enhancing nucleotide analogues, such as between 1 - 6 nucleotide analogues 5' and 3' to the contiguous stretch of nucleotides which is capable of recruiting RNAse - these regions are referred to as regions A and C respectively.

Preferably the gapmer comprises a (poly)nucleotide sequence of formula (5' to 3'), A-B-C, or optionally A-B-C-D or D-A-B-C, wherein; region A (5' region) consists or comprises of at least one nucleotide analogue, such as at least one LNA unit, such as between 1-6 nucleotide analogues, such as LNA units, and; region B consists or comprises of at least five consecutive nucleotides which are capable of recruiting RNAse (when formed in a duplex with a complementary RNA molecule, such as the mRNA target), such as DNA nucleotides, and; region C (3'region) consists or comprises of at least one nucleotide analogue, such as at least one LNA unit, such as between 1-6 nucleotide analogues, such as LNA units, and; region D, when present consists or comprises of 1, 2 or 3 nucleotide units, such as DNA nucleotides.

In some embodiments, region A consists of 1, 2, 3, 4, 5 or 6 nucleotide analogues, such as LNA units, such as between 2-5 nucleotide analogues, such as 2-5 LNA units, such as 3 or 4 nucleotide analogues, such as 3 or 4 LNA units; and/or region C consists of 1, 2, 3, 4, 5 or 6 nucleotide analogues, such as LNA units, such as between 2-5 nucleotide analogues, such as 2-5 LNA units, such as 3 or 4 nucleotide analogues, such as 3 or 4 LNA units.

In some embodiments B consists or comprises of 5, 6, 7, 8, 9, 10, 11 or 12 consecutive nucleotides which are capable of recruiting RNAse, or between 6-10, or between 7-9, such as 8 consecutive nucleotides which are capable of recruiting RNAse. In some embodiments region B consists or comprises at least one DNA nucleotide unit, such as 1-12 DNA units, preferably between 4-12 DNA units, more preferably between 6-10 DNA units, such as between 7-10 DNA units, most preferably 8, 9 or 10 DNA units.

In some embodiments region A consist of 3 or 4 nucleotide analogues, such as LNA, region B consists of 7, 8, 9 or 10 DNA units, and region C consists of 3 or 4 nucleotide analogues, such as LNA. Such designs include (A-B-C) 3-10-3, 3-10-4, 4-10-3, 3-9-3, 3-9-4, 4-9-3, 3-8-3, 3-8-4, 4-8-3, 3-7-3, 3-7-4, 4-7-3, and may further include region D, which may have one or 2 nucleotide units, such as DNA units.

Further gapmer designs are disclosed in WO2004/046160.

US provisional application, 60/977409 refers to 'shortmer' gapmer oligomers, which, in some embodiments may be the gapmer oligomer according to the present invention.

In some embodiments the oligomer is consisting of a contiguous nucleotide sequence of a total of 10, 11, 12, 13 or 14 nucleotide units, wherein the contiguous nucleotide sequence is of formula (5' - 3'), A-B-C, or optionally A-B-C-D or D-A-B-C, wherein; A consists of 1, 2 or 3 nucleotide analogue units, such as LNA units; B consists of 7, 8 or 9 contiguous nucleotide units which are capable of recruiting RNAse when formed in a duplex with a complementary RNA molecule (such as a mRNA target); and C consists of 1, 2 or 3 nucleotide analogue units, such as LNA units. When present, D consists of a single DNA unit.

In some embodiments A consists of 1 LNA unit. In some embodiments A consists of 2 LNA units. In some embodiments A consists of 3 LNA units. In some embodiments C consists of 1 LNA unit. In some embodiments C consists of 2 LNA units. In some embodiments C consists of 3 LNA units. In some embodiments B consists of 7 nucleotide units. In some embodiments B consists of 8 nucleotide units. In some embodiments B consists of 9 nucleotide units. In some embodiments B comprises of between 1 - 9 DNA units, such as 2, 3, 4, 5, 6, 7 or 8 DNA units. In some embodiments B consists of DNA units. In some embodiments B comprises of at least one LNA unit which is in the alpha-L configuration, such as 2, 3, 4, 5, 6, 7, 8 or 9 LNA units in the alpha-L-configuration. In some embodiments B comprises of at least one alpha-L-oxy LNA unit or wherein all the LNA units in the alpha-L- configuration are alpha-L-oxy LNA units. In some embodiments the number of nucleotides present in A-B-C are selected from the group consisting of (nucleotide analogue units - region B - nucleotide analogue units): 1-8-1, 1-8-2, 2-8-1, 2-8-2, 3-8-3, 2-8-3, 3-8-2, 4-8-1, 4-8-2, 1-8-4, 2-8-4, or;1-9-1, 1-9-2, 2-9-1, 2-9-2, 2-9-3, 3-9-2, 1-9-3, 3-9-1, 4-9-1, 1-9-4, or; 1-10-1, 1-10-2, 2-10-1, 2-10-2, 1-10-3, 3-10-1. In some embodiments the number of nucleotides in A-B-C are selected from the group consisting of: 2-7-1, 1-7-2, 2-7-2, 3-7-3, 2-7-3, 3-7-2, 3-7-4, and 4-7-3. In some embodiments both A and C consists of two LNA units each, and B consists of 8 or 9 nucleotide units, preferably DNA units.

### Internucleotide Linkages

The terms "linkage group" or "internucleotide linkage" are intended to mean a group capable of covalently coupling together two nucleotides, two nucleotide analogues, and a nucleotide and a nucleotide analogue, etc. Specific and preferred examples include phosphate groups and phosphorothioate groups.

The nucleotides of the oligomer of the invention or contiguous nucleotides sequence thereof are coupled together via linkage groups. Suitably each nucleotide is linked to the 3' adjacent nucleotide via a linkage group.

Suitable internucleotide linkages include those listed within PCT/DK2006/000512, for example the internucleotide linkages listed on the first paragraph of page 34 of PCT/DK2006/000512.

It is, in some embodiments, preferred to modify the internucleotide linkage from its normal phosphodiester to one that is more resistant to nuclease attack, such as phosphorothioate or boranophosphate - these two, being cleavable by RNase H, also allow that route of antisense inhibition in reducing the expression of the target gene.

Suitable sulphur (S) containing internucleotide linkages as provided herein may be preferred. Phosphorothioate internucleotide linkages are also preferred, particularly for the gap region (B) of gapmers. Phosphorothioate linkages may also be used for the flanking regions (A and C, and for linking A or C to D, and within region D, as appropriate).

Regions A, B and C, may however comprise internucleotide linkages other than phosphorothioate, such as phosphodiester linkages, particularly, for instance when the use of nucleotide analogues protects the internucleotide linkages within regions A and C from endo-nuclease degradation - such as when regions A and C comprise LNA nucleotides.

The internucleotide linkages in the oligomer may be phosphodiester, phosphorothioate or boranophosphate so as to allow RNase H cleavage of targeted RNA. Phosphorothioate is preferred, for improved nuclease resistance and other reasons, such as ease of manufacture.

In one aspect of the oligomer of the invention, the nucleotides and/or nucleotide analogues are linked to each other by means of phosphorothioate groups.

It is recognised that the inclusion of phosphodiester linkages, such as one or two linkages, into an otherwise phosphorothioate oligomer, particularly between or adjacent to nucleotide analogue units (typically in region A and or C) can modify the bioavailability and/or bio-distribution of an oligomer - see WO2008/053314

In some embodiments, such as the embodiments referred to above, where suitable and not specifically indicated, all remaining linkage groups are either phosphodiester or phosphorothioate, or a mixture thereof.

In some embodiments all the internucleotide linkage groups are phosphorothioate. When referring to specific gapmer oligonucleotide sequences, such as those provided herein it will be understood that, in various embodiments, when the linkages are phosphorothioate linkages, alternative linkages, such as those disclosed herein may be used, for example phosphate (phosphodiester) linkages may be used, particularly for linkages between nucleotide analogues, such as LNA, units. Likewise, when referring to specific gapmer oligonucleotide sequences, such as those provided herein, when the C residues are annotated as 5'methyl modified cytosine, in various embodiments, one or more of the Cs present in the oligomer may be unmodified C residues.in some embodimentsin some embodiments

### Oligomeric Compounds

The oligomers of the description may, for example, be selected from the group consisting of: 29 - 55 or 56 -148. Particularly preferred compounds include, but are not necessarily limited to, SEQ ID NOs 67 and 77.

### Conjugates

In the context the term "conjugate" is intended to indicate a heterogenous molecule formed by the covalent attachment ("conjugation") of the oligomer as described herein to one or more non-nucleotide, or non-polynucleotide moieties. Examples of non-nucleotide or non- polynucleotide moieties include macromolecular agents such as proteins, fatty acid chains, sugar residues, glycoproteins, polymers, or combinations thereof. Typically proteins may be antibodies for a target protein. Typical polymers may be polyethylene glycol.

Therefore, in various embodiments, the oligomer of the invention may comprise both a polynucleotide region which typically consists of a contiguous sequence of nucleotides, and a further non-nucleotide region. When referring to the oligomer of the invention consisting of a contiguous nucleotide sequence, the compound may comprise non-nucleotide components, such as a conjugate component.

In some embodimentsof the invention the oligomer (compound) is linked to a ligand or conjugate. For example in order to increase the cellular uptake of the oligomer. In some embodimentsthe conjugate is a sterols, such as cholesterol.

The oligomers of the invention may also be conjugated to active drug substances, for example, aspirin, ibuprofen, a sulfa drug, an antidiabetic, an antibacterial or an antibiotic.

In various embodiments of the invention the oligomeric compound is linked to ligands/conjugates, which may be used, e.g. to increase the cellular uptake of oligomeric compounds. WO2007/031091 provides suitable ligands and conjugates.

The invention also provides for a conjugate comprising the compound according to the invention as herein described, and at least one non-nucleotide or non-polynucleotide moiety covalently attached to said compound. Therefore, in various embodiments where the compound of the invention consists of a specified nucleic acid or nucleotide sequence, as herein disclosed, the compound may also comprise at least one non-nucleotide or non-polynucleotide moiety (e.g. not comprising one or more nucleotides or nucleotide analogues) covalently attached to said compound.

Conjugation (to a conjugate moiety) may enhance the activity, cellular distribution or cellular uptake of the oligomer of the invention. Such moieties include, but are not limited to, antibodies, polypeptides, lipid moieties such as a cholesterol moiety, cholic acid, a thioether, e.g. Hexyl-s-tritylthiol, a thiocholesterol, an aliphatic chain, e.g., dodecandiol or undecyl residues, a phospholipids, e.g., di-hexadecyl-rac-glycerol or triethylammonium 1,2-di-o-hexadecyl-rac-glycero-3-h-phosphonate, a polyamine or a polyethylene glycol chain, an adamantane acetic acid, a palmityl moiety, an octadecylamine or hexylamino-carbonyl-oxycholesterol moiety.

The oligomers of the invention may also be conjugated to active drug substances, for example, aspirin, ibuprofen, a sulfa drug, an antidiabetic, an antibacterial or an antibiotic.

In certain embodiments the conjugated moiety is a sterol, such as cholesterol.
In various embodiments, the conjugated moiety comprises or consists of a positively charged polymer, such as a positively charged peptides of, for example between 1 -50, such as 2 - 20 such as 3 - 10 amino acid residues in length, and/or polyalkylene oxide such as polyethylglycol(PEG) or polypropylene glycol - see WO 2008/034123. Suitably the positively charged polymer, such as a polyalkylene oxide may be attached to the oligomer of the invention via a linker such as the releasable inker described in WO 2008/034123. By way of example, the following conjugate moieties may be used in the conjugates of the invention:

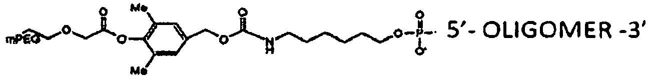

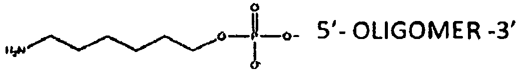

### Activated oligomers

The term "activated oligomer," as used herein, refers to an oligomer of the invention that is covalently linked (i.e., functionalized) to at least one functional moiety that permits covalent linkage of the oligomer to one or more conjugated moieties, i.e., moieties that are not themselves nucleic acids or monomers, to form the conjugates herein described. Typically, a functional moiety will comprise a chemical group that is capable of covalently bonding to the oligomer via, e.g., a 3'-hydroxyl group or the exocyclic NH₂ group of the adenine base, a spacer that is preferably hydrophilic and a terminal group that is capable of binding to a conjugated moiety (e.g., an amino, sulfhydryl or hydroxyl group). In some embodiments, this terminal group is not protected, e.g., is an NH₂ group. In other embodiments, the terminal group is protected, for example, by any suitable protecting group such as those described in "Protective Groups in Organic Synthesis" by Theodora W Greene and Peter G M Wuts, 3rd edition (John Wiley & Sons, 1999). Examples of suitable hydroxyl protecting groups include esters such as acetate ester, aralkyl groups such as benzyl, diphenylmethyl, or triphenylmethyl, and tetrahydropyranyl. Examples of suitable amino protecting groups include benzyl, alpha-methylbenzyl, diphenylmethyl, triphenylmethyl, benzyloxycarbonyl, tert-butoxycarbonyl, and acyl groups such as trichloroacetyl or trifluoroacetyl. In some embodiments, the functional moiety is self-cleaving. In other embodiments, the functional moiety is biodegradable. See e.g., U.S. Patent No. 7,087,229.

In some embodiments, oligomers of the invention are functionalized at the 5' end in order to allow covalent attachment of the conjugated moiety to the 5' end of the oligomer. In other embodiments, oligomers of the invention can be functionalized at the 3' end. In still other embodiments, oligomers of the invention can be functionalized along the backbone or on the heterocyclic base moiety. In yet other embodiments, oligomers of the invention can be functionalized at more than one position independently selected from the 5' end, the 3' end, the backbone and the base.

In some embodiments, activated oligomers of the invention are synthesized by incorporating during the synthesis one or more monomers that is covalently attached to a functional moiety. In other embodiments, activated oligomers of the invention are synthesized with monomers that have not been functionalized, and the oligomer is functionalized upon completion of synthesis. In some embodiments, the oligomers are functionalized with a hindered ester containing an aminoalkyl linker, wherein the alkyl portion has the formula (CH₂)_{w}, wherein w is an integer ranging from 1 to 10, preferably about 6, wherein the alkyl portion of the alkylamino group can be straight chain or branched chain, and wherein the functional group is attached to the oligomer via an ester group (-O-C(O)-(CH₂)_{w}NH).

In other embodiments, the oligomers are functionalized with a hindered ester containing a (CH₂)_{w}-sulfhydryl (SH) linker, wherein w is an integer ranging from 1 to 10, preferably about 6, wherein the alkyl portion of the alkylamino group can be straight chain or branched chain, and wherein the functional group attached to the oligomer via an ester group (-O-C(O)-(CH₂)_{w}SH)

In some embodiments, sulfhydryl-activated oligonucleotides are conjugated with polymer moieties such as polyethylene glycol or peptides (via formation of a disulfide bond).

Activated oligomers containing hindered esters as described above can be synthesized by any method known in the art, and in particular by methods disclosed in PCT Publication No. WO 2008/034122 and the examples therein.

In still other embodiments, the oligomers of the invention are functionalized by introducing sulfhydryl, amino or hydroxyl groups into the oligomer by means of a functionalizing reagent substantially as described in U.S. Patent Nos. 4,962,029 and 4,914,210, i.e., a substantially linear reagent having a phosphoramidite at one end linked through a hydrophilic spacer chain to the opposing end which comprises a protected or unprotected sulfhydryl, amino or hydroxyl group. Such reagents primarily react with hydroxyl groups of the oligomer. In some embodiments, such activated oligomers have a functionalizing reagent coupled to a 5'-hydroxyl group of the oligomer. In other embodiments, the activated oligomers have a functionalizing reagent coupled to a 3'-hydroxyl group. In still other embodiments, the activated oligomers of the description have a functionalizing reagent coupled to a hydroxyl group on the backbone of the oligomer. In yet further embodiments, the oligomer of the invention is functionalized with more than one of the functionalizing reagents as described in U.S. Patent Nos. 4,962,029 and 4,914,210. Methods of synthesizing such functionalizing reagents and incorporating them into monomers or oligomers are disclosed in U.S. Patent Nos. 4,962,029 and 4,914,210.

In some embodiments, the 5'-terminus of a solid-phase bound oligomer is functionalized with a dienyl phosphoramidite derivative, followed by conjugation of the deprotected oligomer with, e.g., an amino acid or peptide via a Diels-Alder cycloaddition reaction.

In various embodiments, the incorporation of monomers containing 2'-sugar modifications, such as a 2'-carbamate substituted sugar or a 2'-(O-pentyl-N-phthalimido)-deoxyribose sugar into the oligomer facilitates covalent attachment of conjugated moieties to the sugars of the oligomer. In other embodiments, an oligomer with an amino-containing linker at the 2'-position of one or more monomers is prepared using a reagent such as, for example, 5'-dimethoxytrityl-2'-O-(e-phthalimidylaminopentyl)-2'-deoxyadenosine-3'-- N,N-diisopropyl-cyanoethoxy phosphoramidite. See, e.g., Manoharan, et al., Tetrahedron Letters, 1991,34,7171.

In still further embodiments, the oligomers of the invention may have amine-containing functional moieties on the nucleobase, including on the N6 purine amino groups, on the exocyclic N2 of guanine, or on the N4 or 5 positions of cytosine. In various embodiments, such functionalization may be achieved by using a commercial reagent that is already functionalized in the oligomer synthesis.

Some functional moieties are commercially available, for example, heterobifunctional and homobifunctional linking moieties are available from the Pierce Co. (Rockford, III.). Other commercially available linking groups are 5'-Amino-Modifier C6 and 3'-Amino-Modifier reagents, both available from Glen Research Corporation (Sterling, Va.). 5'-Amino-Modifier C6 is also available from ABI (Applied Biosystems Inc., Foster City, Calif.) as Aminolink-2, and 3'-Amino-Modifier is also available from Clontech Laboratories Inc. (Palo Alto, Calif.).

### Compositions

The oligomer of the invention may be used in pharmaceutical formulations and compositions. Suitably, such compositions comprise a pharmaceutically acceptable diluent, carrier, salt or adjuvant. PCT/DK2006/000512 provides suitable and preferred pharmaceutically acceptable diluent, carrier and adjuvants. Suitable dosages, formulations, administration routes, compositions, dosage forms, combinations with other therapeutic agents, pro-drug formulations are also provided in PCT/DK2006/000512.

### Applications

The oligomers of the invention may be utilized as research reagents for, for example, diagnostics, therapeutics and prophylaxis.

In research, such oligomers may be used to specifically inhibit the synthesis of PIK3CA protein (typically by degrading or inhibiting the mRNA and thereby prevent protein formation) in cells and experimental animals thereby facilitating functional analysis of the target or an appraisal of its usefulness as a target for therapeutic intervention.

In diagnostics the oligomers may be used to detect and quantitate PIK3CA expression in cell and tissues by northern blotting, *in-situ* hybridisation or similar techniques.

For therapeutics, an animal or a human, suspected of having a disease or disorder, which can be treated by modulating the expression of PIK3CA kinase is treated by administering antisense compounds in accordance with this invention. Such disorders include hyperproliferative disorders such as cancer. Further provided are methods of treating a mammal, such as treating a human, suspected of having or being prone to a disease or condition, associated with expression of PIK3CA kinase by administering a therapeutically or prophylactically effective amount of one or more of the oligomers or compositions of the description.

It has been suggested by leading scientists in the field that pharmaceutical intervention with PIK3CA will result in therapeutic options against cancer, such as those referred to herein.

The description also relates to an oligomer, a composition or a conjugate as defined herein for use as a medicament.

The oligomers and other compositions according to the description can be used for the treatment of conditions associated with over expression or expression of mutated version of the PIK3CA.

The description further provides use of a compound of the description in the manufacture of a medicament for the treatment of a disease, disorder or condition as referred to herein.

Generally stated, in some aspects, the description is directed to a method of treating a mammal suffering from or susceptible to conditions associated with abnormal levels of PIK3CA, comprising administering to the mammal and therapeutically effective amount of an oligomer targeted to PIK3CA that comprises one or more LNA units. The methods of the description are preferably employed for treatment or prophylaxis against diseases caused by abnormal levels of PIK3CA. Alternatively stated, In some embodiments, the description is furthermore directed to a method for treating abnormal levels of PIK3CA, said method comprising administering a oligomer of the invention, or a conjugate of the invention or a pharmaceutical composition of the invention to a patient in need thereof.

The oligomers and other compositions according to the invention can be used for the treatment of conditions associated with over expression or expression of PIK3CA or mutated versions of the PIK3CA.

The disease or disorder, as referred to herein, may, in some embodiments be associated with a mutation in the PIK3CA gene or a gene whose protein product is associated with or interacts with PIK3CA. Therefore, in some embodiments, the target mRNA is a mutated form of the PIK3CA sequence.

The oligomer, a conjugate or a pharmaceutical composition according to the invention is typically administered in an effective amount.

An interesting aspect of the invention is directed to the use of an oligomer (compound) as defined herein or a conjugate as defined herein for the preparation of a medicament for the treatment of a disease, disorder or condition as referred to herein.

The description further relates to use of a compound, composition, or a conjugate as defined herein for the manufacture of a medicament for the treatment of abnormal levels of PIK3CA or expression of mutant forms of PIK3CA (such as allelic variants, such as those associated with one of the diseases referred to herein).

Moreover, the description relates to a method of treating a subject suffering from a disease or condition such as those referred to herein.

A patient who is in need of treatment is a patient (subject) suffering from or likely to suffer from the disease or disorder.

In some embodiments, the term 'treatment' as used herein refers to both treatment of an existing disease (e.g. a disease or disorder as herein referred to), or prevention of a disease, i.e. prophylaxis. It will therefore be recognised that treatment as referred to herein may, in some embodiments, be prophylactic.

The oligomer, a conjugate or a pharmaceutical composition according to the invention is typically administered in an effective amount.

The description also provides for the use of the compound or conjugate of the invention as described for the manufacture of a medicament for the treatment of a disorder as referred to herein, or for a method of the treatment of as a disorder as referred to herein.

The description also provides for a method for treating a disorder as referred to herein said method comprising administering a compound according to the invention as herein described, and/or a conjugate according to the invention, and/or a pharmaceutical composition according to the invention to a patient in need thereof. The pharmaceutical composition according to the invention may be used for the treatment of conditions associated with abnormal levels of PIK3CA kinase, such as hyperproliferative diseases and cancer. The description also provides for a method for treating cancer, said method comprising administering a compound according to the invention as herein described, and/or a conjugate according to the invention, and/or a pharmaceutical composition according to the invention to a patient in need thereof. The description also provides for the use of the compound or conjugate of the invention as described for the manufacture of a medicament for the treatment of cancer, or for a method of the treatment of cancer.

Suitable dosages, formulations, administration routes, compositions, dosage forms, combinations with other therapeutic agents, pro-drug formulations are also provided in PCT/DK2006/000512, although it should be recognised that the aspects of PCT/DK2006/000512 which are only specifically applicable to the treatment of cancer may not be appropriate in the therapeutic/pharmaceutical compositions and methods of the present description.

The invention also provides for a pharmaceutical composition comprising a compound or a conjugate as herein described or a conjugate, and a pharmaceutically acceptable diluent, carrier or adjuvant. PCT/DK2006/000512 provides suitable and preferred pharmaceutically acceptable diluent, carrier and adjuvants.

In some embodiments, the term 'treatment' as used herein refers to both treatment of an exisiting disease (e.g. a cancer as herein referred to), or prevention of a disease, *i.e.* prophylaxis. It will therefore be recognised that treatment as referred to herein may, in some embodiments, be prophylactic.

The invention further provides use of a compound of the invention in the manufacture of a medicament for the treatment of any and all conditions disclosed herein.

Generally stated, in some aspects, the description is directed to a method of treating a mammal suffering from or susceptible to conditions associated with abnormal, typically elevated, levels of PIK3CA kinase, comprising administering to the mammal and therapeutically effective amount of an oligomer targeted to PIK3CAK that comprises one or more LNA units.

An interesting aspect of the invention is directed to the use of an oligomer (compound) as defined herein or as conjugate as defined herein for the preparation of a medicament for the treatment of a condition according to above.

The methods of the description may, in some embodiments, be employed for treatment or prophylaxis against diseases caused by abnormal levels of PIK3CA kinase.

Furthermore, the description described herein encompasses a method of preventing or treating a disease comprising a therapeutically effective amount of a PIK3CA kinase modulating oligomer to a human in need of such therapy. The description further encompasses the use of a short period of administration of a PIK3CA kinase modulating oligonucleotide compound.

Alternatively stated, the description is, in some embodiments, furthermore directed to a method for treating abnormal levels of PIK3CA kinase, said method comprising administering a oligomer of the invention, or a conjugate of the invention or a pharmaceutical composition of the invention to a patient in need thereof and further comprising the administration of a further chemotherapeutic agent. Said further administration may be such that the further chemotherapeutic agent is conjugated to the compound of the invention, is present in the pharmaceutical composition, or is administered in a separate formulation.

The description also, in some aspects, relates to an oligomer, a composition or a conjugate as defined herein for use as a medicament.

The description further relates to use of a compound, composition, or a conjugate as defined herein for the manufacture of a medicament for the treatment of abnormal levels of PIK3CA kinase or expression of mutant forms of PIK3CAK (such as allelic variants, such as those associated with one of the diseases referred to herein).
Moreover, the description relates to a method of treating a subject suffering from a disease or condition selected from hyperproliferative diseases and cancer, such as those referred to herein.

In some embodiments, the description provides for a method for the inhibition of both PIK3CA and beta-catenin in a cell which is expressing both PIK3CA and beta-catenin, said method comprising administering an oligomer, or a conjugate according to the invention to said cell so as to effect the inhibition of PIK3CA and beta-catenin in said cell. Suitably the oligomer which is capable of inhibiting or down-regulating both PI3CA and beta-catenin in a cell has significantly homology to, or is capable of hybriodising to the reverse complement of both target nucleic acids, such as an oligomer with a sequence of nucleobases of SEQ ID 82.

### Medical Indications

Hyperproliferative diseases refer to disorders which are characterised by the uncontrolled and detrimental proliferation of cells within the body, such as cancer, such as those referred to herein.

In some embodiments, said cancer is in the form of i) a solid tumor and/or a carcinoma, and/or ii) a sarcoma, and/or iii) glioma.

Said carcinoma as referred to herein may be selected from the group consisting of malignant melanoma, basal cell carcinoma, ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma and carcinoid tumors. More preferably, said carcinoma is selected from the group consisting of malignant melanoma, non-small cell lung cancer, breast carcinoma, colon carcinoma and renal cell carcinoma. Preferably, said carcinoma is a malignant melanoma, preferably selected from the group consisting of superficial spreading melanoma, nodular melanoma, lentigo maligna melanoma, acral melagnoma, amelanotic melanoma and desmoplastic melanoma.

Said sarcoma as referred to herein may be selected from the group consisting of osteosarcoma, Ewing's sarcoma, chondrosarcoma, malignant fibrous histiocytoma, fibrosarcoma and Kaposi's sarcoma.

In some embodiments the cancer is selected from the group consisting of solid tumours such as glioblastoma, malignant melanoma, medulloblastoma, hepatocellular carcinoma, head and neck squamous cell carcinoma, gastric, ovarian, cervix and colorectal cancers, cancers of the breast, lung and colon, large B-cell lymphoma, anaplastic astrocytoma, anaplastic oligodendroglioma, prostate cancer, endometrial cancer, pancreatic cancer, bowel cancer, leukaemia, esophagus cancer, and thyroid cancer, or in some embodiments, liver or kidney cancer.

In some embodiments, the cancer referred to herein is selected from the group consisting of: Colorectal, glioblastoma, gastric, hepatocellular, breast, ovarian and lung cancers.

In aspect the cancer as referred to herein, may be selected from the group consisting of; non-Hodgkin's lymphoma, Hodgkin's lymphoma, leukemia (e.g., acute leukemia such as acute lymphocytic leukemia, acute myelocytic leukemia, chronic myeloid leukemia, chronic lymphocytic leukemia, multiple myeloma), colon carcinoma, rectal carcinoma, pancreatic cancer, breast cancer, ovarian cancer, prostate cancer, renal cell carcinoma, hepatoma, bile duct carcinoma, choriocarcinoma, cervical cancer, testicular cancer, lung carcinoma, bladder carcinoma, melanoma, head and neck cancer, brain cancer, cancers of unknown primary site, neoplasms, cancers of the peripheral nervous system, cancers of the central nervous system, tumors (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, seminoma, embryonal carcinoma, Wilms' tumor, small cell lung carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, and retinoblastoma), heavy chain disease, metastases, and any disease or disorder characterized by uncontrolled or abnormal cell growth.

In some embodimentsthe cancer referred to herein is selected from: Hodgkin's lymphoma, leukaemia such as acute lyphacytic leukaemia, colon carcinoma, rectal carcinoma, brain cancer, neural blastomas, lung cancer, pancreatic cancer, melanoma, acute mylogenous leukaemia, liver cancer, thyroid cancer, kidney cancer, urinary tract cancer and bladder cancer.

In some embodimentsthe cancer referred to herein is selected from: Hodgkin's lymphoma, leukaemia such as acute lyphacytic leukaemia, colon carcinoma, brain cancer, neural blastomas.

In some embodiments, the cancer referred to herein are selected from the group consisting of cancer diseases is a lung, breast, colon, prostate, pancreas, lung, liver, thyroid, kidney, brain, testes, stomach, intestine, bowel, spinal cord, sinuses, bladder, urinary tract or ovaries cancer.

In some embodiments, for example for the treatment of brain cancer, it is preferred that phosphorothioate linkages are not used in the compound according to the invention.

Further conditions which may be associated with abnormal levels of PIK3CA kinase, and which, therefore may be treated using the compositions, conjugates and compounds according to the invention include disorders selected form the group consisting of hyperproliferative diseases, such as cancer, particularly solid tumours such as glioblastoma, malignant melanoma, medulloblastoma, hepatocellular carcinoma, head and neck squamous cell carcinoma, gastric, ovarian, cervix and colorectal cancers as well as cancers of the breast, lung and colon.

In one aspect the cancers referred to herein may be selected from the group consisiting of solid tumours such as glioblastoma, malignant melanoma, medulloblastoma, hepatocellular carcinoma, head and neck squamous cell carcinoma, gastric, ovarian, cervix and colorectal cancers, cancers of the breast, lung and colon, large B-cell lymphoma, anaplastic astrocytoma, anaplastic oligodendroglioma, prostate cancer, endometrial cancer, pancreatic cancer, bowel cancer, leukaemia, esophagus cancer, and thyroid cancer.

In one aspect the cancers referred to herein may be kidney or liver cancer.

### EMBODIMENTS

The following embodiments of the present description may be used in combination with the other embodiments described herein.
1. An oligomer of between 10-50 nucleobases in length which comprises a contiguous nucleobase sequence of a total of between 10-50 nucleobases, wherein said contiguous nucleobase sequence is at least 80% homologous to a corresponding region of a nucleic acid which encodes a mammalian PIK3CA kinase.
2. The oligomer according to embodiment 1, wherein said oligomer comprises at least one LNA unit.
3. The oligomer according to embodiment 1 or 2, wherein the contiguous nucleobase sequence comprises no more than 3, such as no more than 2 mismatches to the corresponding region of a nucleic acid which encodes a mammalian PIK3CA kinase.
4. The oligomer according to embodiment 3, wherein said contiguous nucleobase sequence comprises a single mismatch to the corresponding region of a nucleic acid which encodes a mammalian PIK3CA kinase, wherein, optionally, the single mismatch corresponds to a single nucleotide point mutation which is associated with a cancer phenotype.
5. The oligomer according to embodiment 1 or 2, wherein said contiguous nucleobase sequence comprises no mismatches, (i.e. is complementary to) the corresponding region of a nucleic acid which encodes a mammalian PIK3CA kinase.
6. The oligomer according to any one of embodiments 1 - 5, wherein the nucleobase sequence of the oligomer consists of the contiguous nucleobase sequence.
7. The oligomer according to any one of embodiments 1- 6, wherein the nucleic acid which encodes a mammalian PIK3CA kinase is the human PIK3CA kinase nucleotide sequence such as SEQ ID No 1, or a variant thereof, such as SEQ ID NO 1 which comprises a single point mutation at a position selected from 1781, 1790 and 3297.
8. The oligomer according to any one of embodiments 1 - 7, wherein the contiguous nucleobase sequence is complementary to a corresponding region of both the human PIK3CA kinase nucleic acid sequence and a non-human mammalian PIK3CA kinase nucleic acid sequence, such as the mouse PIK3CA kinase nucleic acid sequence..
9. The oligomer according to any one of embodiments 1 to 8, wherein the contiguous nucleobase sequence comprises a contiguous subsequence of at least 7, nucleobase residues which, when formed in a duplex with the complementary PIK3CA kinase target RNA is capable of recruiting RNaseH.
10. The oligomer according to embodiment 9, wherein the contiguous nucleobase sequence comprises of a contiguous subsequence of at least 8, at least 9 or at least 10 nucleobase residues which, when formed in a duplex with the complementary PIK3CA kinase target RNA is capable of recruiting RNaseH.
11. The oligomer according to any one of embodiments 9 or 10 wherein said contiguous subsequence is at least 9 or at least 10 nucleobases in length, such as at least 12 nucleobases or at least 14 nucleobases in length, such as 14, 15 or 16 nucleobases residues which, when formed in a duplex with the complementary PIK3CA kinase target RNA is capable of recruiting RNaseH.
12. The oligomer according to embodiment any one of embodiments 1 - 11 wherein said oligomer is conjugated with one or more non-nucleobase compounds.
13. The oligomer according to any one of embodiments 1 - 12, wherein said oligomer has a length of between 10 - 22 nucleobases.
14. The oligomer according to any one of embodiments 1 - 13, wherein said oligomer has a length of between 12 - 18 nucleobases.
15. The oligomer according to any one of embodiments 1 - 14, wherein said oligomer has a length of 14, 15 or 16 nucleobases.
16. The oligomer according to any one of embodiments 1 - 15, wherein said continuous nucleobase sequence corresponds to a contiguous nucleotide sequence present in a nucleic acid sequence selected from the group consisting of SEQ ID NO 110 - 124, or 149 - 160.
17. The oligomer according to any one of embodiments 1-16, wherein the oligomer or contiguous nucleobase sequence comprises, or is selected from a corresponding nucleobase sequence present in a nucleotide sequence selected from the group consisting of SEQ ID NO 2 - 16.
18. The oligomer according to any one of embodiments 1 - 17, wherein said contiguous nucleobase sequence comprises at least one affinity enhancing nucleotide analogue.
19. The oligomer according to embodiment 18, wherein said contiguous nucleobase sequence comprises a total of 2, 3, 4, 5, 6, 7, 8, 9 or 10 affinity enhancing nucleotide analogues, such as between 5 and 8 affinity enhancing nucleotide analogues.
20. The oligomer according to any one of embodiments 1 - 19 which comprises at least one affinity enhancing nucleotide analogue, wherein the remaining nucleobases are selected from the group consisting of DNA nucleotides and RNA nucleotides, preferably DNA nucleotides.
21. The oligomer according to any one of embodiments 1 - 20, wherein the oligomer comprises of a sequence of nucleobases of formula, in 5' to 3' direction, A-B-C, and optionally of formula A-B-C-D, wherein:
   A consists or comprises of at least one nucleotide analogue, such as 1, 2, 3, 4, 5 or 6 nucleotide analogues, preferably between 2-5 nucleotide analogues, preferably 2, 3 or 4 nucleotide analogues, most preferably 2, 3 or 4 consecutive nucleotide analogues and;
   B consists or comprises at least five consecutive nucleobases which are capable of recruiting RNAseH (when formed in a duplex with a complementary RNA molecule, such as the PIK3CAK mRNA target), such as DNA nucleobases, such as 5, 6, 7, 8, 9, 10, 11 or 12 consecutive nucleobases which are capable of recruiting RNAseH, or between 6-10, or between 7-9, such as 8 consecutive nucleobases which are capable of recruiting RNAseH, and;
   C consists or comprises of at least one nucleotide analogue, such as 1, 2, 3, 4, 5, or 6 nucleotide analogues, preferably between 2-5 nucleotide analogues, such as 2, 3 or 4 nucleotide analogues, most preferably 2, 3 or 4 consecutive nucleotide analogues, and;
   D when present, consists or comprises, preferably consists, of one or more DNA nucleotide, such as between 1-3 or 1-2 DNA nucleotides.
22. The oligomer according to embodiment 21, wherein region A consists or comprises of 2, 3 or 4 consecutive nucleotide analogues.
23. The oligomer according to any one of embodiments 21 - 22, wherein region B consists or comprises of 7, 8, 9 or 10 consecutive DNA nucleotides or equivalent nucleobases which are capable of recruiting RNAseH when formed in a duplex with a complementary RNA, such as the PIK3CA kinase mRNA target.
24. The oligomer according to any one of embodiments 21 - 24, wherein region C consists or comprises of 2, 3 or 4 consecutive nucleotide analogues.
25. The oligomer according to any one of embodiments 21 - 24, wherein region D consists, where present, of one or two DNA nucleotides.
26. The oligomer according to any one of embodiments 21 - 25, wherein:
   A Consists or comprises of 3 contiguous nucleotide analogues;
   B Consists or comprises of 7, 8, 9 or 10 contiguous DNA nucleotides or equivalent nucleobases which are capable of recruiting RNAseH when formed in a duplex with a complementary RNA, such as the PIK3CA kinase mRNA target;
   C Consists or comprises of 3 contiguous nucleotide analogues;
   D Consists, where present, of one or two DNA nucleotides.
27. The oligomer according to embodiment 26, wherein the contiguous nucleobase sequence consists of 10, 11, 12, 13 or 14 nucleobases, and wherein;
   A Consists of 1 ,2 or 3 contiguous nucleotide analogues;
   B Consists of 7, 8, or 9 consecutive DNA nucleotides or equivalent nucleobases which are capable of recruiting RNAseH when formed in a duplex with a complementary RNA, such as the PIK3CA kinase mRNA target;
   C Consists of 1 ,2 or 3 contiguous nucleotide analogues;
   D Consists, where present, of one DNA nucleotide.
28. The oligomer according to anyone of embodiments 21 - 27, wherein B comprises at least one LNA nucleobase which is in the alpha-L configuration, such as alpha-L-oxy LNA.
29. The oligomer according to any one of embodiments 1 - 28, wherein the nucleotide analogue(s) are independently or collectively selected from the group consisting of: Locked Nucleic Acid (LNA) units; 2'-O-alkyl-RNA units, 2'-OMe-RNA units, 2'-amino-DNA units, 2'-fluoro-DNA units, PNA units, HNA units, and INA units.
30. The oligomer according to embodiment 29 wherein all the nucleotide analogues(s) are LNA units.
31. The oligomer according to any one of embodiments 1 - 30, which comprises 1, 2, 3, 4, 5, 6, 7, 8, 9 or 10 LNA units such as between 2 and 8 nucleotide LNA units.
32. The oligomer according to any one of the embodiments 29 - 31, wherein the LNAs are independently selected from oxy-LNA, thio-LNA, and amino-LNA, in either of the beta-D and alpha-L configurations or combinations thereof.
33. The oligomer according to embodiment 32, wherein the LNAs are all beta-D-oxy-LNA.
34. The oligomer according to any one of embodiments 21 - 33, wherein the nucleotide analogues of regions A and C are beta-D-oxy-LNA.
35. The oligomer according to any one of embodiments 1 - 34, wherein at least one of the nucleobases present in the oligomeris a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.
36. The oligomer according to any one of embodiments 1 - 35, wherein said oligomer hybridises with a corresponding mammalian PIK3CA kinase mRNA with a Tₘ of at least 50°C.
37. The oligomer according to any one of embodiments 1 - 36, wherein said oligomer hybridises with a corresponding mammalian PIK3CA kinase mRNA with a Tₘ of no greater than 80°C.
38. The oligomer according to any one of embodiments 1 - 37, wherein the internucleoside linkages are independently selected from the group consisting of: phosphodiester, phosphorothioate and boranophosphate.
39. The oligomer according to embodiment 38, wherein the oligomer comprises at least one phosphorothioate internucleoside linkage.
40. The oligomer according to embodiment 39, wherein the internucleoside linkages adjacent to and/or between DNA or RNA units, or within region B are phosphorothioate linkages.
41. The oligomer according to embodiment 39 or 40, wherein the linkages between at least one pair of consecutive nucleotide analogues is a phosphodiester linkage.
42. The oligomer according to embodiment 39 or 40, wherein all the linkages between consecutive nucleotide analogues are phosphodiester linkages.
43. The oligomer according to embodiment 38 wherein all the internucleoside linkages are phosphorothioate linkages.
44. A conjugate comprising the oligomer according to any one of the embodiments 1-43 and at least one non-nucleotide or non-polynucleotide moiety covalently attached to said compound.
45. A pharmaceutical composition comprising an oligomer as defined in any of embodiments 1-43 or a conjugate as defined in embodiment 44, and a pharmaceutically acceptable diluent, carrier, salt or adjuvant.
46. A pharmaceutical composition according to 45, wherein the oligomer is constituted as a pro-drug.
47. Use of an oligomer as defined in any one of the embodiments 1-43, or a conjugate as defined in embodiment 44, for the manufacture of a medicament for the treatment of a disease or disorder selected from the group consisting of hyperproliferative diseases such as cancer.
48. A method for treating a hyperproliferative disease such as cancer, said method comprising administering an oligomer as defined in one of the embodiments 1-43, or a conjugate as defined in embodiment 44, or a pharmaceutical composition as defined in any one of the embodiments 45 - 46, to a patient in need thereof.
49. A method of reducing or inhibiting the expression of PIK3CA kinase in a cell or a tissue, the method comprising the step of contacting said cell or tissue with a compound as defined in one of the embodiments 1-43, or a conjugate as defined in embodiment 44, or a pharmaceutical composition as defined in any one of the embodiments 45 - 46, so that expression of PIK3CA kinase is reduce or inhibited.

### EXAMPLES

### Example 1: Monomer synthesis

The LNA monomer building blocks and derivatives were prepared following published procedures and references cited therein - see WO07/031081 and the references cited therein.

### Example 2: Oligonucleotide synthesis

Oligonucleotides were synthesized according to the method described in WO07/031081. Table 1 shows examples of antisense oligonucleotide sequences of the description. Tables 2 and 3 show examples of antisense oligonucleotides (oligos) of the description.

### Example 3: Design of the oligonucleotides

In accordance with the present description, a series of different oligonucleotides were designed to target different regions of human PIK3CA mRNA (phosphoinositide-3-kinase, catalytic, alpha polypeptide), (GenBank accession number NM_006218, SEQ ID NO:1).

**Table 1 Antisense oligonucleotide sequences of the description**

| SEQ ID NOS 2-16: are oligo sequences designed to target human wild-type PIK3CA mRNA. | | | |
|---|---|---|---|
| **SEQ ID NO** | **Sequence (5'-3')** | **Length (bases)** | **Target site NM_006218** |
| SEQ ID NO: 2 | GAGGCATTCTAAAGTC | 16 | 253-268 |
| SEQ ID NO: 3 | ATTCTTCCCTTTCTGC | 16 | 386-401 |
| SEQ ID NO: 4 | TAGACATACATTGCTC | 16 | 642-657 |
| SEQ ID NO: 5 | TACTTGCCCTGATATT | 16 | 891-906 |
| SEQ ID NO: 6 | CACATAAGGGTTCTCC | 16 | 1247-1262 |
| SEQ ID NO: 7 | AGCCATTCATTCCACC | 16 | 1302-1317 |
| SEQ ID NO: 8 | CAGTAACACCAATAGG | 16 | 1529-1544 |
| SEQ ID NO: 9 | AACTCCAACTCTAAGC | 16 | 1572-1587 |
| SEQ ID NO: 10 | CAGACAGAAGCAATTT | 16 | 1856-1871 |
| SEQ ID NO: 11 | TTATTGTGCATCTCAG | 16 | 2175-2190 |
| SEQ ID NO: 12 | GCAGAGGACATAATTC | 16 | 2466-2481 |
| SEQ ID NO: 13 | GATGTCTGGGTTCTCC | 16 | 2506-2521 |
| SEQ ID NO: 14 | TTCTTCTTGTGATCCA | 16 | 2970-2985 |
| SEQ ID NO: 15 | AAGAAATCCTGTGTCA | 16 | 3024-3039 |
| SEQ ID NO: 16 | TCTCCTGAAACCTCTC | 16 | 3089-3104 |
| SEQ ID NO: 110 | CACGGAGGCATTCTAAAGTCACTA | 24 | 249-272 |
| SEQ ID NO: 111 | AAAAATTCTTCCCTTTCTGCTTCT | 24 | 382-405 |
| SEQ ID NO: 112 | AGGATAGACATACATTGCTCTACT | 24 | 638-661 |
| SEQ ID NO: 113 | AATATACTTGCCCTGATATTCTAA | 24 | 887-910 |
| SEQ ID NO: 114 | TTGTCACATAAGGGTTCTCCTCCA | 24 | 1243-1266 |
| SEQ ID NO: 115 | ATTCAGCCATTCATTCCACCTGGG | 24 | 1298-1321 |
| SEQ ID NO: 116 | GATCCAGTAACACCAATAGGGTTC | 24 | 1525-1548 |
| SEQ ID NO: 117 | GTCAAACTCCAACTCTAAGCATGG | 24 | 1568-1591 |
| SEQ ID NO: 118 | TTAACAGACAGAAGCAATTTGGGT | 24 | 1852-1875 |
| SEQ ID NO: 119 | TGTTTTATTGTGCATCTCAGATTT | 24 | 2171-2194 |
| SEQ ID NO: 120 | TTTTGCAGAGGACATAATTCGACA | 24 | 2462-2485 |
| SEQ ID NO: 121 | ACATGATGTCTGGGTTCTCCCAAT | 24 | 2502-2525 |
| SEQ ID NO: 122 | TTTTTTCTTCTTGTGATCCAAAAA | 24 | 2966-2989 |
| SEQ ID NO: 123 | TATTAAGAAATCCTGTGTCAAAAC | 24 | 3020-3043 |
| SEQ ID NO: 124 | CACATCTCCTGAAACCTCTCAAAT | 24 | 3085-3108 |

**Table 2 Antisense oligonucleotide sequences of the description**

| SEQ ID NOS: 17-22 are oligo sequences designed to target the three hot-spot mutations (E542K, E545K and H1047R) in human PIK3CA mRNA and SEQ ID NOS: 23-28 are their corresponding wild-type oligos. The place of the hot-spot mutation in each oligo is indicated in grey highlight. SEQ ID NO 17, 18, 125, and 126 are directed against the E542K mutation. SEQ ID NO 19, 20, 127 and 128 (SPC4168 and 4169) are directed against the E545K mutation and SEQ ID NO 21, 22, 129 and 130 (SPC4170 and 4171) are directed against the H1047R mutation. | | | |
|---|---|---|---|
| **SEQ ID NO** | **Sequence (5'-3')** | **Length (bases)** | **Target site NM_006218** |
| SEQ ID NO: 17 | GATTTTAGAGAGAGGA | 16 | 1771-1786 |
| SEQ ID NO: 18 | AGTGATTTTAGAGAGA | 16 | 1774 - 1789 |
| SEQ ID NO: 19 | TCCTGCTTAGTGATTT | 16 | 1782 - 1797 |
| SEQ ID NO: 20 | TTCTCCTGCTTAGTGA | 16 | 1785 - 1800 |
| SEQ ID NO: 21 | GCCACCATGACGTGCA | 16 | 3292 - 3307 |
| SEQ ID NO: 22 | ACCATGACGTGCATCA | 16 | 3289 - 3304 |
| SEQ ID NO: 23 | GATTTCAGAGAGAGGA | 16 | 1771-1786 |
| SEQ ID NO: 24 | AGTGATTTCAGAGAGA | 16 | 1774 - 1789 |
| SEQ ID NO: 25 | TCCTGCTCAGTGATTT | 16 | 1782 - 1797 |
| SEQ ID NO: 26 | TTCTCCTGCTCAGTGA | 16 | 1785 - 1800 |
| SEQ ID NO: 27 | GCCACCATGATGTGCA | 16 | 3292 - 3307 |
| SEQ ID NO: 28 | ACCATGATGTGCATCA | 16 | 3289 - 3304 |
| SEQ ID NO: 125 | CAGTGATTTTAGAGAGAGGATCTC | 24 | 1767 - 1790 |
| SEQ ID NO: 126 | GCTCAGTGATTTTAGAGAGAGGAT | 24 | 1770 - 1793 |
| SEQ ID NO: 127 | TTTCTCCTGCTTAGTGATTTCAGA | 24 | 1778 - 1801 |
| SEQ ID NO: 128 | ATCTTTCTCCTGCTTAGTGATTTC | 24 | 1781 - 1804 |
| SEQ ID NO: 129 | TCCAGCCACCATGACGTGCATCAT | 24 | 3288 - 3311 |
| SEQ ID NO: 130 | AGCCACCATGACGTGCATCATTCA | 24 | 3285 -3308 |
| SEQ ID NO: 131 | CAGTGATTTCAGAGAGAGGATCTC | 24 | 1767 - 1790 |
| SEQ ID NO: 132 | GCTCAGTGATTTCAGAGAGAGGAT | 24 | 1770 - 1793 |
| SEQ ID NO: 133 | TTTCTCCTGCTCAGTGATTTCAGA | 24 | 1778 - 1801 |
| SEQ ID NO: 134 | ATCTTTCTCCTGCTCAGTGATTTC | 24 | 1781 - 1804 |
| SEQ ID NO: 135 | TCCAGCCACCATGATGTGCATCAT | 24 | 3288 - 3311 |
| SEQ ID NO: 136 | AGCCACCATGATGTGCATCATTCA | 24 | 3285 -3308 |

**Table 3: Oligonucleotide designs of the description**

| In SEQ ID NOs: 29 - 55, upper case letters indicates nucleotide analogue units, such as LNA, and subscript "s" represents phosphorothiote linkage. Lower case letters represent nucleotide units. Absence of "s" (if any) indicates phosphodiester linkage. | |
|---|---|
| **SEQ ID NO** | **Sequence (5'-3')** |
| SEQ ID NO: 29 | **GₛAₛGₛgₛcₛaₛtₛtₛcₛtₛaₛaₛaₛGₛTₛC** |
| SEQ ID NO: 30 | **AₛTₛTₛcₛtₛtₛcₛcₛcₛtₛtₛtₛcₛTₛGₛC** |
| SEQ ID NO: 31 | **TₛAₛGₛaₛcₛaₛtₛaₛcₛaₛtₛtₛgₛCₛTₛC** |
| SEQ ID NO: 32 | **TₛAₛCₛtₛtₛgₛcₛcₛcₛtₛgₛaₛtₛAₛTₛT** |
| SEQ ID NO: 33 | **CₛAₛCₛaₛtₛaₛaₛgₛgₛgₛtₛtₛCₛTₛCₛC** |
| SEQ ID NO: 34 | **AₛGₛCₛcₛaₛtₛtₛCₛaₛtₛtₛcₛcₛAₛCₛC** |
| SEQ ID NO: 35 | **CₛAₛGₛtₛaₛaₛcₛaₛcₛcₛaₛaₛtₛAₛGₛG** |
| SEQ ID NO: 36 | **AₛAₛCₛtₛcₛcₛaₛaₛcₛtₛcₛtₛaₛAₛGₛC** |
| SEQ ID NO: 37 | **CₛAₛGₛaₛcₛaₛgₛaₛaₛgₛcₛaₛaₛTₛTₛT** |
| SEQ ID NO: 38 | **TₛTₛAₛtₛgₛtₛgₛcₛaₛtₛcₛtₛCₛAₛG** |
| SEQ ID NO: 39 | **GₛCₛAₛgₛaₛgₛgₛaₛcₛaₛtₛaₛaₛTₛTₛC** |
| SEQ ID NO: 40 | **GₛAₛTₛgₛtₛcₛtₛgₛgₛgₛtₛtₛcₛTₛCₛC** |
| SEQ ID NO: 41 | **TₛTₛCₛtₛtₛcₛtₛtₛgₛtₛgₛaₛtₛCₛCₛA** |
| SEQ ID NO: 42 | **AₛAₛGₛaₛaₛaₛaₛtₛcₛcₛtₛgₛtₛgₛTₛCₛA** |
| SEQ ID NO: 43 | **TₛCₛTₛcₛcₛtₛgₛaₛaₛaₛcₛcₛtₛCₛTₛC** |
| SEQ ID NO: 44 | **GₛAₛTₛtₛtₛtₛaₛgₛaₛgₛaₛgₛaₛGₛGₛA** |
| SEQ ID NO: 45 | **AₛGₛTₛgₛaₛtₛtₛtₛtₛaₛgₛaₛAₛGₛA** |
| SEQ ID NO: 46 | **TₛCₛCₛtₛgₛcₛtₛtₛaₛgₛtₛaₛTₛTₛT** |
| SEQ ID NO: 47 | **T_{S}T_{S}C_{S}t_{S}c_{S}cₛt_{S}gₛcₛt_{S}t_{S}a_{S}g_{S}T_{S}G_{S}A** |
| SEQ ID NO: 48 | **GₛCₛCₛaₛcₛcₛaₛtₛgₛaₛcₛgₛtₛGₛCₛA** |
| SEQ ID NO: 49 | **AₛCₛCₛaₛtₛgₛaₛcₛgₛtₛgₛcₛaₛTₛCₛA** |
| SEQ ID NO: 50 | **GₛAₛTₛtₛtₛcₛaₛgₛaₛgₛaₛgₛaₛGₛGₛA** |
| SEQ ID NO: 51 | **AₛGₛTₛgₛaₛtₛtₛtₛcₛaₛgₛaₛgₛAₛGₛA** |
| SEQ ID NO: 52 | **TₛCₛCₛtₛgₛcₛtₛcₛaₛgₛtₛgₛaₛTₛTₛT** |
| SEQ ID NO: 53 | **TₛTₛCₛtₛcₛcₛtₛgₛcₛtₛcₛaₛgₛTₛGₛA** |
| SEQ ID NO: 54 | **GₛCₛCₛaₛcₛcₛaₛtₛgₛaₛtₛgₛtₛGₛCₛA** |
| SEQ ID NO: 55 | **AₛCₛCₛaₛtₛgₛaₛtₛgₛtₛgₛcₛaₛTₛCₛA** |

### Example 4: In vitro model: Cell culture

The effect of antisense oligonucleotides on target nucleic acid expression can be tested in any of a variety of cell types provided that the target nucleic acid is present at measurable levels. The target can be expressed endogenously or by transient or stable transfection of a nucleic acid encoding said target. The expression level of target nucleic acid can be routinely determined using, for example, Northern blot analysis, Real-Time PCR, Ribonuclease protection assays. The following cell types are provided for illustrative purposes, but other cell types can be routinely used, provided that the target is expressed in the cell type chosen.
Cells were cultured in the appropriate medium as described below and maintained at 37°C at 95-98% humidity and 5% CO₂. Cells were routinely passaged 2-3 times weekly.
MCF7: The human breast adenocarcinoma cell line MCF7 was cultured in Eagel MEM (Sigma) + 10% fetal bovine serum (FBS) + 2 mM Glutamax I + gentamicin (25µg/ml) + 1x Non Essential Amino Acid.
PC3: The human prostate adenocarcinoma cell line PC3 was cultured in DMEM (Sigma) + 10% fetal bovine serum (FBS) + 2 mM Glutamax I + gentamicin (25µg/ml).

### Example 5: In vitro model: Treatment with antisense oligonucleotide

The cell lines listed in example 4 were treated with oligonucleotide using the cationic liposome formulation LipofectAMINE 2000 (Gibco) as transfection vehicle. Cells were - seeded in 6-well cell culture plates (NUNC) and treated when 80-90% confluent. Oligo concentrations used ranged from 0.8 nM to 20 nM final concentration. Formulation of oligo-lipid complexes were carried out essentially as described by the manufacturer using serum-free OptiMEM (Gibco) and a final lipid concentration of 2.5 µg/mL (PC3) or 5 µg/mL (MCF7) LipofectAMINE 2000. Cells were incubated at 37°C for 4 hours and treatment was stopped by removal of oligo-containing culture medium. Cells were washed and serum-containing media was added. After oligo treatment cells were allowed to recover for 20 hours before they were harvested for RNA analysis.

### Example 6: In vitro model: Extraction of RNA and cDNA synthesis

Total RNA Isolation and First strand synthesis: Total RNA was extracted from cells transfected as described above and using the Qiagen RNeasy kit (Qiagen cat. no. 74104) according to the manufacturer's instructions. First strand synthesis was performed using Reverse Transcriptase reagents from Ambion according to the manufacturer's instructions. For each sample 0.5 µg total RNA was adjusted to (10.8 µl) with RNase free H₂O and mixed with 2 µl random decamers (50 µM) and 4 µl dNTP mix (2.5 mM each dNTP) and heated to 70 °C for 3 min arfetr which the samples were rapidly cooled on ice. After cooling the samples on ice, 2 µl 10x Buffer RT, 1 µl MMLV Reverse Transcriptase (100 U/µl) and 0.25 µl RNase inhibitor (10 U/µl) was added to each sample, followed by incubation at 42 °C for 60 min, heat inactivation of the enzyme at 95°C for 10 min and then the sample was cooled to 4 °C.

### Example 7: In vitro model: Analysis of Oligonucleotide Inhibition of PIK3CA Expression by Real-time PCR

Antisense modulation of PIK3CA expression can be assayed in a variety of ways known in the art. For example, PIK3CA mRNA levels can be quantitated by, e.g., Northern blot analysis, competitive polymerase chain reaction (PCR), or real-time PCR. Real-time quantitative PCR is presently preferred. RNA analysis can be performed on total cellular RNA or mRNA.

Methods of RNA isolation and RNA analysis such as Northern blot analysis is routine in the art and is taught in, for example, Current Protocols in Molecular Biology, John Wiley and Sons.
Real-time quantitative (PCR) can be conveniently accomplished using the commercially available Multi-Color Real Time PCR Detection System, available from Applied Biosystem. Real-time Quantitative PCR Analysis of PIK3CA mRNA Levels : The sample content of human PIK3CA mRNA was quantified using the human PIK3CA ABI Prism Pre-Developed TaqMan Assay Reagents (Applied Biosystems cat. no. Hs00180679_m1 according to the manufacturer's instructions.
Glyceraldehyde-3-phosphate dehydrogenase (GAPDH) mRNA quantity was used as an endogenous control for normalizing any variance in sample preparation.
The sample content of human GAPDH mRNA was quantified using the human GAPDH ABI Prism Pre-Developed TaqMan Assay Reagent (Applied Biosystems cat. no. 4310884E) according to the manufacturer's instructions.
Real-time Quantitative PCR is a technique well known in the art and is taught in for example Heid et al. Real time quantitative PCR, Genome Research (1996), 6: 986-994.
Real time PCR: The cDNA from the first strand synthesis performed as described in example 5 was diluted 2-20 times, and analyzed by real time quantitative PCR using Taqman 7500 FAST or 7900 FAST from Applied Biosystems. The primers and probe were mixed with 2 x Taqman Fast Universal PCR master mix (2x) (Applied Biosystems Cat.# 4364103) and added to 4 µl cDNA to a final volume of 10 µl. Each sample was analysed in duplicate. Assaying 2 fold dilutions of a cDNA that had been prepared on material purified from a cell line expressing the RNA of interest generated standard curves for the assays. Sterile H₂O was used instead of cDNA for the no template control. PCR program: 95° C for 30 seconds, followed by 40 cycles of 95° C, 3 seconds, 60° C, 20-30 seconds. Relative quantities of target mRNA sequence were determined from the calculated Threshold cycle using the Applied Biosystems Fast System SDS Software Version 1.3.1.21. or SDS Software Version 2.3.

### Example 8: In vitro analysis: Antisense Inhibition of Human PIK3CA mRNA Expression by oligonucleotide compounds

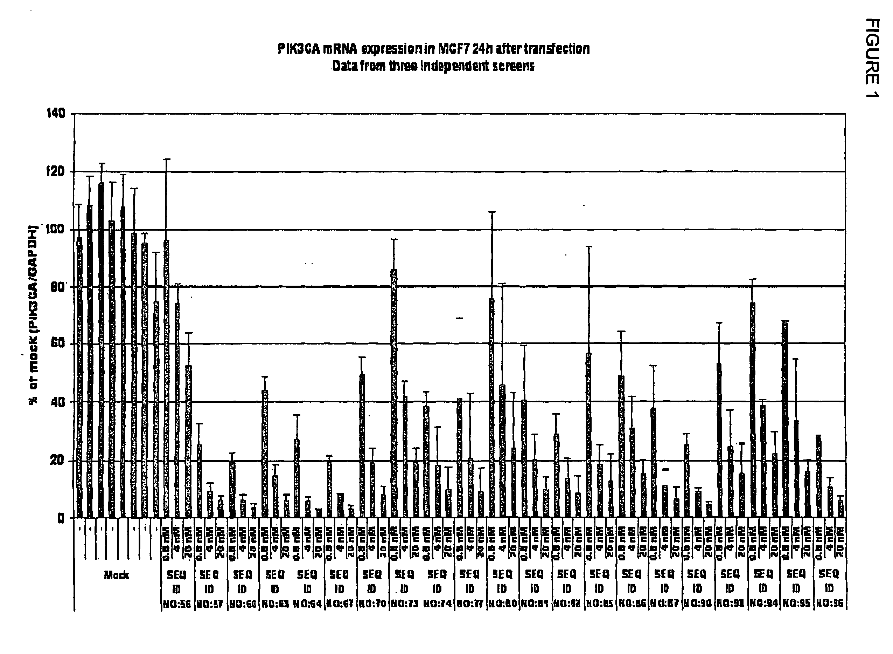

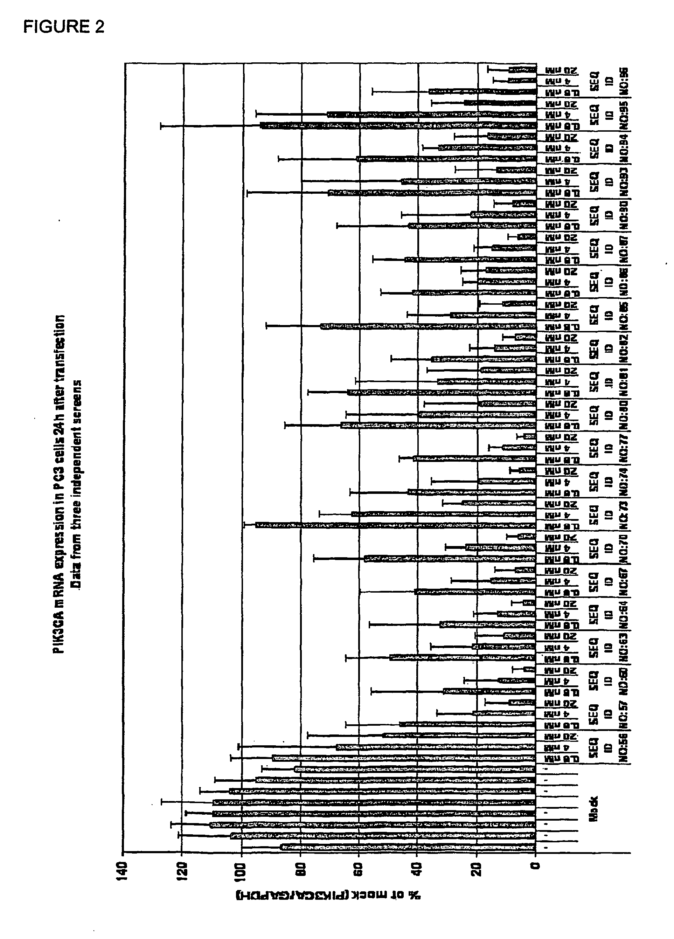

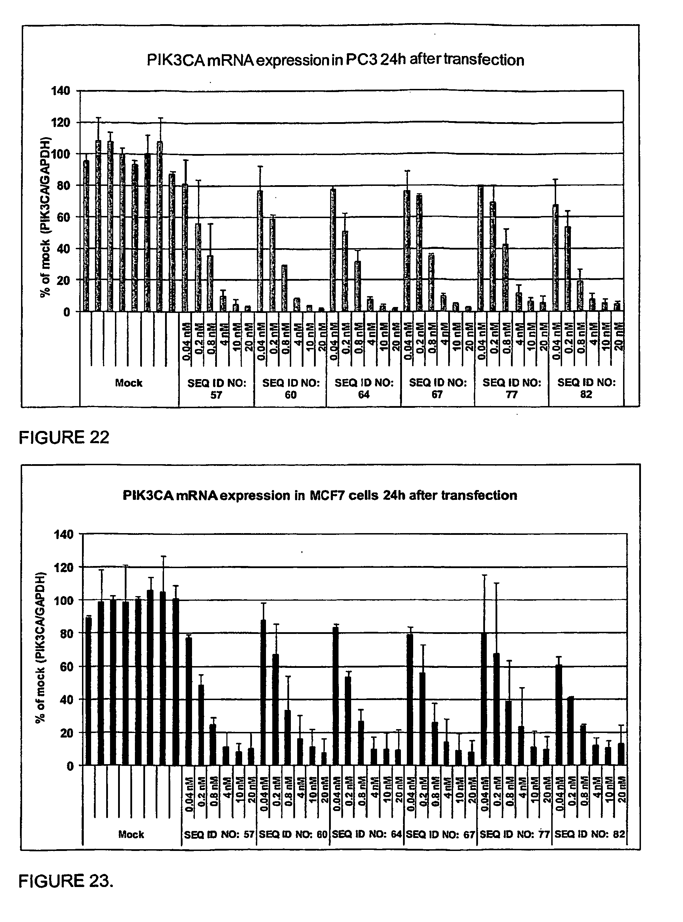

Oligonucleotides presented in Table 3 were evaluated for their potential to knockdown of human PIK3CA mRNA at concentrations of 0.8, 4 and 20 nM in PC3 cells and MCF7 cells (see Figures 1 and 2). The most active oligonucleotides, having SEQ ID NO: 57, 60, 64, 67, 77 and 82 were further evaluated for their knockdown of human PIK3CA at the concentrations 0.04, 0.2, 0.8, 4, 10 and 20nM in PC3 and MCF7 cells (see Figures 22 and 23).

**Table 4: Antisense Inhibition of Human PIK3CA mRNA expression by oligonucleotides.**

| The data in Table 4 are presented as percentage down-regulation relative to mock transfected cells at 20 nM. Lower case letters represent DNA units, bold upper case letters represent β-D-oxy-LNA units. All LNA C are 5'methyl C. Subscript "s" represents phosphorothioate linkage. | | | |
|---|---|---|---|
| **Test substance** | **Sequence (5'-3')** | Percent inhibition of PIK3CA in MCF7 | Percent inhibition of PIK3CA in PC3 |
| SEQ ID NO:56 | **GₛAₛGₛgₛcₛaₛtₛtₛcₛtₛaₛaₛaₛGₛTₛC** | 47 | 48 |
| SEQ ID NO:57 | **AₛTₛTₛcₛtₛtₛcₛcₛcₛtₛtₛtₛcₛTₛGₛC** | 94 | 91 |
| SEQ ID NO:58 | **TₛTₛcₛtₛtₛcₛcₛcₛtₛtₛtₛcₛTₛG** | n.d. | n.d. |
| SEQ ID NO:59 | **TₛcₛtₛtₛcₛcₛcₛtₛtₛtₛcₛT** | n.d. | n.d. |
| SEQ ID NO:60 | **TₛAₛGₛaₛcₛaₛtₛaₛcₛaₛtₛtₛgₛCₛTₛC** | 96 | 96 |
| SEQ ID NO:61 | **AₛGₛaₛcₛaₛtₛaₛcₛaₛtₛtₛgₛCₛT** | n.d. | n.d. |
| SEQ ID NO:62 | **GₛaₛcₛaₛtₛaₛcₛaₛtₛtₛgₛC** | n.d. | n.d. |
| SEQ ID NO:63 | **TₛAₛCₛtₛtₛgₛcₛcₛcₛtₛgₛaₛtₛAₛTₛT** | 94 | 89 |
| SEQ ID NO:64 | **CₛAₛCₛaₛtₛaₛaₛgₛgₛgₛtₛtₛcₛTₛCₛC** | 97 | 95 |
| SEQ ID NO:65 | **AₛCₛaₛtₛaₛaₛgₛgₛgₛtₛtₛcₛTₛC** | n.d. | n.d. |
| SEQ lD NO:66 | **CₛaₛtₛaₛaₛgₛgₛgₛtₛtₛcₛT** | n.d. | n.d. |
| SEQ ID NO:67 | **AₛGₛCₛCₛaₛtₛtₛcₛaₛtₛtₛcₛcₛAₛCₛC** | 97 | 93 |
| SEQ ID NO:68 | **GₛCₛcₛaₛtₛtₛcₛaₛtₛtₛcₛcₛAₛC** | n.d. | n.d. |
| SEQ ID NO:69 | **CₛcₛaₛtₛtₛcₛaₛtₛtₛcₛcₛA** | n.d. | n.d. |
| SEQ ID NO:70 | **CₛAₛGₛtₛaₛcₛcₛaₛcₛcₛaₛaₛtₛAₛGₛG** | 92 | 94 |
| SEQ ID NO:71 | **AₛGₛtₛaₛaₛcₛcₛaₛaₛtₛAₛG** | n.d. | n.d. |
| SEQ ID NO:72 | **GₛtₛaₛaₛcₛaₛcₛcₛaₛaₛtₛA** | n.d. | n.d. |
| SEQ ID NO:73 | **AₛAₛCₛtₛcₛcₛaₛaₛcₛtₛcₛtₛaₛAₛGₛC** | 81 | 75 |
| SEQ ID NO:74 | **CₛAₛGₛaₛcₛaₛgₛaₛaₛgₛcₛaₛaₛTₛTₛT** | 90 | 94 |
| SEQ ID NO:75 | **AₛGₛaₛcₛaₛgₛaₛaₛgₛcₛaₛaₛTₛT** | n.d. | n.d. |
| SEQ ID NO:76 | **GₛaₛcₛaₛgₛaₛaₛgₛcₛaₛaₛT** | n.d. | n.d. |
| SEQ ID NO:77 | **T_{S}TₛAₛtₛtₛgₛtₛgₛcₛaₛtₛcₛtₛCₛAₛG** | 91 | 96 |
| SEQ ID NO:78 | **TₛAₛtₛtₛgₛtₛgₛcₛaₛtₛcₛtₛCₛA** | n.d. | n.d. |
| SEQ ID NO:79 | **AₛtₛtₛgₛtₛgₛcₛaₛtₛcₛtₛC** | n.d. | n.d. |
| SEQ ID NO:80 | **GₛCₛAₛgₛaₛgₛgₛaₛcₛaₛtₛaₛaₛTₛTₛC** | 76 | 80 |
| SEQ ID NO:81 | **GₛAₛTₛgₛtₛcₛtₛgₛgₛgₛtₛtₛcₛTₛCₛC** | 90 | 81 |
| SEQ ID NO:82 | **TₛTₛCₛtₛtₛcₛtₛtₛgₛtₛgₛaₛtₛCₛCₛA** | 92 | 93 |
| SEQ ID NO:83 | **TₛCₛtₛtₛcₛtₛtₛgₛtₛgₛaₛtₛCₛC** | n.d. | n.d. |
| SEQ ID NO:84 | **CₛtₛtₛcₛtₛtₛgₛtₛgₛaₛtₛC** | n.d. | n.d. |
| SEQ ID NO:85 | **AₛAₛGₛaₛaₛaₛtₛcₛcₛtₛgₛtₛgₛTₛCₛA** | 87 | 88 |
| SEQ ID NO:86 | **TₛCₛTₛcₛcₛtₛgₛaₛaₛaₛcₛcₛtₛCₛTₛC** | 85 | 83 |
| SEQ ID NO:87 | **GₛAₛTₛtₛtₛcₛaₛgₛaₛgₛaₛgₛaₛGₛGₛA** | 94 | 94 |
| SEQ ID NO:88 | **AₛTₛtₛtₛcₛaₛgₛaₛgₛaₛgₛaₛGₛG** | n.d. | n.d. |
| SEQ ID NO:89 | **TₛtₛtₛcₛaₛgₛaₛgₛaₛgₛaₛG** | n.d. | n.d. |
| SEQ ID NO:90 | **AₛGₛTₛgₛaₛtₛtₛtₛcₛaₛgₛaₛgₛAₛGₛA** | 96 | 92 |
| SEO ID NO:91 | **GₛTₛgₛaₛtₛtₛtₛcₛaₛgₛaₛgₛAₛG** | n.d. | n.d. |
| SEO ID NO:92 | **TₛgₛaₛtₛtₛtₛcₛaₛgₛaₛgₛA** | n.d. | n.d. |
| SEQ ID NO:93 | **TₛCₛCₛtₛgₛcₛtₛcₛaₛgₛtₛgₛaₛTₛTₛT** | 85 | 86 |
| SEQ ID NO:94 | **TₛTₛCₛtₛCₛCₛtₛgₛcₛtₛcₛaₛgₛTₛGₛA** | 78 | 83 |
| SEQ ID NO:95 | **GₛCₛCₛaₛCₛcₛaₛtₛgₛaₛtₛgₛtₛGₛCₛA** | 84 | 75 |
| SEQ ID NO:96 | **AₛCₛCₛaₛtₛgₛaₛtₛgₛtₛgₛcₛaₛTₛCₛA** | 94 | 91 |
| SEQ ID NO:97 | **CₛCₛaₛtₛgₛaₛtₛgₛtₛgₛcₛaₛTₛC** | n.d. | n.d. |
| SEQ ID NO:98 | **CₛaₛtₛgₛaₛtₛgₛtₛgₛcₛaₛT** | n.d. | n.d. |
| SEQ ID NO:99 | **GₛAₛTₛtₛtₛtₛaₛgₛaₛgₛaₛgₛaₛGₛGₛA** | n.d. | n.d. |
| SEQ ID NO: 137 | **AₛTₛTₛtₛtₛaₛgₛaₛgₛaₛgₛAₛGₛG** | n.d. | n.d. |
| SEQ ID NO: 138 | **TₛTₛtₛtₛaₛgₛaₛgₛaₛgₛAₛG** | n.d. | n.d. |
| SEQ ID NO:100 | **AₛGₛTₛgₛaₛtₛtₛtₛtₛaₛgₛaₛgₛAₛGₛA** | n.d. | n.d. |
| SEQ ID NO: 139 | **GₛTₛGₛaₛtₛtₛtₛtₛaₛgₛaₛGₛAₛG** | n.d. | n.d. |
| SEQ ID NO: 140 | **TₛGₛaₛtₛtₛtₛtₛaₛgₛaₛGₛA** | n.d. | n.d. |
| SEQ ID NO:101 | **TₛCₛCₛtₛgₛcₛtₛtₛaₛgₛtₛgₛaₛTₛTₛT** | n.d. | n.d. |
| SEO ID NO: 141 | **CₛCₛTₛgₛcₛtₛtₛaₛgₛtₛgₛAₛTₛT** | n.d. | n.d. |
| SEQ ID NO: 142 | **CₛTₛgₛcₛtₛtₛaₛgₛtₛgAₛT** | n.d. | n.d. |
| SEQ ID NO:102 | **TₛTₛCₛtₛcₛcₛtₛgₛcₛtₛtₛaₛgₛTₛGₛA** | n.d. | n.d. |
| SEQ ID NO: 143 | **TₛCₛTₛcₛcₛtₛgₛcₛtₛtₛaₛGₛTₛG** | n.d. | n.d. |
| SEQ ID NO: 144 | **CₛTₛcₛcₛtₛgₛcₛtₛtₛaₛGₛT** | n.d. | n.d. |
| SEQ ID NO:103 | **GₛCₛCₛaₛcₛcₛaₛtₛgₛaₛcₛgₛtₛGₛCₛA** | n.d. | n.d. |
| SEQ ID NO:145 | **CₛCₛAₛcₛcₛaₛtₛgₛaₛcₛgₛTₛGₛC** | n.d. | n.d. |
| SEQ ID NO:146 | **CₛAₛcₛcₛaₛtₛgₛaₛcₛgₛTₛG** | n.d. | n.d. |
| SEQ ID NO:104 | **AₛCₛCₛaₛtₛgₛaₛcₛgₛtₛgₛcₛaₛTₛCₛA** | n.d. | n.d. |
| SEQ ID NO:147 | **CₛCₛAₛtₛgₛaₛcₛgₛtₛgₛcₛAₛTₛC** | n.d. | n.d. |
| SEO ID NO:148 | **CₛAₛtₛgₛaₛcₛgₛtₛgₛcₛAₛT** | n.d. | n.d. |

As shown in Table 4, oligonucleotides of SEQ ID NOs: 57, 60, 64, 67, 70, 74, 77, 82, 87, 90 and 96 demonstrated about 90% or greater inhibition of PIK3CA mRNA expression at 20 nM in PC3 and MCF7 cells in these experiments and are therefore preferred.
Also preferred are oligonucleotides based on the illustrated antisense oligo sequences, for example varying the length (shorter or longer) and/or nucleobase content (e.g. the type and/or proportion of analogue units), which also provide good inhibition of PIK3CA expression.

### Example 9: In vitro analysis: Effecf of Antisense Inhibition of Human PIK3CA mRNA on cell proliferafion (MTS assay).

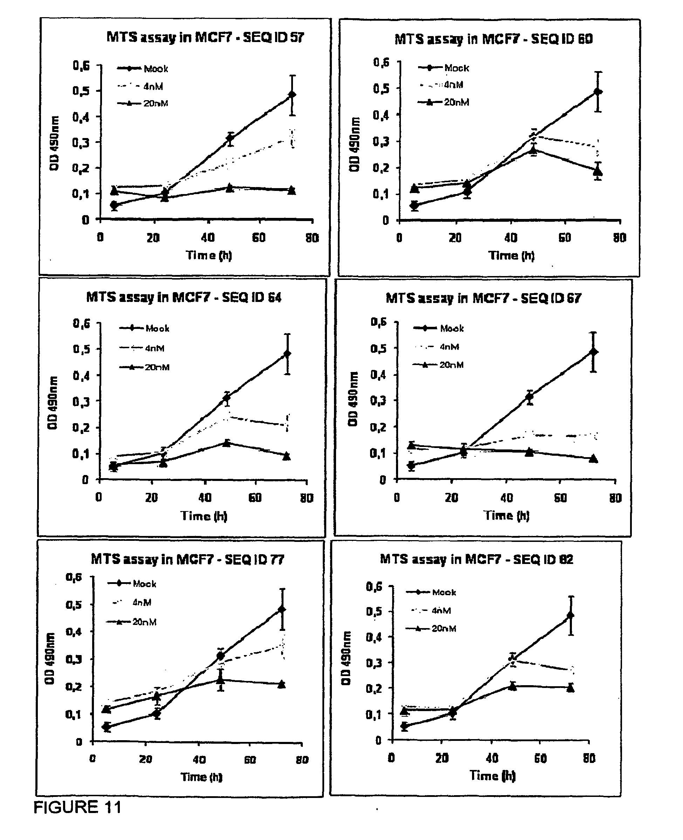

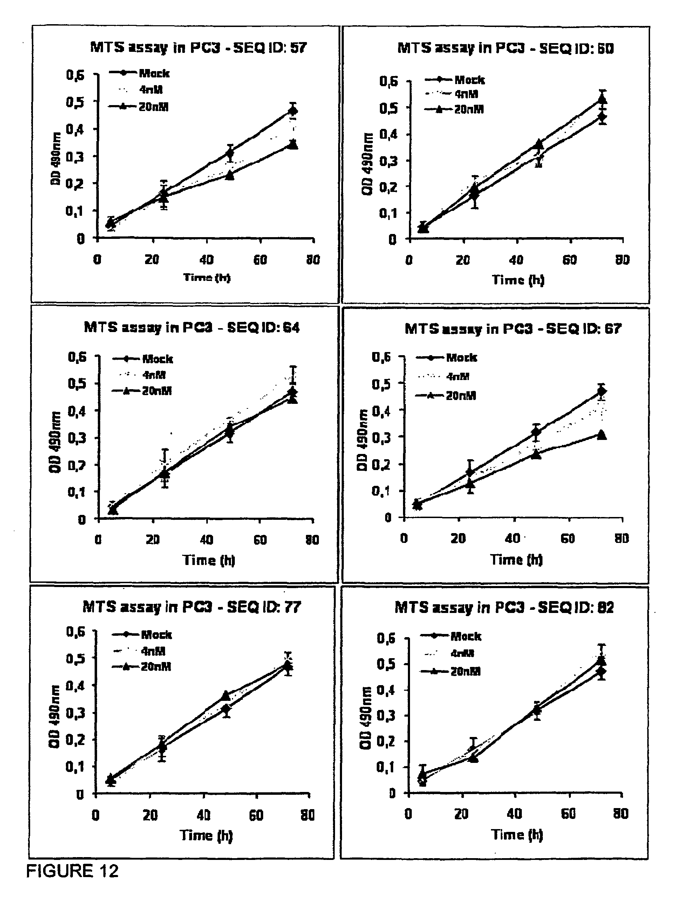

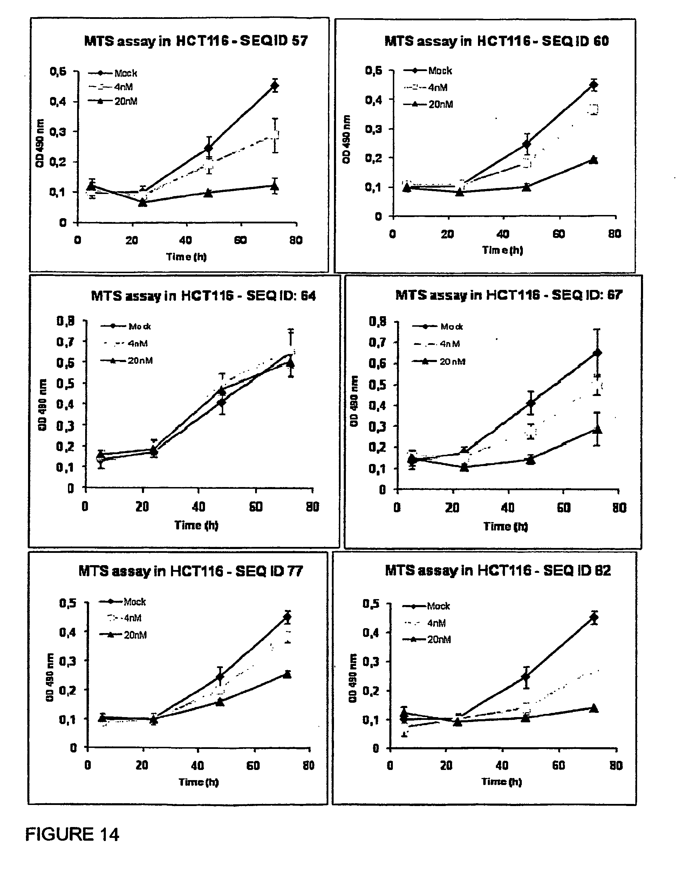

MCF7 breast cancer, PC3 prostate cancer and HCT116 colon cancer cells were treated with oligonucleotide using the cationic liposome formulation LipofectAMINE 2000 (Invitrogen) as transfection vehicle. Cells were seeded in 6-well culture plates (NUNC) the day before transfection at a density of 2.5 x 10⁵ cells/well (MCF7 and HCT116) or 2.4 x 10⁵ cells/well (PC3). The cells were treated when 75-90% confluent with different concentrations of oligos. Formulation of oligo-lipid complexes was carried out using serum-free OptiMEM (Invitrogen) and a final lipid concentration of 2.5 µg/ml (PC3), 5 µg/ml (MCF7) or 10 µg/ml (HCT116) LipofectAMINE 2000. Cells were incubated at 37°C for 4 hours and transfection was stopped by removal of oligo-containing culture medium. After 4 hours of treatment, media was removed and cells were trypsinized and seeded to a density of 5000 cells per well in clear 96 well plate (Scientific Orange no. 1472030100) in 100 µl media. Viable cells were measured at the times indicated by adding 10 µl the tetrazolium compound [3-(4,5-dimethyl-2-yl)-5-(3-carboxymethoxyphenyl)-2-(4-sulfophenyl)-2H-tetrazolium, inner salt; MTS] and an electron coupling reagent (phenazine ethosulfate; PES) (CellTiter 96® AQueous One Solution Cell Proliferation Assay, Promega). Viable cells were measured at 490 nm in a Powerwave (Biotek Instruments). The OD490 nm was plotted against time/h. *(See* *Figures 11**,* *12* *and* *14**).* As shown in figure 11, oligonucleotides of SEQ ID NOs: 57, 60, 64, 67, 77 and 82 inhibited proliferation of the breast cancer cell line MCF7, while none of the oligonucleotides had any pronounced effect on proliferation of PC3 cells (Fig 12). In HCT116 cells, oligonucleotides with SEQ ID NO:s 57, 60, 67, 77 and 82 inhibited the proliferation. The cell line MCF7 has the E545K hot-spot mutation and HCT116 has the H1047R hot-spot mutation in the PIK3CA gene, which makes them more dependent on PIK3CA signalling than the PC3 cell line which has no reported hot-spot mutation in the gene. The hot-spot mutations are activating mutations.

### Example 10: In vitro analysis: Effect on Caspase-3/7 induction after antisense inhibition of PIK3CA in human cancer cell lines

PC3 prostate cancer and HCT116 colon cancer cells were treated with oligonucleotide using the cationic liposome formulation LipofectAMINE 2000 (Invitrogen) as transfection vehicle. Cells were seeded in 6-well culture plates (NUNC) the day before transfection at a density of 2.5 x 10⁵ cells/well (MCF7 and HCT116) or 2.4 x 10⁵ cells/well (PC3). The cells were treated when 75-90% confluent with different concentrations of olidos. Formulation of oligo-lipid complexes was carried out using serum-free OptiMEM (Invitrogen) and a final lipid concentration of 2.5 µg/ml (PC3) or 10 µg/ml (HCT116) LipofectAMINE 2000. Cells were incubated at 37°C for 4 hours and transfection was stopped by removal of oligo-containing culture medium.

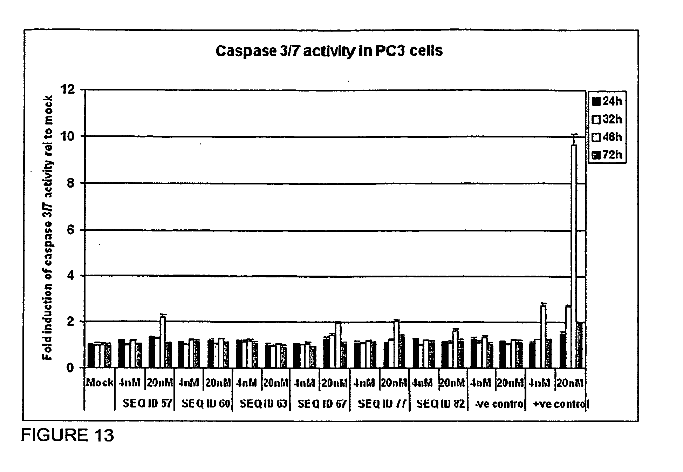

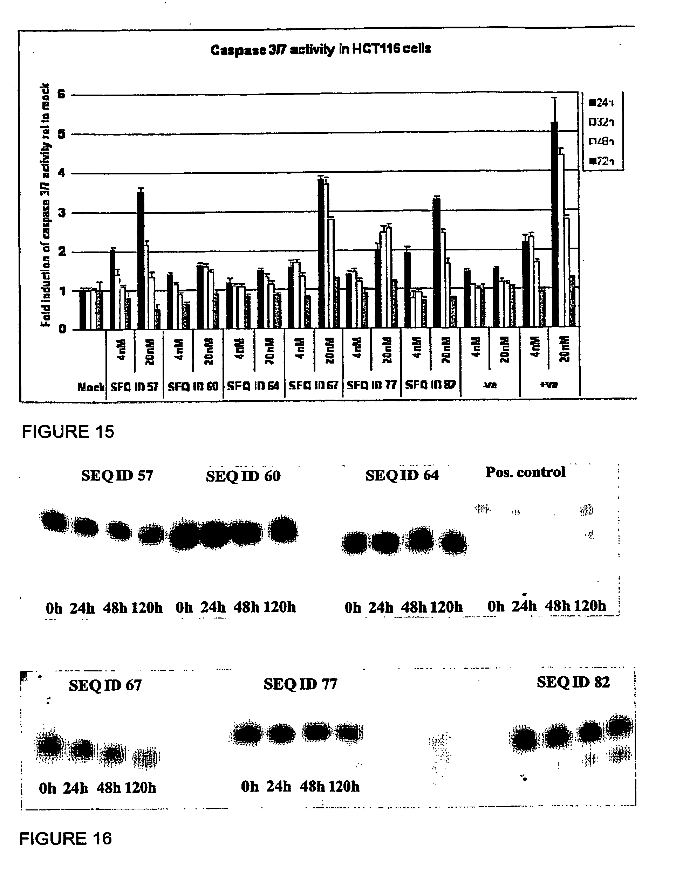

After 4 hours of treatment, media was removed and cells were trypsinized and seeded to a density of 5000 cells per well in white 96 well plate (Nunc) in 100 µl media. Caspase 3/7 activity was measured at the times indicated by adding 100 µl Caspase-Glo 3/7 assay (promega). Caspase 3/7 activity was measured using a luminometer. The the caspase 3/7 activities were measuered at four different time points 24h, 32h, 48h and 72h *(See* *Figure 13* *and* *Figure 15*). SEQ ID NO: 57, 67, 77 and 82 showed dose dependent induction in Caspase 3/7 activity in HCT116 cells, which have the H1047R hot-spot mutation (Figure 15), while the oligonucleotides had no effect on caspase 3/7 induction in PC3 cells.

### Example 11. In vitro analysis: Biostability of PIK3CA oligonucleotides in mouse plasma

Mouse plasma (Lithium heparin plasma fromBomTac:NMRI mice, collected 14-09-05, Taconic Europe) was defrosted and aliquoted into tubes with 45 µl plasmaltube. Following, 5 µl oligo (200 µM) was added to the 45 µl plasma to a final concentration of 20 µM. After thorough mixing, the samples were incubated at 37°C for 0-120 hrs. At different time points (0h, 24h, 48h and 120h) samples were collected and the reaction was quenched by snap freezing the samples in liquid nitrogen. For analysis, samples were added loading buffer and analysed by electrophoresis on a PAGE-sequencing gel under denaturing conditions. The PIK3CA oligonucleotides showed high plasma stability for up to 120 hrs and the results are shown in Figure 16.

### Example 12. In vitro analysis: Tm measurement of PIK3CA oligonucleotides against complementary RNA.

The melting temperature of the LNAoligo/RNA duplexes was determined using a UV-spectrometry system with corresponding software (Perkin Elmer, Fremont, USA). The LNA oligo and its complemetary RNA were added in final concentrations of 1.5 µM to the Tₘ-buffer (200 nM NaCl, 0.2 nM EDTA, 20 mM NaP, pH 7.0). Duplex formation was prepared by heating the samples to 95°C for 3 min followed by cooling at room temperature for 30 min.

Melting temperature (Tₘ) values were measured in a Lambda 25 UV/VIS spectrometer (Perkin Elmer) and data was collected and analysed using the TempLab software (Perkin Elmer). The instrument was programmed to heat the oligo duplex sample from 20-95°C and afterwards cooling the sample to 25°C. During this process the absorbance at 260 nm was recorded. The melting curves were used to calculate Tₘ values.

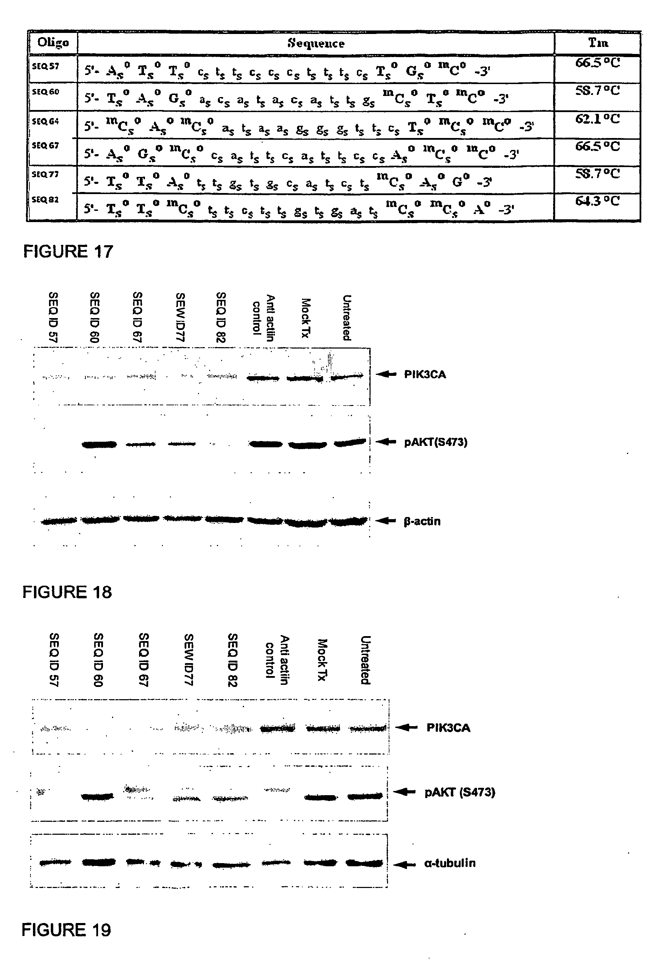

The Tm values for SEQ ID NO: 57, 60, 64, 67, 77 and 82 are presented in Figure 17.

### Example 13: In vitro analysis: Down-regulation of PIK3CA and pAkt in 15PC3 cells.

15PC3 cell were transfected with 30 nM of LNA or treated with LY294200 (a small molecule PI3K inhibitor) for 24 and 48 h. WB: 48 h, 15 ug protein/lane, 8% gel. Significant reduction of PIK3CA levels was observed after transfection of 15PC3 cells with PIK3CA LNAs. (Figure 19) pAkt levels were reduced up to 80% at 24 h post transfection determined by ELISA.

(Figure 19). Transfection with the oligomer having the sequence of SEQ ID NO: 82 had less effect on downstream signals compared to the other tested LNAs.

### Example 14: In vivo analysis: Down-regulation of mouse PIK3CA in mouse liver after i.v. administration of PIK3CA oligonucleotides.

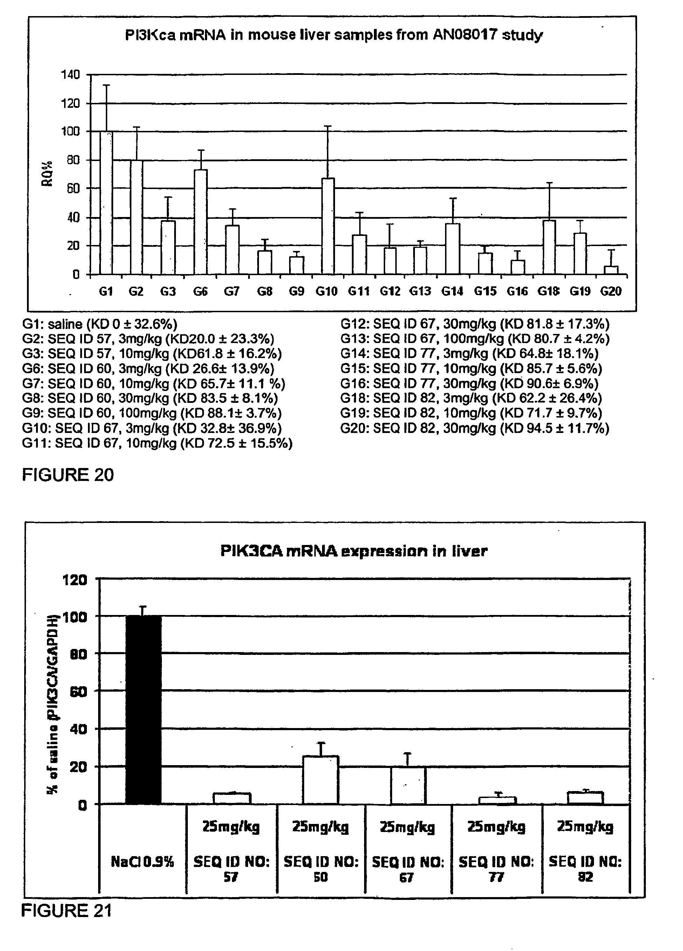

Female NMRI mice received i.v. injection of oligonucleotides having the sequences of SEQ ID NO: 57, 60, 67, 77 and 82 on three consecutive days at a dosage of 25mg/kg, Animals were sacrificed 24h after last dosing. The liver was stored in RNA/ater stabilizing solution until use. Total RNA was extracted from liver tissue and PIK3CA mRNA levels were analyzed with qPCR. Data were compared to PIK3CA expression in saline treated control animals. As shown in Figure 21, all oligonucleotides in the study showed potent down-regulation of PIK3CA mRNA.

All PIK3CA oligomers used in the second screening showed potent down-regulation of PIK3CA in PC3 and MCF7 cells with IC₅₀ values below 1 nM. SEQ ID NO: 82 also shows down-regulation of the control target beta-catenin gene in both MCF7 and PC3 cells (SEQ ID NO: 82 has 2 mismatches when compared to the reverse complement of the best-aligned target region of beta-catenin).

### Example 15: In vivo analysis: Effect of oligomers on lung cancer tumor size in mice

Six- to seven-week old male athymic nu/nu mice (Harlan Sprague Dawley) weighing an average of 27.3±2.4g were used in the study. Five million cells of Calu-6 (lung cancer cell line) were suspended in PBS (Gibco#14190) were injected subcutaneously into each mouse. The mice were injected with two hundred µl of oligomer intravenously when the average tumor size reached 150 mm³. Oligomers were given every 3 days for a total of 5 dosings. The control vehicles were given the same dosing regimen as the oligomers. The tumor volumes for each mouse were determined by measuring two dimensions with calipers and calculated using the formula: tumor volume = (length x width²)/2). Figure 20 shows down regulation of PIK3CA expression in liver by oligomers of the description.

The follwoing oligomers were found to have a good toxicity profile in terms of good animal survivial in in vivo experiemnts at 3mg/kg dose - SEQ IDs 60, 67, 77 & 82. SEQ IDs 60,67 and 77 showed a good toxicity profile at 10mglkg dosage.

| **Table 6: Effect of oligomers on PIK3CA mRNA in mouse liver and tumor and on tumor size** | | | | |
|---|---|---|---|---|
| **Group** | **Dose (mg/kg)** | **Tumor KD** | **Liver KD** | **TGI on day 12 (%)** |
| **Control** | **---** | **ND** | | **---** |
| **Control** | **-** | **ND** | | **-** |
| **SEQ ID 57** | **3** | **ND** | **20 ±23** | **22.9** |
| | **10** | **ND** | **62 ±16** | **18.0** |
| **SEQ ID 60** | **3** | **ND** | **27 ± 14** | **24.7** |
| | **10** | **ND** | **66 ± 11** | **53.6** |
| | **30** | **ND** | **84 ± 8** | **49.4** |
| | **100** | **ND** | **88 ± 4.0** | **54.1** |
| **SEQ ID 67** | **3** | **16 ± 12** | **33 ± 37** | **50.6** |
| | **10** | **36 ±11** | **73 ±16** | **50.5** |
| | **30** | **58 ±5.2** | **82 ± 17** | **44.5** |
| | **100** | **65 ±12** | **81 ± 4.2** | **31.5** |
| **SEQ ID 77** | **3** | **ND** | **65 ±18** | **57.6** |
| | **10** | **ND** | **86 ± 5.6** | **21.2** |
| | **30** | **ND** | **91 ± 6.9** | **48.5** |
| **SEQ ID 82** | **3** | **ND** | **62 ± 26** | **37.5** |
| | **10** | **ND** | **72 ± 10** | **50.0** |

### Example 16: Preparation of conjugates of oligomers with polyethylene glycol

The oligomers having sequences shown as SEQ ID NO: 60 or SEQ ID NO: 87 are functionalized on the 5' terminus by attaching an aminoalkyl group, such as hexan-1-amine blocked with a blocking group such as Fmoc to the 5' phosphate groups of the oligomers using routine phosphoramidite chemistry, oxidizing the resultant compounds, deprotecting them and purifying them to achieve the functionalized oligomers, respectively, having the formulas (IA) and (IB): 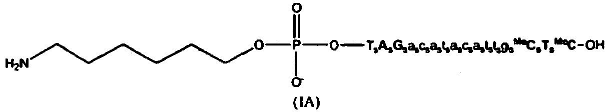 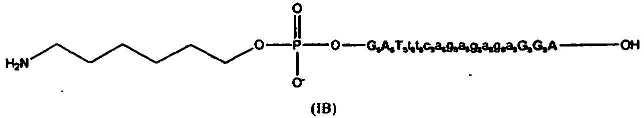 wherein the bold uppercase letters represent nucleoside analogue monomers, lowercase letters represent DNA monomers, the subscript "s" represents a phosphorothioate linkage, and ^{Me}C represents 5-methylcytosine.
A solution of activated PEG, such as the one shown in formula (II): 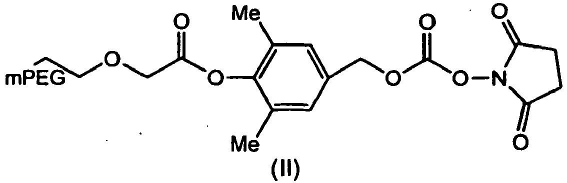 wherein the PEG moiety has an average molecular weight of 12,000, and each of the compounds of formulas (IA) and (IB) in PBS buffer are stirred in separate vessels at room temperature for 12 hours. The reaction solutions are extracted three times with methylene chloride and the combined organic layers are dried over magnesium sulphate and filtered and the solvent is evaporated under reduced pressure. The resulting residues are dissolved in double distilled water and loaded onto an anion exchange column. Unreacted PEG linker is eluted with water and the products are eluted with NH₄HCO₃ solution. Fractions containing pure products are pooled and lypophilized to yield the conjugates SEQ ID NOs: 60 and 87, respectively as show in formulas (IIIA) and (IIIB): 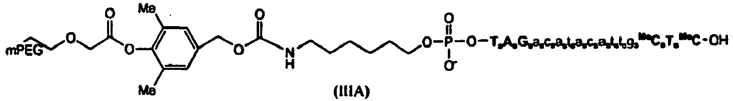 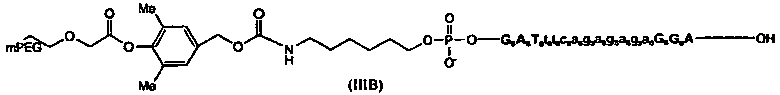 wherein each of the oligomers of SEQ ID NOs: 60 and 87 is attached to a PEG polymer having average molecular weight of 12,000 via a releasable linker.

Chemical structures of PEG polymer conjugates that can be made with oligomers having sequences shown in SEQ ID NOs: 67, 77 and 82 using the process described above are respectively shown in formulas (IVA), (IVB) and (IVC): 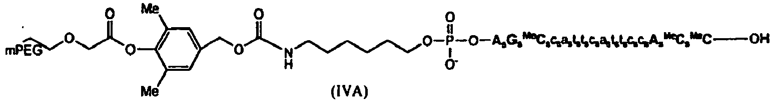 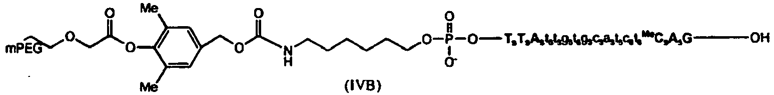 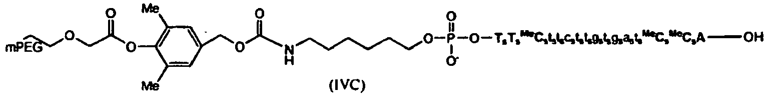 wherein bold uppercase letters represent beta-D-oxy-LNA monomers, lowercase letters represent DNA monomers, the subscript "s" represents a phosphorothioate linkage and ^{Me}C represent 5-methylcytosine.
Activated oligomers that can be used in this process to respectively make the conjugates shown in formulas (IVA), (IVB) and (IVC) have the chemical structures shown in formulas (VA), (VB) and (VC):

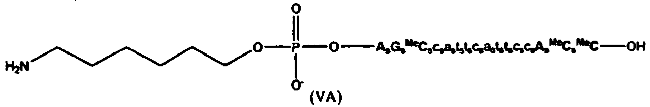

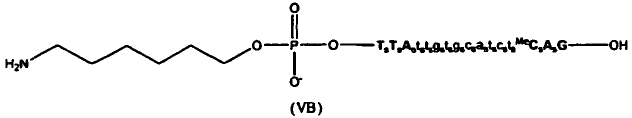

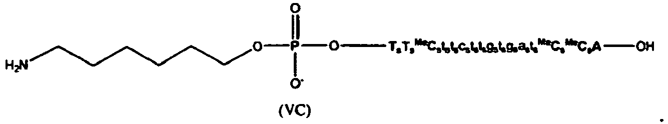

## Claims

1. An oligomer having a contiguous nucleotide sequence of a total of 10 - 30 nucleotides, wherein said contiguous nucleotide sequence is at least 80% homologous to the reverse complement of a corresponding region of a mammalian PIK3CA gene or mRNA, wherein said oligomer comprises at least one LNA unit; and wherein the contiguous nucleotide sequence comprises at least 10 contiguous nucleotides which are 100% identical to a corresponding region of SEQ ID NO 115 or SEQ ID NO 7.

2. The oligomer according to claim 1, wherein the contiguous nucleotide sequence comprises SEQ ID No 115 or SEQ ID No 7.

3. The oligomer according to claim 1 or 2, wherein the contiguous nucleotide sequence is 10-22 nucleotides in length.

4. The oligomer according to any one of claims 1 to 3, wherein the contiguous nucleotide sequence is 10 - 18 nucleotides in length.

5. The oligomer according to any one of claims 1 to 4, wherein the contiguous nucleotide sequence comprises other nucleotide analogues.

6. The oligomer according to claim 5, wherein the nucleotide analogues are sugar modified nucleotides, such as sugar modified nucleotides selected from the group consisting of: Locked Nucleic Acid (LNA) units; 2'-O-alkyl-RNA units, 2'-OMe-RNA units, 2'-amino-DNA units, and 2'-fluoro-DNA units.

7. The oligomer according to claim 5, wherein the nucleotide analogues are LNA.

8. The oligomer according to any one of claims 5 to 7 which is a gapmer.

9. The oligomer according to any one of claims 1 to 8, which is capable of down-regulating the expression of PIK3CA gene or mRNA in a cell which is expressing PIK3CA gene or mRNA.

10. The oligomer according to any one of claims 1 to 9, wherein the oligomer consists of
5'- AₛGₛ^{Me}CₛcₛaₛtₛtₛcₛaₛtₛtₛcₛcₛAₛ^{Me}Cₛ^{Me}C-3' (SEQ ID NO: 67)
wherein uppercase letters denote beta-D-oxy-LNA monomers and lowercase letters denote DNA monomers, the subscript "s" denotes a phosphorothioate linkage, and ^{Me}C denotes a beta-D-oxy-LNA monomer containing a 5-methylcytosine base.

11. A conjugate comprising the oligomer according to any one of claims 1 to 10, and at least one non-nucleotide or non-polynucleotide moiety covalently attached to said oligomer.

12. A pharmaceutical composition comprising the oligomer according to any one of claims 1 to 10, or the conjugate according to claim 11, and a pharmaceutically acceptable diluent, carrier, salt or adjuvant.

13. The oligomer according to any one of claims 1 to 10, or the conjugate according to claim 11, for use as a medicament, such as for the treatment of hyperproliferative diseases such as cancer.

14. The use of an oligomer according to any one of the claims 1 to 10, or a conjugate as defined in claim 11, for the manufacture of a medicament for the treatment of hyperproliferative diseases such as cancer.

15. An *in vitro* method for the inhibition of PIK3CA in a cell which is expressing PIK3CA, said method comprising administering an oligomer according to any one of the claims 1 to 10, or a conjugate according to claim 11 to said cell so as to inhibit PIK3CA in said cell.

## Patentansprüche

1. Oligomer mit einer zusammenhängenden Nukleotidsequenz von insgesamt 10 - 30 Nukleotiden, wobei die zusammenhängende Nukleotidsequenz eine Homologie von wenigstens 80% zu dem reversen Komplement eines entsprechenden Bereichs eines PIK3CA-Gens oder einer PIK3CA-mRNA eines Säugers aufweist, wobei das Oligomer wenigstens eine LNA-Einheit umfasst; und wobei die zusammenhängende Nukleotidsequenz wenigstens 10 zusammenhängende Nukleotide umfasst, die mit einem entsprechenden Bereich der SEQ ID NO 115 oder SEQ ID NO 7 zu 100% identisch sind.

2. Oligomer nach Anspruch 1, wobei die zusammenhängende Nukleotidsequenz SEQ ID Nr. 115 oder SEQ ID Nr. 7 umfasst.

3. Oligomer nach Anspruch 1 oder 2, wobei die zusammenhängende Nukleotidsequenz eine Länge von 10 - 22 Nukleotiden aufweist.

4. Oligomer nach einem der Ansprüche 1 bis 3, wobei die zusammenhängende Nukleotidsequenz eine Länge von 10 - 18 Nukleotiden aufweist.

5. Oligomer nach einem der Ansprüche 1 bis 4, wobei die zusammenhängende Nukleotidsequenz andere Nukleotidanaloge umfasst.

6. Oligomer nach Anspruch 5, wobei es sich bei den Nukleotidanalogen um zucker-modifizierte Nukleotide handelt, wie etwa zucker-modifizierte Nukleotide, die aus der aus LNA(Locked Nucleic Acid)-Einheiten; 2'-O-Alkyl-RNA-Einheiten, 2'-OMe-RNA-Einheiten, 2'-Amino-DNA-Einheiten und 2'-Fluor-DNA-Einheiten bestehenden Gruppe ausgewählt sind.

7. Oligomer nach Anspruch 5, wobei es sich bei den Nukleotidanalogen um LNA handelt.

8. Oligomer nach einem der Ansprüche 5 bis 7, bei dem es sich um ein Gapmer handelt.

9. Oligomer nach einem der Ansprüche 1 bis 8, das zur Herunterregulation der Expression von PIK3CA-Gen oder -mRNA in einer Zelle, die PIK3CA-Gen bzw. - mRNA exprimiert, fähig ist.

10. Oligomer nach einem der Ansprüche 1 bis 9, wobei das Oligomer aus
**5'- AₛGₛ^{Me}CₛcₛaₛtₛtₛcₛaₛtₛtₛcₛcₛAₛ^{Me}Cₛ^{Me}C-3' (SEQ ID NO: 67)**
besteht, wobei Großbuchstaben Beta-D-oxy-LNA-Monomere und Kleinbuchstaben DNA-Monomere bezeichnen, das tiefgestellte "s" eine Phosphorothioat-Verknüpfung und ^{Me}C ein Beta-D-oxy-LNA-Monomer mit einer 5-Methylcytosin-Base bezeichnet.

11. Konjugat, umfassend das Oligomer nach einem der Ansprüche 1 bis 10 und wenigstens eine kovalent an das Oligomer gebundene Nicht-Nukleotid- oder Nicht-Polynukleotid-Gruppierung.

12. Pharmazeutische Zusammensetzung, umfassend das Oligomer nach einem der Ansprüche 1 bis 10 oder das Konjugat nach Anspruch 11 sowie ein pharmazeutisch unbedenkliches Verdünnungsmittel, Trägermittel, Salz oder Hilfsmittel.

13. Oligomer nach einem der Ansprüche 1 bis 10 oder Konjugat nach Anspruch 11 zur Verwendung als Arzneimittel, wie etwa zur Behandlung von Hyperproliferationskrankheiten, wie etwa Krebs.

14. Verwendung eines Oligomers nach einem der Ansprüche 1 bis 10 oder eines Konjugats nach Anspruch 11 zur Herstellung eines Arzneimittels zur Behandlung von Hyperproliferationskrankheiten, wie etwa Krebs.

15. In-vitro-Verfahren zur Hemmung von PIK3CA in einer Zelle, die PIK3CA exprimiert, wobei man bei dem Verfahren der Zelle ein Oligomer nach einem der Ansprüche 1 bis 10 oder ein Konjugat nach Anspruch 11 verabreicht, um PIK3CA in der Zelle zu hemmen.

## Revendications

1. Oligomère ayant une séquence nucléotidique contiguë d'un total de 10 à 30 nucléotides, dans lequel ladite séquence nucléotidique contiguë est au moins 80 % homologue au complément inverse d'une région correspondante d'un gène ou ARNm PIK3CA de mammifère, ledit oligomère comprenant au moins un motif de LNA ; et la séquence nucléotidique contiguë comprenant au moins 10 nucléotides contigus qui sont à 100 % identiques à une région correspondante de SEQ ID NO 115 ou SEQ ID NO 7.

2. Oligomère selon la revendication 1 dans lequel la séquence nucléotidique contiguë comprend SEQ ID NO 115 ou SEQ ID NO 7.

3. Oligomère selon la revendication 1 ou 2, dans lequel la séquence nucléotidique contiguë est de 10 à 22 nucléotides de longueur.

4. Oligomère selon l'une quelconque des revendications 1 à 3, dans lequel la séquence nucléotidique contiguë est de 10 à 18 nucléotides de longueur.

5. Oligomère selon l'une quelconque des revendications 1 à 4, dans lequel la séquence nucléotidique contiguë comprend d'autres analogues de nucléotide.

6. Oligomère selon la revendication 5, dans lequel les analogues de nucléotide sont des nucléotides à glucide modifié, tels que les nucléotides à glucide modifié choisis dans le groupe constitué de : motifs d'acide nucléique bloqué (LNA) ; motifs 2'-O-alkyl-ARN, motifs 2'-OMe-ARN, motifs 2'-amino-ADN, et motifs 2'-fluoro-ADN.

7. Oligomère selon la revendication 5, dans lequel les analogues de nucléotide sont des LNA.

8. Oligomère selon l'une quelconque des revendications 5 à 7 qui est un gapmère.

9. Oligomère selon l'une quelconque des revendications 1 à 8, qui est capable de réguler à la baisse l'expression d'un gène ou ARNm PIK3CA dans une cellule qui exprime un gène ou ARNm PIK3CA.

10. Oligomère selon l'une quelconque des revendications 1 à 9, l'oligomère étant constitué de
**5'- AₛGₛ^{Me}CₛcₛaₛtₛtₛcₛaₛtₛtₛcₛcₛAₛ^{Me}Cₛ^{Me}C-3' (SEQ ID NO: 67)**
où les lettres en majuscules désignent des monomères bêta-D-oxy-LNA et les lettres en minuscules désignent des monomères d'ADN, l'indice « s » désigne un lieur phosphorothioate, et ^{Me}C désigne un monomère bêta-D-oxy-LNA contenant une base 5-méthylcytosine.

11. Conjugué comprenant l'oligomère selon l'une quelconque des revendications 1 à 10, et au moins un fragment non-nucléotide ou non-polynucléotide lié de façon covalente audit oligomère.

12. Composition pharmaceutique comprenant l'oligomère selon l'une quelconque des revendications 1 à 10, ou le conjugué selon la revendication 11, et un diluant, véhicule, sel ou adjuvant pharmaceutiquement acceptable.

13. Oligomère selon l'une quelconque des revendications 1 à 10, ou conjugué selon la revendication 11, pour utilisation en tant que médicament, par exemple pour le traitement des maladies hyperprolifératives, tel que le cancer.

14. Utilisation d'un oligomère selon l'une quelconque des revendications 1 à 10, ou d'un conjugué tel que défini dans la revendication 11, pour la fabrication d'un médicament pour le traitement des maladies hyperprolifératives, tel que le cancer.

15. Procédé *in vitro* pour l'inhibition de PIK3CA dans une cellule qui exprime le PIK3CA, ledit procédé comprenant l'administration d'un oligomère selon l'une quelconque des revendications 1 à 10, ou un conjugué selon la revendication 11, à ladite cellule de manière à inhiber le PIK3CA dans ladite cellule.


### SEQUENCE LISTING

<110> Santaris Pharma A/S
<120> RNA ANTAGONIST COMPOUNDS FOR THE MODULATION OF PIK3CA EXPRESSION.
<130> 1044WO
<150> US 60/992050
   <151> 2007-12-02
<160> 160
<170> PatentIn version 3.5
<210> 1
   <211> 3724
   <212> DNA
   <213> homo sapiens
<400> 1
   tctccctcgg cgccgccgcc gccgcccgcg gggctgggac ccgatgcggt tagagccgcg 60
   gagcctggaa gagccccgag cgtttctgct ttgggacaac catacatcta attccttaaa 120
   gtagttttat atgtaaaact tgcaaagaat cagaacaatg cctccacgac catcatcagg 180
   tgaactgtgg ggcatccact tgatgccccc aagaatccta gtagaatgtt tactaccaaa 240
   tggaatgata gtgactttag aatgcctccg tgaggctaca ttaataacca taaagcatga 300
   actatttaaa gaagcaagaa aataccccct ccatcaactt cttcaagatg aatcttctta 360
   cattttcgta agtgttactc aagaagcaga aagggaagaa ttttttgatg aaacaagacg 420
   actttgtgac cttcggcttt ttcaaccctt tttaaaagta attgaaccag taggcaaccg 480
   tgaagaaaag atcctcaatc gagaaattgg ttttgctatc ggcatgccag tgtgtgaatt 540
   tgatatggtt aaagatccag aagtacagga cttccgaaga aatattctga acgtttgtaa 600
   agaagctgtg gatcttaggg acctcaattc acctcatagt agagcaatgt atgtctatcc 660
   tccaaatgta gaatcttcac cagaattgcc aaagcacata tataataaat tagataaagg 720
   gcaaataata gtggtgatct gggtaatagt ttctccaaat aatgacaagc agaagtatac 780
   tctgaaaatc aaccatgact gtgtaccaga acaagtaatt gctgaagcaa tcaggaaaaa 840
   aactcgaagt atgttgctat cctctgaaca actaaaactc tgtgttttag aatatcaggg 900
   caagtatatt ttaaaagtgt gtggatgtga tgaatacttc ctagaaaaat atcctctgag 960
   tcagtataag tatataagaa gctgtataat gcttgggagg atgcccaatt tgatgttgat 1020
   ggctaaagaa agcctttatt ctcaactgcc aatggactgt tttacaatgc catcttattc 1080
   cagacgcatt tccacagcta caccatatat gaatggagaa acatctacaa aatccctttg 1140
   ggttataaat agtgcactca gaataaaaat tctttgtgca acctacgtga atgtaaatat 1200
   tcgagacatt gataagatct atgttcgaac aggtatctac catggaggag aacccttatg 1260
   tgacaatgtg aacactcaaa gagtaccttg ttccaatccc aggtggaatg aatggctgaa 1320
   ttatgatata tacattcctg atcttcctcg tgctgctcga ctttgccttt ccatttgctc 1380
   tgttaaaggc cgaaagggtg ctaaagagga acactgtcca ttggcatggg gaaatataaa 1440
   cttgtttgat tacacagaca ctctagtatc tggaaaaatg gctttgaatc tttggccagt 1500
   acctcatgga ttagaagatt tgctgaaccc tattggtgtt actggatcaa atccaaataa 1560
   agaaactcca tgcttagagt tggagtttga ctggttcagc agtgtggtaa agttcccaga 1620
   tatgtcagtg attgaagagc atgccaattg gtctgtatcc cgagaagcag gatttagcta 1680
   ttcccacgca ggactgagta acagactagc tagagacaat gaattaaggg aaaatgacaa 1740
   agaacagctc aaagcaattt ctacacgaga tcctctctct gaaatcactg agcaggagaa 1800
   agattttcta tggagtcaca gacactattg tgtaactatc cccgaaattc tacccaaatt 1860
   gcttctgtct gttaaatgga attctagaga tgaagtagcc cagatgtatt gcttggtaaa 1920
   agattggcct ccaatcaaac ctgaacaggc tatggaactt ctggactgta attacccaga 1980
   tcctatggtt cgaggttttg ctgttcggtg cttggaaaaa tatttaacag atgacaaact 2040
   ttctcagtat ttaattcagc tagtacaggt cctaaaatat gaacaatatt tggataactt 2100
   gcttgtgaga tttttactga agaaagcatt gactaatcaa aggattgggc actttttctt 2160
   ttggcattta aaatctgaga tgcacaataa aacagttagc cagaggtttg gcctgctttt 2220
   ggagtcctat tgtcgtgcat gtgggatgta tttgaagcac ctgaataggc aagtcgaggc 2280
   aatggaaaag ctcattaact taactgacat tctcaaacag gagaagaagg atgaaacaca 2340
   aaaggtacag atgaagtttt tagttgagca aatgaggcga ccagatttca tggatgctct 2400
   acagggcttt ctgtctcctc taaaccctgc tcatcaacta ggaaacctca ggcttgaaga 2460
   gtgtcgaatt atgtcctctg caaaaaggcc actgtggttg aattgggaga acccagacat 2520
   catgtcagag ttactgtttc agaacaatga gatcatcttt aaaaatgggg atgatttacg 2580
   gcaagatatg ctaacacttc aaattattcg tattatggaa aatatctggc aaaatcaagg 2640
   tcttgatctt cgaatgttac cttatggttg tctgtcaatc ggtgactgtg tgggacttat      2700
   tgaggtggtg cgaaattctc acactattat gcaaattcag tgcaaaggcg gcttgaaagg      2760
   tgcactgcag ttcaacagcc acacactaca tcagtggctc aaagacaaga acaaaggaga      2820
   aatatatgat gcagccattg acctgtttac acgttcatgt gctggatact gtgtagctac      2880
   cttcattttg ggaattggag atcgtcacaa tagtaacatc atggtgaaag acgatggaca      2940
   actgtttcat atagattttg gacacttttt ggatcacaag aagaaaaaat ttggttataa      3000
   acgagaacgt gtgccatttg ttttgacaca ggatttctta atagtgatta gtaaaggagc      3060
   ccaagaatgc acaaagacaa gagaatttga gaggtttcag gagatgtgtt acaaggctta      3120
   tctagctatt cgacagcatg ccaatctctt cataaatctt ttctcaatga tgcttggctc      3180
   tggaatgcca gaactacaat cttttgatga cattgcatac attcgaaaga ccctagcctt      3240
   agataaaact gagcaagagg ctttggagta tttcatgaaa caaatgaatg atgcacatca      3300
   tggtggctgg acaacaaaaa tggattggat cttccacaca attaaacagc atgcattgaa      3360
   ctgaaaagat aactgagaaa atgaaagctc actctggatt ccacactgca ctgttaataa      3420
   ctctcagcag gcaaagaccg attgcatagg aattgcacaa tccatgaaca gcattagaat      3480
   ttacagcaag aacagaaata aaatactata taatttaaat aatgtaaacg caaacagggt      3540
   ttgatagcac ttaaactagt tcatttcaaa attaagcttt agaataatgc gcaatttcat      3600
   gttatgcctt aagtccaaaa aggtaaactt tgaagattgt ttgtatcttt ttttaaaaaa      3660
   caaaacaaaa caaaaatccc caaaatatat agaaatgatg gagaaggaaa aaaaaaaaaa      3720
   aaaa                                                                   3724
<210> 2
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 2
   gaggcattct aaagtc 16
<210> 3
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 3
   attcttccct ttctgc 16
<210> 4
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 4
   tagacataca ttgctc 16
<210> 5
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 5
   tacttgccct gatatt 16
<210> 6
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 6
   cacataaggg ttctcc 16
<210> 7
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 7
   agccattcat tccacc 16
<210> 8
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 8
   cagtaacacc aatagg 16
<210> 9
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 9
   aactccaact ctaagc 16
<210> 10
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 10
   cagacagaag caattt 16
<210> 11
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 11
   ttattgtgca tctcag 16
<210> 12
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 12
   gcagaggaca taattc 16
<210> 13
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 13
   gatgtctggg ttctcc 16
<210> 14
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 14
   ttcttcttgt gatcca 16
<210> 15
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 15
   aagaaatcct gtgtca 16
<210> 16
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 16
   tctcctgaaa cctctc 16
<210> 17
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 17
   gattttagag agagga 16
<210> 18
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 18
   agtgatttta gagaga 16
<210> 19
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 19
   tcctgcttag tgattt 16
<210> 20
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 20
   ttctcctgct tagtga 16
<210> 21
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 21
   gccaccatga cgtgca 16
<210> 22
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 22
   accatgacgt gcatca 16
<210> 23
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 23
   gatttcagag agagga 16
<210> 24
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 24
   agtgatttca gagaga 16
<210> 25
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 25
   tcctgctcag tgattt 16
<210> 26
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 26
   ttctcctgct cagtga 16
<210> 27
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 27
   gccaccatga tgtgca 16
<210> 28
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<400> 28
   accatgatgt gcatca 16
<210> 29
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 29
   gaggcattct aaagtc 16
<210> 30
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 30
   attcttccct ttctgc 16
<210> 31
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 31
   tagacataca ttgctc 16
<210> 32
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 32
   tacttgccct gatatt 16
<210> 33
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 33
   cacataaggg ttctcc 16
<210> 34
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 34
   agccattcat tccacc 16
<210> 35
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 35
   cagtaacacc aatagg 16
<210> 36
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 36
   aactccaact ctaagc 16
<210> 37
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 37
   cagacagaag caattt 16
<210> 38
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 38
   ttattgtgca tctcag 16
<210> 39
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 39
   gcagaggaca taattc 16
<210> 40
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 40
   gatgtctggg ttctcc 16
<210> 41
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 41
   ttcttcttgt gatcca 16
<210> 42
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 42
   aagaaatcct gtgtca 16
<210> 43
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 43
   tctcctgaaa cctctc 16
<210> 44
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 44
   gattttagag agagga 16
<210> 45
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 45
   agtgatttta gagaga 16
<210> 46
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 46
   tcctgcttag tgattt 16
<210> 47
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 47
   ttctcctgct tagtga 16
<210> 48
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 48
   gccaccatga cgtgca 16
<210> 49
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 49
   accatgacgt gcatca 16
<210> 50
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 50
   gatttcagag agagga 16
<210> 51
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 51
   agtgatttca gagaga 16
<210> 52
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 52
   tcctgctcag tgattt 16
<210> 53
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 53
   ttctcctgct cagtga 16
<210> 54
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 54
   gccaccatga tgtgca 16
<210> 55
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 Gapmer - optionally phosphorothioate
<400> 55
   accatgatgt gcatca 16
<210> 56
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 56
   gaggcattct aaagtc 16
<210> 57
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 57
   attcttccct ttctgc 16
<210> 58
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 58
   ttcttccctt tctg 14
<210> 59
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 59
   tcttcccttt ct 12
<210> 60
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 60
   tagacataca ttgctc 16
<210> 61
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 61
   agacatacat tgct 14
<210> 62
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 62
   gacatacatt gc 12
<210> 63
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 63
   tacttgccct gatatt 16
<210> 64
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 64
   cacataaggg ttctcc 16
<210> 65
   <211> 14
   <212> DNA
   <213> artificial
<220>
   <223> LNA oligomer Sequence/oligomer Sequence motif
<220>
   <221> misc feature
   <222> (1)..(14)
   <223> 3-9-2 LNA Gapmer - fully phosphorothioate
<400> 65
   acataagggt tctc 14
<210> 66
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 66
   cataagggtt ct 12
<210> 67
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 67
   agccattcat tccacc 16
<210> 68
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 68
   gccattcatt ccac 14
<210> 69
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 69
   ccattcattc ca 12
<210> 70
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 70
   cagtaacacc aatagg 16
<210> 71
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 71
   agtaacacca atag 14
<210> 72
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate

<400> 72
   gtaacaccaa ta 12
<210> 73
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 73
   aactccaact ctaagc 16
<210> 74
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 74
   cagacagaag caattt 16
<210> 75
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 75
   agacagaagc aatt 14
<210> 76
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 76
   gacagaagca at 12
<210> 77
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 77
   ttattgtgca tctcag 16
<210> 78
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 78
   tattgtgcat ctca 14
<210> 79
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 79
   attgtgcatc tc 12
<210> 80
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 80
   gcagaggaca taattc 16
<210> 81
   <211> 16
   <212> DNA
   <213> artificial
<220>
   <223> LNA oligomer Sequence/oligomer Sequence motif
<220>
   <221> misc_feature
   <222> (1).. (16)
   <223> 3-10-3 LNA Gapmer - fully phosphorothioate
<400> 81
   gatgtctggg ttctcc 16
<210> 82
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 82
   ttcttcttgt gatcca 16
<210> 83
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 83
   tcttcttgtg atcc 14
<210> 84
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 84
   cttcttgtga tc **1**2
<210> 85
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 85
   aagaaatcct gtgtca 16
<210> 86
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 86
   tctcctgaaa cctctc 16
<210> 87
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 87
   gatttcagag agagga 16
<210> 88
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 88
   atttcagaga gagg 14
<210> 89
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 89
   tttcagagag ag 12
<210> 90
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 90
   agtgatttca gagaga 16
<210> 91
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 91
   gtgatttcag agag 14
<210> 92
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 92
   tgatttcaga ga 12
<210> 93
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 93
   tcctgctcag tgattt 16
<210> 94
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 94
   ttctcctgct cagtga 16
<210> 95
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 95
   gccaccatga tgtgca 16
<210> 96
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 96
   accatgatgt gcatca 16
<210> 97
   <211> 14
   <212> DNA
   <213> artificial
<220>
   <223> LNA oligomer Sequence/oligomer Sequence motif
<220>
   <221> misc_feature
   <222> (1)..(14)
   <223> 3-9-2 LNA Gapmer - fully phosphorothioate
<400> 97
   ccatgatgtg catc 14
<210> 98
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 98
   catgatgtgc at 12
<210> 99
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 99.
   gattttagag agagga 16
<210> 100
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 100
   agtgatttta gagaga 16
<210> 101
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 101
   tcctgcttag tgattt 16
<210> 102
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 102
   ttctcctgct tagtga 16
<210> 103
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 103
   gccaccatga cgtgca 16
<210> 104
   <211> 16
   <212> DNA
   <213> artificial
<220>
   <223> LNA oligomer Sequence/oligomer Sequence motif
<220>
   <221> misc_feature
   <222> (1)..(16)
   <223> 3-10-3 LNA Gapmer - fully phosphorothioate
<400> 104
   accatgacgt gcatca 16
<210> 105
   <211> 1068
   <212> PRT
   <213> homo sapiens
<400> 105
<210> 106
   <211> 3207
   <212> DNA
   <213> Mus musculus
<400> 106
   atgcctccac gaccatcttc gggtgaactg tggggcatcc acttgatgcc cccacgaatc      60
   ctagtggaat gtttactccc caatggaatg atagtgactt tagaatgcct ccgtgaggcc     120
   acactcgtca ccatcaaaca tgaactgttc agagaggcca ggaaataccc tctccatcag     180
   cttctgcaag acgaaacttc ttacattttc gtaagtgtca cccaagaagc agaaagggaa      240
   gaattttttg atgaaacaag acgactttgt gaccttcggc tttttcaacc ctttttaaaa      300
   gttattgaac cagtaggcaa ccgtgaagaa aagatcctca atcgagaaat tggttttgtt     360
   attggcatgc cagtgtgtga atttgatatg gttaaagatc cagaagtcca agactttcga     420
   aggaacattc tgaatgtttg caaagaagct gtggacctgc gggatctcaa ctcgcctcat     480
   agcagagcaa tgtatgtcta ccctccaaat gtcgagtctt ccccagaact gccaaagcac     540
   atctacaaca agttagataa aggacaaatc atagtggtga tttgggtaat agtctctcca     600
   aacaacgaca agcagaagta cactctgaag atcaatcatg actgtgtgcc agagcaagtc     660
   attgctgaag ccatcaggaa aaagactcgg agcatgttgt tgtcctctga gcagctgaaa     720
   ctctgtgtct tagaatatca gggcaagtat attctgaaag tgtgtggctg tgacgaatac     780
   ttcctggaaa agtaccctct gagtcagtac aagtacataa gaagctgtat aatgctgggg     840
   aggatgccca acttgatgct gatggccaaa gaaagcctat actctcagct gccgattgat     900
   agcttcacca tgccgtcata ctccaggcgc atctccacag ccacacccta catgaatgga     960
   gagacatcta cgaaatccct ctgggtcata aatagtgcgc tcagaataaa aattctttgt    1020
   gcaacctatg taaatgtaaa tattcgagac attgataaga tctatgttcg aacaggtatc    1080
   taccatggag gagaaccctt atgtgacaat gtgaacactc aaagagtacc ttgttccaat    1140
   cctaggtgga atgaatggct gaattatgat atatacattc ctgatcttcc tcgtctggcg    1200
   cgcctttgcc tttcaatctg ctctgttaaa ggccgaaagg gtgctaagga ggagcactgt    1260
   ccgttggcct ggggaaacat aaacttgttt gattatacag acaccctagt gtccgggaaa    1320
   atggctttga atctctggcc tgtaccgcat gggttagaag atctgctgaa ccctattggt    1380
   gttactgggt caaatccaaa taaagaaact ccatgcttag agttggagtt tgattggttc    1440
   agcagtgtgg tgaagtttcc agacatgtct gtgatcgaag aacatgccaa ttggtccgtg    1500
   tcccgagaag ctggattcag ttactcccat acaggactga gtaacagact agccagagac    1560
   aatgagttaa gagaaaatga caaggaacag ctccgagcac tttgcacccg ggacccacta    1620
   tctgaaatca ctgaacaaga gaaagacttc ctatggagcc acagacacta ctgcgtaact    1680
   attcctgaaa tcctacccaa attgcttctg tctgtcaagt ggaattccag agacgaagtg    1740
   gcccagatgt actgcttagt aaaagattgg cctccaatca aaccagagca agccatggaa    1800
   ctcctggact gtaactatcc agatcctatg gttcggagtt ttgctgttcg gtgcttagaa    1860
   aaatatttaa cagatgacaa actttctcag tacctcattc aacttgtaca ggtcttaaaa    1920
   tatgaacagt atttggataa cctgcttgtg agatttttac tcaagaaagc attgacaaat    1980
   caaaggattg gccatttttt cttttggcat ttaaaatctg agatgcacaa taagactgtc    2040
   agtcagaggt ttggcctgct attggagtcc tactgccgtg cctgtgggat gtatctgaag    2100
   cacctgaaca gacaagtaga ggccatggag aagctcatca acctaacgga catccttaag    2160
   caggagaaga aggatgagac acaaaaggta cagatgaagt ttttggttga acagatgaga     2220
   cagccagact tcatggatgc tttgcagggt tttctgtccc ctctgaatcc tgctcaccaa      2280
   ctaggaaacc tcaggcttga agagtgtcga attatgtcct ctgcaaaaag gccactgtgg    2340
   ttgaattggg agaacccaga catcatgtca gagctactgt ttcagaacaa tgagatcatc    2400
   tttaaaaatg gcgacgactt acggcaagat atgttaaccc ttcagatcat ccgaatcatg    2460
   gagaacatct ggcaaaacca aggccttgac cttcgcatgc taccttatgg ctgtctatcc    2520
   attggggact gtgtgggtct catcgaggtg gtgagaaact ctcacaccat catgcaaatc    2580
   cagtgcaaag gaggcctgaa gggggcgctg cagttcaaca gccacacact gcatcaatgg    2640
   ctcaaggaca agaacaaggg cgagatatat gatgcagcca ttgacctgtt cactcggtcc    2700
   tgcgctgggt actgcgtggc aacctttatc ttgggaattg gagaccggca caacagcaac    2760
   atcatggtga aagatgacgg acagctgttt catatagatt ttgggcactt tttggatcac    2820
   aagaagaaaa aatttggcta taagcgggaa cgtgtgccat ttgtgttgac acaggatttc    2880
   ttgattgtga ttagtaaggg agcacaagag tacaccaaga ccagagagtt tgagaggttt    2940
   caggagatgt gttacaaggc ttacctagca attcggcagc atgccaatct cttcatcaac    3000
   cttttttcaa tgatgcttgg ctctggaatg ccagaactac aatcttttga tgacattgca   3060
   tatatccgaa agactctagc cttggacaaa actgagcaag aagctttgga atatttcaca    3120
   aagcaaatga atgatgcaca tcatggtgga tggacgacaa aaatggattg gatcttccac    3180
   accatcaagc agcatgcttt gaactga                                        3207
<210> 107
   <211> 1068
   <212> PRT
   <213> Mus musculus
<400> 107
   Met Pro Pro Arg Pro Ser Ser Gly Glu Leu Trp Gly Ile His Leu Met
   1               5                   10                  15
   Pro Pro Arg Ile Leu Val Glu Cys Leu Leu Pro Asn Gly Met Ile Val
               20                  25                  30
   Thr Leu Glu Cys Leu Arg Glu Ala Thr Leu Val Thr Ile Lys His Glu
           35                  40                  45
   Leu Phe Arg Glu Ala Arg Lys Tyr Pro Leu His Gln Leu Leu Gln Asp
       50                  55                  60
   Glu Thr Ser Tyr Ile Phe Val Ser Val Thr Gln Glu Ala Glu Arg Glu
   65                  70                  75                  80
   Glu Phe Phe Asp Glu Thr Arg Arg Leu Cys Asp Leu Arg Leu Phe Gln
                   85                  90                  95
   Pro Phe Leu Lys Val Ile Glu Pro Val Gly Asn Arg Glu Glu Lys Ile
               100                 105                 110
   Leu Asn Arg Glu Ile Gly Phe Val Ile Gly Met Pro Val Cys Glu Phe
           115                 120                 125
   Asp Met Val Lys Asp Pro Glu Val Gln Asp Phe Arg Arg Asn Ile Leu
       130                 135                 140
   Asn Val Cys Lys Glu Ala Val Asp Leu Arg Asp Leu Asn Ser Pro His
   145                 150                 155                 160
   Ser Arg Ala Met Tyr Val Tyr Pro Pro Asn Val Glu Ser Ser Pro Glu
                   165                 170                 175
   Leu Pro Lys His Ile Tyr Asn Lys Leu Asp Lys Gly Gln Ile Ile Val
               180                 185                 190
   Val Ile Trp Val Ile Val Ser Pro Asn Asn Asp Lys Gln Lys Tyr Thr
           195                 200                 205
   Leu Lys Ile Asn His Asp Cys Val Pro Glu Gln Val Ile Ala Glu Ala
       210                 215                 220
   Ile Arg Lys Lys Thr Arg Ser Met Leu Leu Ser Ser Glu Gln Leu Lys
   225                 230                 235                 240
   Leu Cys Val Leu Glu Tyr Gln Gly Lys Tyr Ile Leu Lys Val Cys Gly
                   245                 250                 255
   Cys Asp Glu Tyr Phe Leu Glu Lys Tyr Pro Leu Ser Gln Tyr Lys Tyr
               260                 265                 270
   Ile Arg Ser Cys Ile Met Leu Gly Arg Met Pro Asn Leu Met Leu Met
           275                 280                 285
   Ala Lys Glu Ser Leu Tyr Ser Gln Leu Pro Ile Asp Ser Phe Thr Met
   290 295 300
   Pro Ser Tyr Ser Arg Arg Ile Ser Thr Ala Thr Pro Tyr Met Asn Gly
   305 310 315 320
   Glu Thr Ser Thr Lys Ser Leu Trp Val Ile Asn Ser Ala Leu Arg Ile
   325 330 335
   Lys Ile Leu Cys Ala Thr Tyr Val Asn Val Asn Ile Arg Asp Ile Asp
   340 345 350
   Lys Ile Tyr Val Arg Thr Gly Ile Tyr His Gly Gly Glu Pro Leu Cys
   355 360 365
   Asp Asn Val Asn Thr Gln Arg Val Pro Cys Ser Asn Pro Arg Trp Asn
   370 375 380
   Glu Trp Leu Asn Tyr Asp Ile Tyr Ile Pro Asp Leu Pro Arg Leu Ala
   385 390 395 400
   Arg Leu Cys Leu Ser Ile Cys Ser Val Lys Gly Arg Lys Gly Ala Lys
   405 410 415
   Glu Glu His Cys Pro Leu Ala Trp Gly Asn Ile Asn Leu Phe Asp Tyr
   420 425 430
   Thr Asp Thr Leu Val Ser Gly Lys Met Ala Leu Asn Leu Trp Pro Val
   435 440 445
   Pro His Gly Leu Glu Asp Leu Leu Asn Pro Ile Gly Val Thr Gly Ser
   450 455 460
   Asn Pro Asn Lys Glu Thr Pro Cys Leu Glu Leu Glu Phe Asp Trp Phe
   465 470 475 480
   Ser Ser Val Val Lys Phe Pro Asp Met Ser Val Ile Glu Glu His Ala
   485 490 495
   Asn Trp Ser Val Ser Arg Glu Ala Gly Phe Ser Tyr Ser His Thr Gly
   500 505 510
   Leu Ser Asn Arg Leu Ala Arg Asp Asn Glu Leu Arg Glu Asn Asp Lys
   515 520 525
   Glu Gln Leu Arg Ala Leu Cys Thr Arg Asp Pro Leu Ser Glu Ile Thr
   530 535 540
   Glu Gln Glu Lys Asp Phe Leu Trp Ser His Arg His Tyr Cys Val Thr
   545 550 555 560
   Ile Pro Glu Ile Leu Pro Lys Leu Leu Leu Ser Val Lys Trp Asn Ser
   565 570 575
   Arg Asp Glu Val Ala Gln Met Tyr Cys Leu Val Lys Asp Trp Pro Pro
   580 585 590
   Ile Lys Pro Glu Gln Ala Met Glu Leu Leu Asp Cys Asn Tyr Pro Asp
   595 600 605
   Pro Met Val Arg Ser Phe Ala Val Arg Cys Leu Glu Lys Tyr Leu Thr
   610 615 620
   Asp Asp Lys Leu Ser Gln Tyr Leu Ile Gln Leu Val Gln Val Leu Lys
   625 630 635 640
   Tyr Glu Gln Tyr Leu Asp Asn Leu Leu Val Arg Phe Leu Leu Lys Lys
   645 650 655
   Ala Leu Thr Asn Gln Arg Ile Gly His Phe Phe Phe Trp His Leu Lys
   660 665 670
   Ser Glu Met His Asn Lys Thr Val Ser Gln Arg Phe Gly Leu Leu Leu
   675 680 685
   Glu Ser Tyr Cys Arg Ala Cys Gly Met Tyr Leu Lys His Leu Asn Arg
   690 695 700
   Gln Val Glu Ala Met Glu Lys Leu Ile Asn Leu Thr Asp Ile Leu Lys
   705 710 715 720
   Gln Glu Lys Lys Asp Glu Thr Gln Lys Val Gln Met Lys Phe Leu Val
   725 730 735
   Glu Gln Met Arg Gln Pro Asp Phe Met Asp Ala Leu Gln Gly Phe Leu
   740 745 750
   Ser Pro Leu Asn Pro Ala His Gln Leu Gly Asn Leu Arg Leu Glu Glu
   755 760 765
   Cys Arg Ile Met Ser Ser Ala Lys Arg Pro Leu Trp Leu Asn Trp Glu
   770 775 780
   Asn Pro Asp Ile Met Ser Glu Leu Leu Phe Gln Asn Asn Glu Ile Ile
   785 790 795 800
   Phe Lys Asn Gly Asp Asp Leu Arg Gln Asp Met Leu Thr Leu Gln Ile
   805 810 815
   Ile Arg Ile Met Glu Asn Ile Trp Gln Asn Gln Gly Leu Asp Leu Arg
   820 825 830
   Met Leu Pro Tyr Gly Cys Leu Ser Ile Gly Asp Cys Val Gly Leu Ile
   835 840 845
   Glu Val Val Arg Asn Ser His Thr Ile Met Gln Ile Gln Cys Lys Gly
   850 855 860
   Gly Leu Lys Gly Ala Leu Gln Phe Asn Ser His Thr Leu His Gln Trp
   865 870 875 880
   Leu Lys Asp Lys Asn Lys Gly Glu Ile Tyr Asp Ala Ala Ile Asp Leu
   885 890 895
   Phe Thr Arg Ser Cys Ala Gly Tyr Cys Val Ala Thr Phe Ile Leu Gly
   900 905 910
   Ile Gly Asp Arg His Asn Ser Asn Ile Met Val Lys Asp Asp Gly Gln
   915 920 925
   Leu Phe His Ile Asp Phe Gly His Phe Leu Asp His Lys Lys Lys Lys
   930 935 940
   Phe Gly Tyr Lys Arg Glu Arg Val Pro Phe Val Leu Thr Gln Asp Phe
   945 950 955 960
   Leu Ile Val Ile Ser Lys Gly Ala Gln Glu Tyr Thr Lys Thr Arg Glu
   965 970 975
   Phe Glu Arg Phe Gln Glu Met Cys Tyr Lys Ala Tyr Leu Ala Ile Arg
   980 985 990
   Gln His Ala Asn Leu Phe Ile Asn Leu Phe Ser Met Met Leu Gly Ser
   995 1000 1005
   Gly Met Pro Glu Leu Gln Ser Phe Asp Asp Ile Ala Tyr Ile Arg
   1010 1015 1020
   Lys Thr Leu Ala Leu Asp Lys Thr Glu Gln Glu Ala Leu Glu Tyr
   1025 1030 1035
   Phe Thr Lys Gln Met Asn Asp Ala His His Gly Gly Trp Thr Thr
   1040 1045 1050
   Lys Met Asp Trp Ile Phe His Thr Ile Lys Gln His Ala Leu Asn
   1055 1060 1065
<210> 108
   <211> 4326
   <212> DNA
   <213> Macaca mulatta
<400> 108
   tctccctcgg cgccgccgcc gccgcccgcg gggctgggac ccgatgcggt tagagccgcg      60
   gagcctggaa gagccccgag cgtttctgct ttgggacaac catacgtcta attctttaaa     120
   gtagttttat atgtaaaacc tgcaaaaaat cagaacaatg cctccacgac catcatcagg     180
   tgaactgtgg ggcatccact tgatgccccc aagaatccta gtagaatgtt tactaccaaa     240
   tggaatgata gtgactttag aatgcctccg tgaggctaca ttaataacca taaagcatga     300
   actatttaaa gaagcaagaa aataccccct ccatcaactt cttcaagatg aatcttctta     360
   cattttcgta agtgttaccc aagaagcaga aagggaagaa ttttttgatg aaacaagacg     420
   actttgtgac cttcggcttt ttcaaccctt tttaaaagta attgaaccag taggcaaccg     480
   tgaagaaaag atcctcaatc gagaaattgg ttttgctatt ggcatgccag tgtgtgaatt     540
   tgatatggtt aaagatccag aagtacagga cttccgaaga aatattctga atgtttgtaa     600
   agaagctgtg gatcttaggg atctcaattc acctcatagt agagcaatgt atgtctatcc     660
   tccaaatgta gaatcttcgc cagaattgcc aaagcacata tataataaat tagataaagg     720
   gcaaataata gtggtgattt gggtaatagt ttctccaaat aatgacaagc agaagtatac     780
   tctgaaaatc aaccatgact gtgtaccaga acaagtaatt gctgaagcaa tcaggaaaaa     840
   aactcgaagt atgttgctat cctctgaaca gctaaaactc tgtgttttag aatatcaggg     900
   caagtatatt ttaaaagtgt gtggatgtga tgaatacttc ctagaaaaat atcctctgag     960
   tcagtataag tatataagaa gctgtataat gcttgggagg atgcccaatt tgatgttgat    1020
   ggctaaagaa agcctttatt ctcaactgcc aatggactgt tttacaatgc catcttattc    1080
   cagacgcatt tccacagcta caccatatat gaatggagaa acatctacaa aatccctttg    1140
   ggttataaat agtgcactca gaataaaaat tctttgtgca acctacgtga atgtaaatat    1200
   tcgagacatt gataagatct atgttcgaac aggtatctac catggaggag aacccttatg    1260
   tgacaatgtg aacactcaaa gagtaccttg ttccaatccc aggtggaatg aatggctgaa    1320
   ttatgatata tacattcctg accttcctcg tgctgctcga ctttgccttt ccatttgctc    1380
   tgttaaaggc cgaaagggtg ctaaagagga acactgtcca ttggcatggg gaaatataaa    1440
   cttgtttgat tacacagaca ctctagtatc tggaaaaatg gctttgaatc tttggccagt    1500
   acctcatgga ttagaagatt tgctgaaccc tattggtgtt actggatcaa atccaaataa    1560
   agaaactcca tgcttagagt tggagtttga ctggttcagc agtgtggtaa agttcccaga    1620
   tatgtcagtg attgaagagc atgccaattg gtctgtgtcc cgagaagcag gatttagcta    1680
   ttcccacgca ggactgagta acaggctagc tagagacaat gaattaaggg aaaatgacaa    1740
   agaacagctc aaagcaattt ctacacgaga tcctctctct gaaatcactg agcaggagaa    1800
   agattttctg tggagccaca gacactattg tgtaactatc cccgaaattc tacccaaatt    1860
   gcttctgtct gttaaatgga attctagaga tgaagtagcc cagatgtatt gcttggtaaa    1920
   agactggcct ccaatcaaac ctgaacaggc tatggaactt ctggactgta attacccaga    1980
   tcctatggtt cgaggttttg ctgttcggtg cttggaaaaa tatttaacag atgacaaact    2040
   ttctcagtat ttaattcagc tagtacaggt cctaaaatat gaacaatatt tggataactt    2100
   gcttgtgaga tttttactga agaaagcatt gactaatcaa aggattgggc actttttctt    2160
   ttggcattta aaatctgaga tgcacaataa aacagttagc cagaggtttg gcctgctttt    2220
   ggagtcctat tgtcgtgcat gtgggatgta tttgaagcac ctgaataggc aagtcgaggc    2280
   aatggaaaag ctcattaact taactgacat tctcaaacag gagaaaaagg atgaaacaca    2340
   aaaggtacag atgaagtttt tagttgagca aatgaggcga ccagatttca tggatgctct    2400
   gcagggcttt ctatctcctc taaaccctgc tcatcaacta ggaaatctca ggcttgaaga    2460
   gtgtcgaatt atgtcctctg caaaaaggcc actgtggttg aattgggaga acccagacat    2520
   catgtcagag ttactgtttc agaacaatga gatcatcttt aaaaatgggg atgatttacg    2580
   gcaagatatg ctaacacttc aaattattcg tattatggaa aatatctggc aaaatcaagg    2640
   tcttgatctt cgaatgttac cttatggttg tctgtcaatc ggtgactgtg tgggacttat    2700
   tgaggtggtg cgaaattctc acactattat gcaaattcag tgcaaaggcg gcttgaaagg    2760
   tgcactgcag ttcaacagcc acactctaca tcagtggctc aaagacaaga acaaaggaga    2820
   aatatatgat gcagccattg acctgtttac acgttcatgt gctggatatt gtgtcgctac    2880
   cttcattctg ggaattggag atcgtcacaa tagtaacatc atggtgaaag acgatggaca    2940
   actgtttcat atagattttg gacacttttt ggatcacaag aagaaaaaat ttggctataa    3000
   acgagaacgt gtgccatttg ttttgacaca ggatttctta atagtgatta gtaaaggagc    3060
   ccaagaatgc acaaagacaa gagaatttga gaggtttcag gagatgtgtt acaaggctta    3120
   tctagctatt cgacagcatg ccaatctctt cataaatctt ttctcaatga tgcttggctc    3180
   tggaatgcca gaactacaat cttttgatga cattgcatac attcgaaaga ccctagcctt    3240
   agataaaact gagcaagagg ctttggaata tttcatgaaa caaatgaatg atgcacatca    3300
   tggtggctgg acaacaaaaa tggattggat cttccacaca attaaacagc atgcattgaa    3360
   ctgaaaagat aactgagaaa atgaaagctc actctctgga ttccacactg cactgttaat    3420
   aactatcagc aggcaaagac cgattgcata ggaattgcac aatccatgaa cagcattaga    3480
   atttacagca agaacagaaa taaaatacta tataatttaa tgtaaacgca aacagggttt    3540
   gatagcactt aaactagttc atttcacaat taagctttag aataatgcgc aatttcatgt    3600
   tatgccttaa gtccaaaaag gtaaactttg aagattgttt gtatcttttt ttaaaaaaca    3660
   aaacaaaaca aaaatcccca aaatatatag aaatgatgga gaaggaaaaa tgatgttttt    3720
   ttttgtcttg caaatgttct atgttttgaa atgtggacac aacaaaagct gttattgttt    3780
   taggtgtaag taaactggag tttatgttaa attacattaa gattggaaaa gaatgaaaat    3840
   ttcttatttt tccattgctg ttcaatttat agtttgagtg ggtttttgac tccttgttta    3900
   atgaagaaaa atgcttgggg tggaagggac tcttgagatt tcaccagaga ctttttcttt    3960
   ttaataaatc aaaccttttg atgatttgag gtcttatctg cagttttgga agcagtcaca    4020
   aatgagacct gttataaggt ggtatttttt ttttttttct ggacagtatt taaaggattt    4080
   tatttcccag ggaaattctg ggctcccaca gagtttaaaa aaaaaaaaaa aaaaaaatca    4140
   tagaaaaaga atgaacagga atagttctta ttccaaaatt gtacagtatt caccttaagt    4200
   tgattttttt ctccttttgc agttgaactg aatacatttt tcatgcatgt tttccaaaaa    4260
   atagaagtat taatgttatt aaaaagatta ttttttttat taaaggctat ttatattata    4320
   gaaact                                                               4326
<210> 109
   <211> 1068
   <212> PRT
   <213> Macaca mulatta
<400> 109

<210> 110
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 110
   cacggaggca ttctaaagtc acta 24
<210> 111
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 111
   aaaaattctt ccctttctgc ttct 24
<210> 112
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 112
   aggatagaca tacattgctc tact 24
<210> 113
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 113
   aatatacttg ccctgatatt ctaa 24
<210> 114
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 114
   ttgtcacata agggttctcc tcca 24
<210> 115
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 115
   attcagccat tcattccacc tggg 24
<210> 116
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 116
   gatccagtaa caccaatagg gttc 24
<210> 117
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 117
   gtcaaactcc aactctaagc atgg 24
<210> 118
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 118
   ttaacagaca gaagcaattt gggt 24
<210> 119
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 119
   tgttttattg tgcatctcag attt 24
<210> 120
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 120
   ttttgcagag gacataattc gaca 24
<210> 121
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 121
   acatgatgtc tgggttctcc caat 24
<210> 122
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 122
   ttttttcttc ttgtgatcca aaaa 24
<210> 123
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 123
   tattaagaaa tcctgtgtca aaac 24
<210> 124
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 124
   cacatctcct gaaacctctc aaat 24
<210> 125
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 125
   cagtgatttt agagagagga tctc 24
<210> 126
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 126
   gctcagtgat tttagagaga ggat 24
<210> 127
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 127
   tttctcctgc ttagtgattt caga 24
<210> 128
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 128
   atctttctcc tgcttagtga tttc 24
<210> 129
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 129
   tccagccacc atgacgtgca tcat 24
<210> 130
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 130
   agccaccatg acgtgcatca ttca 24
<210> 131
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 131
   cagtgatttc agagagagga tctc 24
<210> 132
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 132
   gctcagtgat ttcagagaga ggat 24
<210> 133
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 133
   tttctcctgc tcagtgattt caga 24
<210> 134
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 134
   atctttctcc tgctcagtga tttc 24
<210> 135
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 135
   tccagccacc atgatgtgca tcat 24
<210> 136
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<400> 136
   agccaccatg atgtgcatca ttca 24
<210> 137
   <211> 14
   <212> DNA
   <213> artificial
<220>
   <223> LNA oligomer Sequence/oligomer Sequence motif
<220>
   <221> misc_feature
   <222> (1)..(14)
   <223> 3-8-3 LNA Gapmer - fully phosphorothioate
<400> 137
   attttagaga gagg 14
<210> 138
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 138
   ttttagagag ag 12
<210> 139
   <211> 14
   <212> DNA
   <213> artificial
<220>
   <223> LNA oligomer Sequence/oligomer Sequence motif
<220>
   <221> misc_feature
   <222> (1)..(14)
   <223> 3-8-3 LNA Gapmer - fully phosphorothioate
<400> 139
   gtgattttag agag 14
<210> 140
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 140
   tgattttaga ga 12
<210> 141
   <211> 14
   <212> DNA
   <213> artificial
<220>
   <223> LNA oligomer Sequence/oligomer Sequence motif
<220>
   <221> misc_feature
   <222> (1)..(14)
   <223> 3-8-3 LNA Gapmer - fully phosphorothioate
<400> 141
   cctgcttagt gatt 14
<210> 142
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 142
   ctgcttagtg at 12
<210> 143
   <211> 14
   <212> DNA
   <213> artificial
<220>
   <223> LNA oligomer Sequence/oligomer Sequence motif
<220>
   <221> misc_feature
   <222> (1)..(14)
   <223> 3-8-3 LNA Gapmer - fully phosphorothioate
<400> 143
   tctcctgctt agtg 14
<210> 144
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 144
   ctcctgctta gt 12
<210> 145
   <211> 14
   <212> DNA
   <213> artificial
<220>
   <223> LNA oligomer Sequence/oligomer Sequence motif
<220>
   <221> misc_feature
   <222> (1)..(14)
   <223> 3-8-3 LNA Gapmer - fully phosphorothioate
<400> 145
   ccaccatgac gtgc 14
<210> 146
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 146
   caccatgacg tg 12
<210> 147
   <211> 14
   <212> DNA
   <213> artificial
<220>
   <223> LNA oligomer Sequence/oligomer Sequence motif
<220>
   <221> misc_feature
   <222> (1)..(14)
   <223> 3-8-3 LNA Gapmer - fully phosphorothioate
<400> 147
   ccatgacgtg catc 14
<210> 148
   <211> 12
   <212> DNA
   <213> artificial
<220>
   <223> LNA oligomer Sequence/oligomer Sequence motif
<220>
   <221> misc_feature
   <222> (1)..(12)
   <223> 2-8-2 LNA Gapmer - fully phosphorothioate
<400> 148
   catgacgtgc at 12
<210> 149
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<220>
   <221> misc_feature
   <222> (13)..(13)
   <223> n is a, c, g, or t
<400> 149
   gctcagtgat ttnagagaga ggat 24
<210> 150
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<220>
   <221> misc_feature
   <222> (12)..(12)
   <223> n is a, c, g, or t
<400> 150
   tttctcctgc tnagtgattt caga 24
<210> 151
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<220>
   <221> misc_feature
   <222> (6)..(6)
   <223> n is a, c, g, or t
<400> 151
   gatttnagag agagga 16
<210> 152
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<220>
   <221> misc_feature
   <222> (9)..(9)
   <223> n is a, c, g, or t
<400> 152
   agtgatttna gagaga 16
<210> 153
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<220>
   <221> misc_feature
   <222> (15)..(15)
   <223> n is a, c, g, or t
<400> 153
   atctttctcc tgctnagtga tttc 24
<210> 154
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<220>
   <221> misc_feature
   <222> (10)..(10)
   <223> n is a, c, g, or t
<400> 154
   cagtgatttn agagagagga tctc 24
<210> 155
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<220>
   <221> misc_feature
   <222> (8)..(8)
   <223> n is a, c, g, or t
<400> 155
   tcctgctnag tgattt 16
<210> 156
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<220>
   <221> misc_feature
   <222> (11)..(11)
   <223> n is a, c, g, or t
<400> 156
   ttctcctgct nagtga 16
<210> 157
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<220>
   <221> misc_feature
   <222> (12)..(12)
   <223> n is a, c, g, or t
<400> 157
   agccaccatg angtgcatca ttca 24
<210> 158
   <211> 24
   <212> DNA
   <213> artificial
<220>
   <223> LNA oligomer Sequence/oligomer Sequence motif
<220>
   <221> misc_feature
   <222> (1)..(24)
   <223> nucleotide or nucleotide analogues - optionally phosphorothioate
<220>
   <221> misc_feature
   <222> (15)..(15)
   <223> n is a, c, g, or t
<400> 158
   tccagccacc atgangtgca tcat 24
<210> 159
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<220>
   <221> misc_feature
   <222> (11)..(11)
   <223> n is a, c, g, or t
<400> 159
   gccaccatga ngtgca 16
<210> 160
   <211> 16
   <212> DNA
   <213> artificial
<220>
   <223> LNA oligomer Sequence/oligomer Sequence motif
<220>
   <221> misc_feature
   <222> (1)..(16)
   <223> nucleotide or nucleotide analogues - optionally phosphorothioate
<220>
   <221> misc_feature
   <222> (8)..(8)
   <223> n is a, c, g, or t
<400> 160
   accatgangt gcatca 16